(19) **Europäisches Patentamt / European Patent Office / Office européen des brevets**

(11) **EP 2 275 135 A1**

(12) **EUROPEAN PATENT APPLICATION**
published in accordance with Art. 153(4) EPC

(43) Date of publication:
**19.01.2011 Bulletin 2011/03**

(51) Int Cl.:
*A61K 39/395* (2006.01)     *A61K 31/404* (2006.01)
*A61K 31/44* (2006.01)     *A61K 45/00* (2006.01)
*A61P 35/00* (2006.01)     *A61P 43/00* (2006.01)

(21) Application number: **09727052.4**

(22) Date of filing: **19.03.2009**

(86) International application number:
**PCT/JP2009/001249**

(87) International publication number:
**WO 2009/122667 (08.10.2009 Gazette 2009/41)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority:  **04.04.2008   JP 2008098309**
   **26.09.2008   PCT/JP2008/002690**

(71) Applicant: **Chugai Seiyaku Kabushiki Kaisha Kita-ku Tokyo 115-8543 (JP)**

(72) Inventors:
• **KINOSHITA, Yasuko**
   **Kamakura-shi**
   **Kanagawa 247-8530 (JP)**

• **SUGIMOTO, Masamichi**
   **Kamakura-shi**
   **Kanagawa 247-8530 (JP)**
• **ISHIGURO, Takahiro**
   **Kamakura-shi**
   **Kanagawa 247-8530 (JP)**

(74) Representative: **Woods, Geoffrey Corlett**
   **J.A. Kemp & Co.**
   **14 South Square**
   **Gray's Inn**
   **London**
   **WC1R 5JJ (GB)**

(54) **THERAPEUTIC FOR HEPATIC CANCER**

(57)    A novel pharmaceutical composition for treating or preventing hepatocellular carcinoma and a method of treatment are provided. A pharmaceutical composition for treating or preventing liver cancer is obtained by combining a chemotherapeutic agent with an anti-glypican 3 antibody. Also disclosed is a pharmaceutical composition for treating or preventing liver cancer which comprises as an active ingredient an anti-glypican 3 antibody for use in combination with a chemotherapeutic agent, or which comprises as an active ingredient a chemotherapeutic agent for use in combination with an anti-glypican 3 antibody. Using the chemotherapeutic agent and the anti-glypican 3 antibody in combination yields better therapeutic effects than using the chemotherapeutic agent alone, and mitigates side effects that arise from liver cancer treatment with the chemotherapeutic agent.

【FIG.1】

EP 2 275 135 A1

**Description**

Related Applications

This application claims priority from JP Appln No. 2008-98309, filed on April 4, 2008 and PCT/JP2008/002690, file on September 26, 2008, the contents of which are herein incorporated by reference.

Field of the Invention

The present invention relates to a pharmaceutical composition for effectively treating or preventing liver cancer comprising a combination of a chemotherapy agent and an anti-glypican 3 antibody, as well as a method of treatment using the pharmaceutical composition.

Description of the Related Art

The annual number of deaths from hepatocellular carcinoma is 600,000, making it the fifth leading cause of death from cancer worldwide (Llovet, J.M., Burroughs, A., Bruix, J.: Lancet 362, 1907-17 (2003)). The majority of hepatocellular carcinoma patients die within one year from diagnosis of this disease. Unfortunately, hepatocellular carcinoma is frequently diagnosed at a late stage where curative treatment is not very successful. In such patients, the effects of treatment modalities that include chemotherapy, chemoembolization, cauterization or electron beam therapy remain inadequate. Most patients exhibit a relapse of the disease accompanied by vascular infiltration and multiple intrahepatic metastases, which will rapidly progress to the advanced stage. The five-year survival rate is only 7% (Bosch, F.X., Ribes, J., Cleries, R.: Gastroenterology 127, S5-16 (2004)). Patients with hepatocellular carcinoma amenable to the surgical resection of localized tumors have relatively good prognosis, although the five-year survival rate is still between 15% and 39% (Takenaka, K., Kawahara, N., Yamamoto, K., Kajiyama, K., Maeda, T., Itasaka, H., Shirabe, K., Nishizaki, T., Yanaga, K., Sugimachi, K.: Arch Surg. 131, 71-6 (1996)). Accordingly, there exists a desire in the art for a new way to treat this highly malignant disease.

Hepatocellular carcinoma reportedly accounts for more than 90% of primary liver cancers in Japan. Methods for treating hepatocellular cancer include a chemotherapy-based transcatheter arterial embolization (TAE) therapy, where selective necrosis of the hepatocellular carcinoma is induced by infusion of a mixture of an oil-based contrast medium (Lipiodol), a carcinostatic and an obstructing substance (Gelfoam) into the hepatic artery (serving as the nutrient supply pathway to the tumor) and thereby obstruct the nutrient artery. In addition, clinical trials are being conducted on systemic chemotherapy using such chemotherapeutic agents as fluorouracil (5-FU), uracil-tegafur (UFT), mitomycin C (MMC), mitoxantrone (DHAD), adriamycin (ADR), epirubicin (EPI) and cisplatin (CDDP), either alone or in combination with interferon (IFN) (Yeo, W., Mok, T.S., Zee, B., Leung, T.W., Lai, P.B., Lau, W.Y., Koh, J., Mo, F.K., Yu, S.C., Chan, A.T., Hui, P., Ma, B., Lam, K.C., Ho, W.M., Wong, H.T., Tang, A., Johnson, P.J.: J. Natl. Cancer Inst. 97, 1532-8 (2005)). However, a standard therapy for liver cancer has yet to be established (Furuse, J., Ishii, H., Nakachi, K., Suzuki, E., Shimizu, S., Nakajima, K.: Cancer Sci., Oct. 22 (E-Pub) (2007)).

Recently, a number of drugs targeted to growth factors are being investigated for the treatment of liver cancer. These investigations suggest that epidermal growth factor receptor/human epidermal receptor 1 (EGFR/HER1) is expressed in an active form in human liver cancer cells. Erlotinib, an inhibitor of epidermal growth factor receptor/human epidermal receptor 1, and lapatinib, a double tyrosine kinase inhibitor of epidermal growth factor receptor/human epidermal receptor 1 and ErbB-2 (Her2/neu), have been investigated in phase II clinical trials. The rate of response in patients given erlotinib was 4 to 9%, the time to progression was from 2.1 to 3.2 months, and the survival period was from 5.8 to 13 months. However, the rate of response in patients given lapatinib was 0% and the time to progression was 1.8 months (Philip, P.A., Mahoney, M.R., Allmer, C., Thomas, J., Pitot, H.C., Kim, G., Donehower, R.C., Fitch, T., Picus, J., Erlichman, C.: J. Clin. Oncol. 23, 6657-63 (2005)). The orally active form of the kinase inhibitor Sorafenib (Nexavar, BAY43-9006) inhibits Raf/MEK/ERK signal transduction at the Raf kinase step, thereby blocking the growth of cancer cells. In addition, by targeting VEGFR-2, VEGFR-3 and PDGFR-$\beta$ tyrosine kinase, Sorafenib elicits an antiangiogenic effect, and thus has exhibited beneficial effects compared with the chemotherapeutic agents listed above. In a phase II clinical trial on non-Japanese and Japanese subjects, the time to progression was from 4.2 to 4.9 months, the response rate was from 2 to 4%, and the progression-free survival period was from 9.2 to 15.6 months (Thomas, M.B., Dutta, A., Brown, T., Charnsangavej, C., Rashid, A., Hoff, P.M., Dancey, J., Abbruzzese, J.L.: J. Clin. Oncol., 2005 ASCO Annual Meeting Proceedings, 23, 16S (2005)). Sunitinib (SU11248) is a multi-kinase inhibitor like Sorafenib which has an activity of inhibiting two or more types of kinase (Mendel DB, Laird AD, Xin X, Louie SG, Christensen JG, Li G, Schreck RE, Abrams TJ, Ngai TJ, Lee LB, Murray LJ, Carver J, Chan E, Moss KG, Haznedar JO, Sukbuntherng J, Blake RA, Sun L, Tang C, Miller T, Shirazian S, McMahon G, Cherrington JM; Clin Cancer Res (2003), 9, 327-37) and is under a clinical test for the treatment of hepatocarcinoma.

In the clinical test where 34 patients with advanced hepatocarcinoma received Sunitinib, 1 patient had a partial response after 12 weeks of treatment, and 17 patients achieved stable condition. It was demonstrated that the median overall survival was 9.8 months, the median progression-free survival of 3.9 months (95% confidence interval 2.6-6.9), the progression-free survival at three months was 56%, and the progression-free survival at six months was 32%, suggesting that Sunitinib exhibits an anti-tumor activity against hepatocarcinoma (Zhu A, Sahani D, di Tomaso E et al.,; 99th AACR annual meeting. San Diego, CA, USA 12-16 April (2008)). Generally, as liver cancer progresses, various symptoms specific to liver cancer and associated with liver dysfunction are observed, such as lack of appetite, weight loss, general sense of fatigue, palpable right hypochondrial mass, right hypochondrial pain, sense of abdominal fullness, fever and jaundice. However, chemotherapeutic agents such as Sorafenib and lapatinib typically have a number of complications, including such side effects as diarrhea or constipation, anemia, suppression of the immune system (to a degree as to provoke infections or sepsis of lethal severity), hemorrhaging, cardiac toxicity, hepatic toxicity, renal toxicity, lack of appetite and weight loss.

Although particular early-stage symptoms are not usually noted early in liver cancer, various symptoms specific to liver cancer and associated with liver dysfunction are observed as liver cancer progresses, including lack of appetite, weight loss, general sense of fatigue, palpable right hypochondrial mass, right hypochondrial pain, sense of abdominal fullness, fever and jaundice. It has been clinically observed that such symptoms are enhanced by the use of the above-mentioned chemotherapeutic agents. For example, lack of appetite in a patient in which hepatocellular cancer has been detected, and symptoms such as loss of weight that are associated with or independent of lack of appetite, are sometimes enhanced by the administration of chemotherapeutic agents to the patient. When such symptoms develop, it is sometimes necessary to discontinue the use of the chemotherapeutic agents. Hence, expansion of the above symptoms is a factor that hampers treatment with chemotherapeutic agents.

Accordingly, there exists a desire for the establishment of better treatment modalities in terms of enhancing the therapeutic effects and improving the quality of life of the patient receiving treatment.

SUMMARY OF THE INVENTION

It is therefore an object of the present invention to provide a liver cancer drug which, in patients that are observed to have symptoms distinctive to liver cancer, such as weight loss associated with cancer progression, is capable of reducing the side effects characteristic of chemotherapeutic agents, such as diarrhea or constipation, anemia, suppression of the immune system (to a degree as to provoke infections or sepsis of lethal severity), hemorrhaging, cardiac toxicity, hepatic toxicity, renal toxicity, lack of appetite and weight loss that arise with the administration of chemotherapeutic agents such as kinase inhibitors, and which moreover is capable of enhancing the therapeutic effects against liver cancer.

The inventors have discovered that by combining a therapeutic antibody which binds to a protein that is highly expressed in liver cancer cells and has the ability to elicit cytotoxicity against such cells with a liver cancer drug comprising as the active ingredient a chemotherapeutic agent effective against liver cancer cells, better therapeutic effects can be achieved in the liver cancer patient than when such a chemotherapeutic agent is used alone. Moreover, the inventors have also found that the drug of the present invention, in addition to having the desirable effects mentioned above, significantly reduces side effects characteristic of chemotherapeutic agents, such as diarrhea or constipation, anemia, suppression of the immune system (to a degree as to provoke infections or sepsis of lethal severity), hemorrhaging, cardiac toxicity, hepatic toxicity, renal toxicity, lack of appetite and weight loss, thereby exhibiting good therapeutic effects.

In addition, as shown in the examples below, when a non-human animal liver cancer model implanted with HepG2 was used as a model of the symptoms such as lack of appetite and weight loss associated with liver cancer progression, such weight loss was further enhanced by the administration of a chemotherapeutic agent, in particular Sorafenib, while the weight loss was suppressed by administration of the drug according to the invention.

The present invention provides:

[1] A pharmaceutical composition for treating or preventing a liver cancer comprising a combination of a chemotherapeutic agent and an anti-glypican 3 antibody;
[2] The pharmaceutical composition according to [1], wherein the pharmaceutical composition is a combination preparation;
[3] The pharmaceutical composition according to [1], wherein the chemotherapeutic agent and the anti-glypican 3 antibody are concomitantly administered;
[4] The pharmaceutical composition according to [3], wherein the chemotherapeutic agent and the anti-glypican 3 antibody are administered simultaneously or sequentially;
[5] The pharmaceutical composition according to [3], wherein the chemotherapeutic agent and the anti-glypican 3 antibody are administered separately;
[6] A pharmaceutical composition for treating or preventing a liver cancer for use in combination with a chemotherapeutic agent, said composition comprising an anti-glypican 3 antibody as an active ingredient;

[7] The pharmaceutical composition according to [6], wherein the anti-glypican 3 antibody is administered simultaneously with the chemotherapeutic agent;

[8] The pharmaceutical composition according to [6], wherein the anti-glypican 3 antibody is administered before or after administration of the chemotherapeutic agent;

[9] A pharmaceutical composition for treating or preventing a liver cancer for use in combination with an anti-glypican 3 antibody, said composition comprising a chemotherapeutic agent as an active ingredient;

[10] The pharmaceutical composition according to [9], wherein the chemotherapeutic agent is administered simultaneously with the anti-glypican 3 antibody;

[11] The pharmaceutical composition according to [9], wherein the chemotherapeutic agent is administered before or after administration of the anti-glypican 3 antibody;

[12] The pharmaceutical composition according to any one of [1]-[11], wherein the chemotherapeutic agent is a kinase inhibitor;

[13] The pharmaceutical composition according to [12], wherein the chemotherapeutic agent is a multi-kinase inhibitor;

[14] The pharmaceutical composition according to [12] or [13], wherein the chemotherapeutic agent is Sorafenib (BAY43-9006);

[15] The pharmaceutical composition according to [12] or [13], wherein the chemotherapeutic agent is Sunitinib;

[16] The pharmaceutical composition according to any one of [1]-[15], wherein the anti-glypican 3 antibody has cytotoxicity;

[17] The pharmaceutical composition according to any one of [1]-[16], wherein the anti-glypican 3 antibody comprises:

the H chain variable region comprising the CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and

the L chain variable region comprising the CDR1, 2 and 3 of:

CDR1 comprising the amino acid sequence shown in SEQ ID NO:8,
CDR2 comprising the amino acid sequence shown in SEQ ID NO:24, and
CDR3 comprising the amino acid sequence shown in SEQ ID NO:25;

[18] The pharmaceutical composition according to any one of [1]-[17], wherein the anti-glypican 3 antibody is capable of binding to an epitope to which a second antibody can bind, wherein said second antibody comprises:

the H chain variable region comprising CDR1, 2, and 3 comprising the amino acid sequence shown in SEQ ID NOs:5, 6, and 7, respectively, and
the L chain variable region comprising CDR1, 2, and 3 comprising the amino acid sequence shown in SEQ ID NOs:8, 24, and 25, respectively;

[19] The pharmaceutical composition according to any one of [1]-[16] or [18], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of: CDR1 comprising the amino acid sequence shown in any one of SEQ ID NOs:9-23,
CDR2 comprising the amino acid sequence shown in SEQ ID NO:24, and
CDR3 comprising the amino acid sequence shown in SEQ ID NO:25;

[20] The pharmaceutical composition according to any one of [1]-[16] or [18], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:26, and
CDR3 comprising the amino acid sequence shown in SEQ ID NO:7; and
the L chain variable region comprising CDR1, 2 and 3 of: CDR1 comprising the amino acid sequence shown in SEQ ID NO:28,
CDR2 comprising the amino acid sequence shown in SEQ ID NO:24, and
CDR3 comprising the amino acid sequence shown in SEQ ID NO:25;

[21] The pharmaceutical composition according to any one of [1]-[16] or [18], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:30, and
CDR3 comprising the amino acid sequence shown in SEQ ID NO:7; and
the L chain variable region comprising CDR1, 2 and 3 of: CDR1 comprising the amino acid sequence shown in SEQ ID NO:32,
CDR2 comprising the amino acid sequence shown in SEQ ID NO:24, and
CDR3 comprising the amino acid sequence shown in SEQ ID NO:25;

[22] The pharmaceutical composition according to any one of [1]-[21], wherein the anti-glypican 3 antibody is a humanized antibody;
[23] The pharmaceutical composition according to [22], wherein the anti-glypican 3 antibody comprises:

the H chain variable region comprising the amino acid sequence shown in SEQ ID NO:3; and
the L chain variable region comprising the amino acid sequence shown in SEQ ID NO:4;

[24] The pharmaceutical composition according to [22], wherein the anti-glypican 3 antibody comprises:

the H chain variable region comprising the amino acid sequence shown in SEQ ID NO:3; and
the L chain variable region comprising the amino acid sequence wherein the 34th Gly of SEQ ID NO:4 is substituted with another amino acid residue;

[25] The pharmaceutical composition according to [22], wherein the anti-glypican 3 antibody comprises:

the H chain variable region comprising the amino acid sequence shown in SEQ ID NO:27; and
the L chain variable region comprising the amino acid sequence shown in SEQ ID NO:29;

[26] The pharmaceutical composition according to [22], wherein the anti-glypican 3 antibody comprises:

the H chain variable region comprising the amino acid sequence shown in SEQ ID NO:31; and
the L chain variable region comprising the amino acid sequence shown in SEQ ID NO:33;

[27] An agent for reducing a side-effect caused by a treatment of a liver cancer by chemotherapeutic agent, said agent comprising a therapeutic antibody as an active ingredient;
[28] The side-effect reducing agent according to [27], wherein the chemotherapeutic agent is a kinase inhibitor;
[29] The side-effect reducing agent according to [27] or [28], wherein the chemotherapeutic agent is a multi-kinase inhibitor;
[30] The side-effect reducing agent according to [28] or [29], wherein the chemotherapeutic agent is Sorafenib (BAY43-9006);
[31] The side-effect reducing agent according to [28] or [29], wherein the chemotherapeutic agent is Sunitinib;
[32] The side-effect reducing agent according to any one of [27]-[31], wherein the side effect is weight loss;
[33] The side-effect reducing agent according to any one of [27]-[32], wherein the therapeutic antibody is an anti-glypican 3 antibody;
[34] The side-effect reducing agent according to [33], wherein the anti-glypican 3 antibody has cytotoxicity;

[35] The side-effect reducing agent according to [33] or [34], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of:
CDR1 comprising the amino acid sequence shown in SEQ ID NO:8,
CDR2 comprising the amino acid sequence shown in SEQ ID NO:24, and
CDR3 comprising the amino acid sequence shown in SEQ ID NO:25;

[36] The side-effect reducing agent according to any one of [33]-[35], wherein the anti-glypican 3 antibody is capable of binding to an epitope to which a second antibody can bind, wherein said second antibody comprises:

the H chain variable region comprising CDR1, 2, and 3 comprising the amino acid sequence shown in SEQ ID NOs:5, 6, and 7, respectively, and
the L chain variable region comprising CDR1, 2, and 3 comprising the amino acid sequence shown in SEQ ID NOs:8, 24, and 25, respectively;

[37] The side-effect reducing agent according to any one of [33], [34] or [36], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of: CDR1 comprising the amino acid sequence shown in any one of SEQ ID NOs:9-23,
CDR2 comprising the amino acid sequence shown in SEQ ID NO:24, and CDR3 comprising the amino acid sequence shown in SEQ ID NO:25;

[38] The side-effect reducing agent according to any one of [33], [34] or [36], wherein the anti-glypican 3 antibody comprises the H chain variable region comprising CDR1, 2 and 3 of:

CDR1 comprising the amino acid sequence shown in SEQ ID NO:5,
CDR2 comprising the amino acid sequence shown in SEQ ID NO:26, and
CDR3 comprising the amino acid sequence shown in SEQ ID NO:7; and
the L chain variable region comprising CDR1, 2 and 3 of: CDR1 comprising the amino acid sequence shown in SEQ ID NO:28,
CDR2 comprising the amino acid sequence shown in SEQ ID NO:24, and
CDR3 comprising the amino acid sequence shown in SEQ ID NO:25;

[39] The side-effect reducing agent according to any one of [33], [34] or [36], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:30, and
CDR3 comprising the amino acid sequence shown in SEQ ID NO:7; and
the L chain variable region comprising CDR1, 2 and 3 of: CDR1 comprising the amino acid sequence shown in SEQ ID NO:32,
CDR2 comprising the amino acid sequence shown in SEQ ID NO:24, and
CDR3 comprising the amino acid sequence shown in SEQ ID NO:25;

[40] The side-effect reducing agent according to any one of [33]-[39], wherein the anti-glypican 3 antibody is a humanized antibody;

[41] The side-effect reducing agent according to [40], wherein the anti-glypican 3 antibody comprises:

the H chain variable region comprising the amino acid sequence shown in SEQ ID NO:3; and
the L chain variable region comprising the amino acid sequence shown in SEQ ID NO:4;

[42] The side-effect reducing agent according to [40], wherein the anti-glypican 3 antibody comprises:

the H chain variable region comprising the amino acid sequence shown in SEQ ID NO:3; and
the L chain variable region comprising the amino acid sequence wherein the 34th Gly of SEQ ID NO:4 is substituted with another amino acid residue;

[43] The side-effect reducing agent according to [40], wherein the anti-glypican 3 antibody comprises:

the H chain variable region comprising the amino acid sequence shown in SEQ ID NO:27; and
the L chain variable region comprising the amino acid sequence shown in SEQ ID NO:29;

[44] The side-effect reducing agent according to [40], wherein the anti-glypican 3 antibody comprises:

the H chain variable region comprising the amino acid sequence shown in SEQ ID NO:31; and
the L chain variable region comprising the amino acid sequence shown in SEQ ID NO:33;

[45] A pharmaceutical composition for enhancing the efficacy of treatment of a liver cancer by a chemotherapeutic agent, said composition comprising an anti-glypican 3 antibody;
[46] The pharmaceutical composition according to [45], wherein the anti-glypican 3 antibody is administered simultaneously with the chemotherapeutic agent;
[47] The pharmaceutical composition according to [45], wherein the anti-glypican 3 antibody is administered before or after administration of the chemotherapeutic agent;
[48] The pharmaceutical composition according to any one of [45]-[47], wherein the chemotherapeutic agent is a kinase inhibitor;
[49] The pharmaceutical composition according to [48], wherein the chemotherapeutic agent is a multi-kinase inhibitor;
[50] The pharmaceutical composition according to any one of [45]-[49], wherein the chemotherapeutic agent is Sorafenib (BAY43-9006);
[51] The pharmaceutical composition according to any one of [45]-[49], wherein the chemotherapeutic agent is Sunitinib;
[52] The pharmaceutical composition according to any one of [45]-[51], wherein the anti-glypican 3 antibody has cytotoxicity;
[53] The pharmaceutical composition according to any one of [45]-[52], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of: CDR1 comprising the amino acid sequence shown in SEQ ID NO:8,
CDR2 comprising the amino acid sequence shown in SEQ ID NO:24, and
CDR3 comprising the amino acid sequence shown in SEQ ID NO:25;

[54] The pharmaceutical composition according to any one of [45]-[53], wherein the anti-glypican 3 antibody is capable of binding to an epitope to which a second antibody can bind, wherein said second antibody comprises:

the H chain variable region comprising CDR1, 2, and 3 comprising the amino acid sequence shown in SEQ ID NOs:5, 6, and 7, respectively, and
the L chain variable region comprising CDR1, 2, and 3 comprising the amino acid sequence shown in SEQ ID NOs:8, 24, and 25, respectively;

[55] The pharmaceutical composition according to any one of [45]-[52] or [54], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO: 6, and
CDR3 comprising the amino acid sequence shown in SEQ ID NO:7; and
the L chain variable region comprising CDR1, 2 and 3 of: CDR1 comprising the amino acid sequence shown in any one of SEQ ID NOs:9-23,
CDR2 comprising the amino acid sequence shown in SEQ ID NO:24, and
CDR3 comprising the amino acid sequence shown in SEQ ID NO:25;

[56] The pharmaceutical composition according to any one of [45]-[52] or [54], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:26, and
CDR3 comprising the amino acid sequence shown in SEQ ID NO:7; and
the L chain variable region comprising CDR1, 2 and 3 of: CDR1 comprising the amino acid sequence shown in SEQ ID NO:28,
CDR2 comprising the amino acid sequence shown in SEQ ID NO:24, and
CDR3 comprising the amino acid sequence shown in SEQ ID NO:25;

[57] The pharmaceutical composition according to any one of [45]-[52] or [54], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:30, and
CDR3 comprising the amino acid sequence shown in SEQ ID NO:7; and
the L chain variable region comprising CDR1, 2 and 3 of: CDR1 comprising the amino acid sequence shown in SEQ ID NO:32,
CDR2 comprising the amino acid sequence shown in SEQ ID NO:24, and
CDR3 comprising the amino acid sequence shown in SEQ ID NO:25;

[58] The pharmaceutical composition according to any one of [45]-[57], wherein the anti-glypican 3 antibody is a humanized antibody;
[59] The pharmaceutical composition according to [58], wherein the anti-glypican 3 antibody comprises:

the H chain variable region comprising the amino acid sequence shown in SEQ ID NO:3; and
the L chain variable region comprising the amino acid sequence shown in SEQ ID NO:4;

[60] The pharmaceutical composition according to [58], wherein the anti-glypican 3 antibody comprises:

the H chain variable region comprising the amino acid sequence shown in SEQ ID NO:3; and
the L chain variable region comprising the amino acid sequence wherein the 34th Gly of SEQ ID NO:4 is substituted with another amino acid residue;

[61] The pharmaceutical composition according to [58], wherein the anti-glypican 3 antibody comprises:

the H chain variable region comprising the amino acid sequence shown in SEQ ID NO:27; and
the L chain variable region comprising the amino acid sequence shown in SEQ ID NO:29;

[62] The pharmaceutical composition according to [58], wherein the anti-glypican 3 antibody comprises:

the H chain variable region comprising the amino acid sequence shown in SEQ ID NO:31; and
the L chain variable region comprising the amino acid sequence shown in SEQ ID NO:33;

[63] A method for treating or preventing a liver cancer in a subject comprising administering to the subject a combination of an effective amount of a chemotherapeutic agent and an anti-glypican 3 antibody;
[64] The method according to [63], wherein the chemotherapeutic agent and the anti-glypican 3 antibody are administered simultaneously or sequentially;
[65] The method according to [63], wherein the chemotherapeutic agent and the anti-glypican 3 antibody are administered separately;
[66] The method according to any one of [63]-[65], wherein the chemotherapeutic agent is a kinase inhibitor;
[67] The method according to [66], wherein the chemotherapeutic agent is a multi-kinase inhibitor;
[68] The method according to [66] or [67], wherein the chemotherapeutic agent is Sorafenib (BAY43-9006);
[69] The method according to [66] or [67], wherein the chemotherapeutic agent is Sunitinib;
[70] The method according to any one of [63]-[69], wherein the anti-glypican 3 antibody has cytotoxicity;
[71] The method according to any one of [63]-[70], wherein the anti-glypican 3 antibody comprises:

the H chain variable region comprising the CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising the CDR1, 2 and 3 of:

CDR1 comprising the amino acid sequence shown in SEQ ID NO:8,
CDR2 comprising the amino acid sequence shown in SEQ ID NO:24, and
CDR3 comprising the amino acid sequence shown in SEQ ID NO:25;

[72] The method according to any one of [63]-[71], wherein the anti-glypican 3 antibody is capable of binding to an epitope to which a second antibody can bind, wherein said second antibody comprises:

the H chain variable region comprising CDR1, 2, and 3 comprising the amino acid sequence shown in SEQ ID NOs:5, 6, and 7, respectively, and
the L chain variable region comprising CDR1, 2, and 3 comprising the amino acid sequence shown in SEQ ID NOs:8, 24, and 25, respectively;

[73] The method according to any one of [63]-[70] or [72], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of:

CDR1 comprising the amino acid sequence shown in any one of SEQ ID NOs:9-23,
CDR2 comprising the amino acid sequence shown in SEQ ID NO:24, and
CDR3 comprising the amino acid sequence shown in SEQ ID NO:25;

[74] The method according to any one of [63]-[70] or [72], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:26, and
CDR3 comprising the amino acid sequence shown in SEQ ID NO:7; and
the L chain variable region comprising CDR1, 2 and 3 of: CDR1 comprising the amino acid sequence shown in SEQ ID NO:28,
CDR2 comprising the amino acid sequence shown in SEQ ID NO:24, and

CDR3 comprising the amino acid sequence shown in SEQ ID NO:25;

[75] The method according to any one of [63]-[70] or [72], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:30, and
CDR3 comprising the amino acid sequence shown in SEQ ID NO:7; and
the L chain variable region comprising CDR1, 2 and 3 of: CDR1 comprising the amino acid sequence shown in SEQ ID NO:32,
CDR2 comprising the amino acid sequence shown in SEQ ID NO:24, and
CDR3 comprising the amino acid sequence shown in SEQ ID NO:25;

[76] The method according to any one of [63]-[75], wherein the anti-glypican 3 antibody is a humanized antibody;
[77] The method according to [76], wherein the anti-glypican 3 antibody comprises:

the H chain variable region comprising the amino acid sequence shown in SEQ ID NO:3; and
the L chain variable region comprising the amino acid sequence shown in SEQ ID NO:4;

[78] The method according to [76], wherein the anti-glypican 3 antibody comprises:

the H chain variable region comprising the amino acid sequence shown in SEQ ID NO:3; and
the L chain variable region comprising the amino acid sequence wherein the 34th Gly of SEQ ID NO:4 is substituted with another amino acid residue;

[79] The method according to [76], wherein the anti-glypican 3 antibody comprises:

the H chain variable region comprising the amino acid sequence shown in SEQ ID NO:27; and
the L chain variable region comprising the amino acid sequence shown in SEQ ID NO:29;

[80] The method according to [76], wherein the anti-glypican 3 antibody comprises:

the H chain variable region comprising the amino acid sequence shown in SEQ ID NO:31; and
the L chain variable region comprising the amino acid sequence shown in SEQ ID NO:33;

[81] A method for reducing a side-effect caused by a treatment of a liver cancer by chemotherapeutic agent in a subject comprising administering to the subject an effective amount of a therapeutic antibody;
[82] The method according to [81], wherein the chemotherapeutic agent is a kinase inhibitor;
[83] The method according to [81] or [82], wherein the chemotherapeutic agent is a multi-kinase inhibitor;
[84] The method according to [82] or [83], wherein the chemotherapeutic agent is Sorafenib (BAY43-9006);
[85] The method according to [82] or [83], wherein the chemotherapeutic agent is Sunitinib;
[86] The method according to any one of [81]-[85], wherein the side effect is weight loss;
[87] The method according to any one of [81]-[86], wherein the therapeutic antibody is an anti-glypican 3 antibody;
[88] The method according to [87], wherein the anti-glypican 3 antibody has cytotoxicity;
[89] The method according to [87] or [88], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of: CDR1 comprising the amino acid sequence shown in SEQ ID NO:8,
CDR2 comprising the amino acid sequence shown in SEQ ID NO:24, and
CDR3 comprising the amino acid sequence shown in SEQ ID NO:25;

[90] The method according to any one of [87]-[89], wherein the anti-glypican 3 antibody is capable of binding to an epitope to which a second antibody can bind, wherein said second antibody comprises:

the H chain variable region comprising CDR1, 2, and 3 comprising the amino acid sequence shown in SEQ ID NOs:5, 6, and 7, respectively, and
the L chain variable region comprising CDR1, 2, and 3 comprising the amino acid sequence shown in SEQ ID NOs:8, 24, and 25, respectively;

[91] The method according to any one of [87], [88] or [90], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of: CDR1 comprising the amino acid sequence shown in any one of SEQ ID NOs:9-23,
CDR2 comprising the amino acid sequence shown in SEQ ID NO:24, and CDR3 comprising the amino acid sequence shown in SEQ ID NO:25;

[92] The method according to any one of [87], [88] or [89], wherein the anti-glypican 3 antibody comprises the H chain variable region comprising CDR1, 2 and 3 of:

CDR1 comprising the amino acid sequence shown in SEQ ID NO:5,
CDR2 comprising the amino acid sequence shown in SEQ ID NO:26, and
CDR3 comprising the amino acid sequence shown in SEQ ID NO:7; and
the L chain variable region comprising CDR1, 2 and 3 of: CDR1 comprising the amino acid sequence shown in SEQ ID NO:28,
CDR2 comprising the amino acid sequence shown in SEQ ID NO:24, and
CDR3 comprising the amino acid sequence shown in SEQ ID NO:25;

[93] The method according to any one of [87], [88] or [90], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:30, and
CDR3 comprising the amino acid sequence shown in SEQ ID NO:7; and
the L chain variable region comprising CDR1, 2 and 3 of: CDR1 comprising the amino acid sequence shown in SEQ ID NO:32,
CDR2 comprising the amino acid sequence shown in SEQ ID NO:24, and
CDR3 comprising the amino acid sequence shown in SEQ ID NO:25;

[94] The method according to any one of [87]-[93], wherein the anti-glypican 3 antibody is a humanized antibody;
[95] The method according to [94], wherein the anti-glypican 3 antibody comprises:

the H chain variable region comprising the amino acid sequence shown in SEQ ID NO:3; and
the L chain variable region comprising the amino acid sequence shown in SEQ ID NO:4;

[96] The method according to [94], wherein the anti-glypican 3 antibody comprises:

the H chain variable region comprising the amino acid sequence shown in SEQ ID NO:3; and
the L chain variable region comprising the amino acid sequence wherein the 34th Gly of SEQ ID NO:4 is substituted with another amino acid residue;

[97] The method according to [94], wherein the anti-glypican 3 antibody comprises:

the H chain variable region comprising the amino acid sequence shown in SEQ ID NO:27; and
the L chain variable region comprising the amino acid sequence shown in SEQ ID NO:29;

[98] The method according to [94], wherein the anti-glypican 3 antibody comprises:

the H chain variable region comprising the amino acid sequence shown in SEQ ID NO:31; and
the L chain variable region comprising the amino acid sequence shown in SEQ ID NO:33;

[99] A method for enhancing the efficacy of treatment of a liver cancer by a chemotherapeutic agent in a subject comprising administering to the subject an effective amount of an anti-glypican 3 antibody;
[100] The method according to [99], wherein the anti-glypican 3 antibody is administered simultaneously with the chemotherapeutic agent;
[101] The method according to [99], wherein the anti-glypican 3 antibody is administered before or after administration of the chemotherapeutic agent;
[102] The method according to any one of [99]-[101] wherein the chemotherapeutic agent is a kinase inhibitor;
[103] The method according to [102], wherein the chemotherapeutic agent is a multi-kinase inhibitor;
[104] The method according to any one of [99]-[103], wherein the chemotherapeutic agent is Sorafenib (BAY43-9006);
[105] The method according to any one of [99]-[103], wherein the chemotherapeutic agent is Sunitinib;
[106] The method according to any one of [99]-[105], wherein the anti-glypican 3 antibody has cytotoxicity;
[107] The method according to any one of [99]-[106], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of: CDR1 comprising the amino acid sequence shown in SEQ ID NO:8,
CDR2 comprising the amino acid sequence shown in SEQ ID NO:24, and
CDR3 comprising the amino acid sequence shown in SEQ ID NO:25;

[108] The method according to any one of [99]-[107], wherein the anti-glypican 3 antibody is capable of binding to an epitope to which a second antibody can bind, wherein said second antibody comprises:

the H chain variable region comprising CDR1, 2, and 3 comprising the amino acid sequence shown in SEQ ID NOs:5, 6, and 7, respectively, and
the L chain variable region comprising CDR1, 2, and 3 comprising the amino acid sequence shown in SEQ ID NOs:8, 24, and 25, respectively;

[109] The method according to any one of [99]-[106] or [108], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of: CDR1 comprising the amino acid sequence shown in any one of SEQ ID NOs:9-23,
CDR2 comprising the amino acid sequence shown in SEQ ID NO:24, and
CDR3 comprising the amino acid sequence shown in SEQ ID NO:25;

[110] The method according to any one of [99]-[106] or [108], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:26, and
CDR3 comprising the amino acid sequence shown in SEQ ID NO:7; and
the L chain variable region comprising CDR1, 2 and 3 of: CDR1 comprising the amino acid sequence shown in SEQ ID NO:28,
CDR2 comprising the amino acid sequence shown in SEQ ID NO:24, and

CDR3 comprising the amino acid sequence shown in SEQ ID NO:25;

[111] The method according to any one of [99]-[106] or [108], wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:30, and
CDR3 comprising the amino acid sequence shown in SEQ ID NO:7; and
the L chain variable region comprising CDR1, 2 and 3 of: CDR1 comprising the amino acid sequence shown in SEQ ID NO:32,
CDR2 comprising the amino acid sequence shown in SEQ ID NO:24, and
CDR3 comprising the amino acid sequence shown in SEQ ID NO:25;

[112] The method according to any one of [99]-[111], wherein the anti-glypican 3 antibody is a humanized antibody;
[113] The method according to [112] wherein the anti-glypican 3 antibody comprises:

the H chain variable region comprising the amino acid sequence shown in SEQ ID NO:3; and
the L chain variable region comprising the amino acid sequence shown in SEQ ID NO:4;

[114] The method according to [112], wherein the anti-glypican 3 antibody comprises:

the H chain variable region comprising the amino acid sequence shown in SEQ ID NO:3; and
the L chain variable region comprising the amino acid sequence wherein the 34th Gly of SEQ ID NO:4 is substituted with another amino acid residue;

[115] The method according to [112], wherein the anti-glypican 3 antibody comprises:

the H chain variable region comprising the amino acid sequence shown in SEQ ID NO:27; and
the L chain variable region comprising the amino acid sequence shown in SEQ ID NO:29;

[116] The method according to [112], wherein the anti-glypican 3 antibody comprises:

the H chain variable region comprising the amino acid sequence shown in SEQ ID NO:31; and
the L chain variable region comprising the amino acid sequence shown in SEQ ID NO:33.

## BRIEF DESCRIPTION OF THE DRAWINGS

Fig. 1 is a graph showing the growth suppressing effects on the liver cancer cell by the combined administration of doxorubicin (DOX) or mitoxantrone (MX) and GC33 antibody;
Fig. 2 is a graph showing the antitumor effects, based on the change in tumor volume, of hGC33 antibody and Sorafenib in a mouse model implanted with cells of the human liver cancer cell line Huh-7;
Fig. 3 is a graph showing the antitumor effects, based on the change in tumor volume, of hGC33 antibody and Sorafenib in a mouse model implanted with cells of the human liver cancer cell line HepG2; and
Fig. 4 is a graph showing the effects of hGC33 antibody and Sorafenib on the weight loss of a mouse model implanted with cells of the human liver cancer cell line HepG2, based on changes in the weight of the model.
Fig. 5 is a graph showing the anti-tumor effects of the antibody pH7pL16 and Sorafenib on a mouse model implanted with a human liver cancer cell line HepG2 as expressed in the change in the tumor volume.
Fig. 6 is a graph showing the effect of the antibody pH7pL16 and Sorafenib on the weight loss in a mouse model implanted with a human liver cancer cell line HepG2 as expressed in the change in the body weight.
Fig. 7 is a graph showing the anti-tumor effects of the antibody hGC33 and Sunitinib on a mouse model implanted with a human liver cancer cell line HepG2 as expressed in the change in the tumor volume (mean $\pm$ SD).
Fig. 8 shows the amino acid sequence of the H chain- and the L chain- variable regions of humanized antibodies preferably used in the present invention.
Fig. 9 shows the amino acid sequence of CDRs of the H chain- and the L chain- variable regions of humanized antibodies preferably used in the present invention.

### DESCRIPTION OF THE PREFERRED EMBODIMENTS

The present invention provides a pharmaceutical composition for treating or preventing liver cancer comprising a combination of a chemotherapeutic agent and an anti-glypican 3 antibody.

As used herein, the phrase "pharmaceutical composition for treating or preventing liver cancer comprising a combination of a chemotherapeutic agent and an anti-glypican 3 antibody" refers to a pharmaceutical composition in which a chemotherapeutic agent and an anti-glypican 3 antibody are combined for concurrent, separate, or successive administration in the treatment or prevention of liver cancer. The pharmaceutical composition of the invention may be provided in the form of a combination preparation which contains both a chemotherapeutic agent and an anti-glypican 3 antibody. Alternatively, a drug comprising the chemotherapeutic agent and a drug comprising the anti-glypican 3 antibody may be separately provided, and used concurrently, separately, or successively. It is also possible to provide a kit composed of a drug comprising the chemotherapeutic agent and a drug comprising the anti-glypican 3 antibody.

In the above pharmaceutical composition, when the chemotherapeutic agent and the anti-glypican 3 antibody are provided in separate drugs, these drugs may have the same dosage form or different dosage forms. For example, both may be of mutually differing drug forms selected from parenteral drugs, injections, drops and intravenous fluids, or both may be of the same dosage form selected from parenteral drugs, injections, drops and intravenous fluids. In addition, one or more other type of drug may also be combined in the above-mentioned pharmaceutical composition.

In another aspect, the invention provides a pharmaceutical composition comprising an anti-glypican 3 antibody as the active ingredient for use in combination with a chemotherapeutic agent to treat or prevent liver cancer. When the pharmaceutical composition comprising an anti-glypican 3 antibody as the active ingredient is used in combination with a chemotherapeutic agent, it may be administered simultaneously with the chemotherapeutic agent, or may be administered before or after the chemotherapeutic agent. When the anti-glypican 3 antibody is administered before or after the chemotherapeutic agent, the dosing period may be optimized by measuring the residual concentration of the chemotherapeutic agent in the subject. The concentration can be determined by subjecting samples collected from the subject to a method of analysis familiar to persons of ordinary skill in the art using a separatory apparatus such as any of various types of chromatographs.

In a further aspect, the invention provides a pharmaceutical composition comprising a chemotherapeutic drug as the active ingredient for use in combination with an anti-glypican 3 antibody to treat or prevent liver cancer. When the pharmaceutical composition comprising a chemotherapeutic agent as the active ingredient is used together with an anti-glypican 3 antibody, it may be administered simultaneously with the anti-glypican 3 antibody, or may be administered before or after the anti-glypican 3 antibody. When the chemotherapeutic agent antibody is administered before or after the anti-glypican 3 antibody, the dosing period may be optimized by measuring the residual concentration of the anti-glypican 3 antibody in the subject. The concentration can be determined by subjecting samples collected from the subject to an immunological measurement familiar to persons of ordinary skill in the art, such as the ELISA technique described below.

### Chemotherapeutic Agent

The chemotherapeutic agent used in the invention includes all chemotherapeutic agents which are being used, or which have been suggested as useful, in cancer chemotherapy. The chemotherapeutic drug may be locally injected or may be administered systemically. When drug is locally injected, injection can be performed by a method known by those skilled in the art. For example, in transcatheter arterial embolization (TAE), selective necrosis of hepatocellular carcinoma is induced by infusing a mixture of an oil-based contrast medium (Lipiodol), a carcinostatic and an obstructing substance (Gelfoam) into the hepatic artery serving as the nutrient supply pathway to the tumor, and thereby obstructing the nutrient artery. On the other hand, in systemic chemotherapy, drugs such fluorouracil (5-FU), uracil-tegafur (UFT), mitomycin C (MMC), mitoxantrone (DHAD), adriamycin (ADR) (another name for which is doxorubicin (DXR)), epirubicin (EPI) or cisplatin (CDDP), are used alone or in combination with interferon (IFN). In addition, chemotherapeutic agents such as lapatinib having a mechanism of action that involves kinase inhibition are also preferably used as the chemotherapeutic agent in the present invention.

Sorafenib, which has a mechanism of action involving kinase inhibition, is also advantageously used as the chemotherapeutic agent in the present invention. Regardless of the mechanism of action, a chemotherapeutic agent advantageously used in the present invention may include those elicit side effects characteristic of chemotherapeutic agents when administered to a subject having liver cancer, such as diarrhea or constipation, anemia, suppression of the immune system (to a degree as to provoke infections or sepsis of lethal severity), hemorrhaging, cardiac toxicity, hepatic toxicity, renal toxicity, lack of appetite and weight loss.

Sorafenib (4-[4-[[4-chloro-3-(trifluoromethyl)phenyl]carbamoylamino]phenoxy]-N-methylpyridine-2-carboxamide) is an orally active, low-molecular-weight compound having a molecular weight of 464.7. Sorafenib tosylate (BAY 43-9006), the tosylic (toluenesulfonic) salt thereof, has been approved in Europe and the United States as a therapeutic agent for use in systemic chemotherapy for liver cancer. In addition to Sorafenib, Sunitinib (SU11248) shown to have a anti-tumor activity against hepatocarcinoma in a clinical test on hepatocarcinoma patients may also be employed as a chemotherapeutic agent in the present invention. Sorafenib or Sunitinib may also be preferably used as pharmaceutically acceptable salts thereof. Suitable examples of such salts include inorganic acid salts such as the hydrochloride, hydrobromide, hydroiodide, sulfate and phosphate; sulfonic acid salts such as the methanesulfonate, benzenesulfonate and toluenesulfonate; carboxylic acid salts such as the formate, acetate, oxalate, maleate, fumarate, citrate, malate, succinate, malonate, gluconate, mandelate, benzoate, salicylate, fluoroacetate, trifluoroacetate, tartrate, propionate and glutarate; alkali metal salts such as the lithium salt, sodium salt, potassium salt, cesium salt and rubidium salt; alkaline earth metal salts such as the magnesium salt and calcium salt; and ammonium salts such as the ammonium salt, alkylammonium salts, dialkylammonium salts, trialkylammonium salts and tetraalkylammonium salts. Of these, the use of BAY 43-9006, which is the tosylic (toluenesulfonic acid) salt, is especially preferred.

Either an oral or a parenteral route of administration may be preferably employed in chemotherapy, although the use of oral administration is preferred. The dosage form used for oral administration may be suitably selected from any dosage form such as liquid preparations, powders, granules, tablets, enteric-coated preparations and capsules. Chemotherapeutic agents having these dosage forms are prepared by a method known to persons of ordinary skill in the art. For example, preparation may be carried out by suitable combination with a pharmacologically acceptable carrier or solvent, such as sterilized water or physiological saline, vegetable oil, emulsifying agent, suspending agent, surfactant, stabilizer, fragrance, excipient, vehicle, preservative and binder, and intimate mixture in a unit dose form required for generally accepted pharmaceutical practice, followed by freeze drying, tabletting and other preparation-forming operations.

The chemotherapeutic agent may also be used parenterally in the form of an injection, such as a sterile solution or suspension in water or some other pharmacologically acceptable liquid. The amount of active ingredient in these preparations is suitably selected so as to enable the administration of a suitable dose within the indicated range. Sterile compositions for injection may be formulated in accordance with conventional pharmaceutical practice using a vehicle such as distilled water for injection. Exemplary aqueous solutions for injection include physiological saline, and isotonic solutions containing glucose and other adjuvants, such as D-sorbitol, D-mannose, D-mannitol and sodium chloride; concomitant use may be made of suitable soluble adjuvants, such as alcohols (e.g., ethanol, polyols such as propylene glycol and polyethylene glycol, and nonionic surfactants such as Polysorbate 80™ and HCO-50). Examples of oil-based liquids include sesame oil and soybean oil; concomitant use may be made of soluble adjuvants such as benzyl benzoate and benzyl alcohol. In addition, suitable formulation is possible with buffering agents (e.g., phosphate buffers, sodium acetate buffers), soothing agents (e.g., procaine hydrochloride), stabilizers (e.g., benzyl alcohol, phenol) and antioxidants.

Therapeutic Antibody

Any antibody which binds with proteins expressed in liver cancer cells and has the ability to elicit cytotoxicity against such cells may be suitably used as the therapeutic antibody in the pharmaceutical composition of the present invention. The proteins which are preferred as the target molecules for the antibody are proteins which are expressed on the surface of the liver cancer cells. From the standpoint of achieving antibody treatment effects, it is preferable that a higher number of target molecules are expressed on the cell surface, although such effects are not necessarily dependent on the number of molecules. It is desirable to select as the target a molecule which is specifically expressed in cancer cells as compared with expression in normal cells. A preferred example of such a protein is glypican 3.

Glypican 3 is one of the family of heparan sulfate proteoglycans present on cell surfaces. It has been suggested that glypican 3 may participate in cell division during development and in cancer cell proliferation, although its function is not yet well understood. It has been found that certain types of antibodies which bind to glypican 3 exhibit a cell growth-suppressing effect due to an antigen-dependent cellular cytotoxicity (abbreviated below as "ADCC activity") and a complement-dependent cellular cytotoxicity (abbreviated below as "CDC activity") (see WO 2003/000883). Moreover, it is known that the GC33 antibody which binds to a specific epitope exhibits greater ADCC and CDC activities against liver cancer cells (see WO 2006/006693). Preferably, anti-glypican 3 antibodies may be used in the liver cancer drug of the present invention. An example of such a preferred anti-glypican 3 antibody is the GC33 antibody (WO 2006/006693). The amino acid sequences of the variable regions of the H chains and the L chains in the GC33 antibody, are shown in SEQ ID NOs: 1 and 2, respectively.

The anti-glypican 3 antibody may be obtained from a hybridoma based on a known method (see WO 2006/006693). Alternatively, the anti-glypican 3 antibody may be created by genetic engineering. For example, a recombinant antibody may be produced using a genetic recombination technique that involves cloning the antibody gene from a hybridoma, inserting the gene into a suitable vector, and introducing the vector into a host (e.g., see Vandamme, A.M. et al.: Eur. J. Biochem. 192, 767-75 (1990)). Specifically, mRNA coding for the variable (V) region of the anti-glypican 3 antibody is isolated from a hybridoma which produces the anti-glypican 3 antibody. mRNA isolation is carried out by preparation from the hybridoma cells using a known method, such as guanidine ultracentrifugation (Chirgwin, J.M. et al.: Biochemistry 18, 5294-5299 (1979)) or the AGPC method (Chomczynski, P. et al: Anal. Biochem. 162, 156-159 (1987)), followed by preparation of the target mRNA using, for example, an mRNA Purification Kit (available from Pharmacia). Alternatively, the mRNA may be directly prepared from the hybridoma by using a QuickPrep mRNA Purification Kit (Pharmacia).

Using reverse transcriptase, cDNA for the antibody V region is synthesized from the mRNA thus obtained. cDNA synthesis may be carried out using, for example, an AMV Reverse Transcriptase First-strand cDNA Synthesis Kit (available from Seikagaku Corporation). Alternatively, preferable use may be made of, for example, the 5'-Ampli FINDER RACE Kit (Clontech) and the 5'-RACE method (Frohman, M.A. et al.: Proc. Natl. Acad. Sci. USA 85, 8998-9002 (1988); Belyavsky, A. et al.: Nucleic Acids Res. 17, 2919-2932 (1989)) which employs the polymerase chain reaction (PCR) to synthesize and amplify the cDNA. In the course of such cDNA synthesis, suitable restriction enzyme sites are introduced to both ends of the cDNA, as described below in detail. The resulting cDNA sequence is confirmed, then the cDNA coding for the V region of the target anti-glypican 3 antibody is inserted into an expression vector bearing the constant region (C region) of the desired antibody to be fused in-frame with the DNA coding for the C region.

To produce the anti-glypican 3 antibody used in the present invention, a region that controls expression of the antibody gene is integrated into the expression vector so that expression takes place under the control of, for example, enhancers and promoters. Then the host cell is transformed with the expression vector to obtain a recombinant cell which expresses DNA coding for anti-glypican 3 antibody.

Antibody gene expression may be carried out by separately integrating DNA coding for the antibody heavy chains (H chains) or light chains (L chains) into expression vectors and cotransforming the host cell, or by integrating DNA coding for the H chains and the L chains into a single expression vector and transforming the host cell (see WO 1994/011523).

In cases where the antibody gene is isolated and introduced into a suitable host to produce the antibody, a suitable host and expression vector combination may be preferably used. When a eukaryotic cell is used as the host, the use of an animal cell, plant cell or fungal cell is preferred. Illustrative examples of animal cells include (1) mammalian cells, such as CHO, COS, myeloma, baby hamster kidney (BHK), Hela and Vero; (2) amphibian cells such as African clawed frog (*Xenopus*) oocytes; and (3) insect cells, such as sf9, sf21 and Tn5. Illustrative examples of plant cells include cells derived from the genus *Nicotiana,* such as from *Nicotiana tabacum,* which cells are, for example, callus cultured. Illustrative examples of fungal cells include yeasts, such as those of the genus *Saccharomyces* (e.g., *Saccharomyces serevisiae*); and filamentous fungi, such as those of the genus *Aspergillus* (e.g., *Aspergillus niger*)*.* When a prokaryotic cell is to be used, it is preferable to use a production system that employs bacterial cells. Illustrative examples of suitable bacterial cells include *Escherichia coli* and *Bacillus subtilis.* The expression vector containing the target antibody gene is introduced into these cells via transformation and the transformed cells are cultured in vitro. The desired antibody can be obtained from the transformed cell culture.

The production of recombinant antibodies is not limited only to the above-described host cells, but transgenic animals may also preferably used. For example, the antibody gene can be constructed as a fused gene by inserting it in-frame into a gene coding for a protein primary produced in milk (e.g., goat β-casein). DNA fragments containing the fused gene in which the antibody gene is inserted are introduced into goat embryos, and the resulting embryos are implanted in a female goat. The desired antibodies can be obtained from milk produced by the transgenic goats born to the goats that received the embryos, or from the offspring of the transgenic goats. To increase the amount of milk containing the desired antibody that is produced by the transgenic goats, hormones may be suitably used in the transgenic goats (Ebert, K.M., et al.: Bio/Technology 12, 699-702 (1994)).

In the present invention, genetically recombinant antibodies which have been artificially modified in order to, for example, lower the heteroantigenicity to humans, such as chimeric antibodies and humanized antibodies, may be used. These modified antibodies may be produced using a known method. Chimeric antibody is an antibody composed of the heavy chain and light chain variable regions of a non-human mammalian antibody, such as a mouse antibody, and the heavy chain and the light chain constant regions of a human antibody. Such an chimeric antibody can be obtained by ligating DNA coding for the variable regions of a mouse antibody with DNA coding for the constant regions of a human antibody, and expressing the antibody in a suitable host. A preferred example of a humanized antibody is the hGC33 antibody (WO 2006/006693). The amino acid sequence of the H chain and L chain variable regions of the hGC33 antibody are shown as SEQ ID NOs: 3 and 4, respectively.

The C regions of human antibodies are used in the constant regions of chimeric antibodies and humanized antibodies. For example, Cγ1, Cγ2, Cγ3 and Cγ4 may be used as the H chains, and Cκ and Cλ may be used as the L chains. The sequences for these regions are known. To improve the antibody or the production stability thereof, the C regions of the human antibody may be modified.

Chimeric antibodies are composed of V regions from a non-human mammalian antibody and C regions from a human antibody. Humanized antibodies are composed of complementarity determining regions (CDR) from a non-human mammalian antibody, framework regions (FR) from a human antibody, and C regions from a human antibody. Because humanized antibodies will have lower antigenicity in the human body, they are useful as an active ingredient in the drug of the invention.

Humanized antibodies, which are also referred to as reshaped human antibodies, are obtained by, for example, replacing the CDR of a mouse antibody with the CDR of a human antibody. Common genetic recombination techniques for doing such replacement are known. Specifically, a DNA sequence is designed so that the CDR of a mouse antibody and the FR of a human antibody are fused in-frame, and synthesized by a PCR method using as the primers a plurality of oligonucleotides designed so as to have overlapping portions in their ends. A humanized antibody can be produced by inserting into an expression vector the DNA obtained as described above and DNA coding for the human antibody C region so that they fuse in-frame, and expressing the resulting DNA in a suitable host cell (see European Patent No. 239400 and WO 96/002576).

The human antibody-derived FR regions used in the production of humanized antibody are selected so that the CDR will form a good antigen binding site when ligated with the FR. The binding activity of the humanized antibody thus produced to the antigen is qualitatively or quantitatively measured and evaluated, and the FR of the human antibody can be suitably selected based on the binding activity. If necessary, the amino acids of the FR in the V region of the antibody may be substituted so that the CDR of the reshaped human antibody forms a suitable antigen-binding site. The above-mentioned amino acid substitution is easily introduced by a conventional PCR method. By measuring and evaluating the binding activity of the variant antibody having such an amino acid substitution, an modified FR sequence having the desired qualities is selected (Sato, K. et al.: Cancer Res. 53, 851-856 (1993)).

Methods for obtaining human antibodies are also known. For example, a desired human antibody having an antigen-binding activity can be obtained by sensitizing human lymphocytes in vitro with the desired antigen or cells that express the desired antigen, then fusing the sensitized lymphocytes with human myeloma cells, such as U266 (see Japanese Patent Publication No. H1-59878). Alternatively, the desired human antibodies may be obtained by immunizing a transgenic animal having the full repertoire of human antibody genes with the desired antigen (see International Publications WO 1993/012227, WO 1992/03918, WO 1994/002602, WO 1994/025585, WO 1996/034096 and WO 1996/033735). In addition, a technique for obtaining human antibodies by panning against a human antibody library is also known. For example, it is possible to express the V region of a human antibody as a single-chain antibody (scFv) at the surface of phages using the phage display technique, and select the phages which bind to the antigen. The DNA sequence coding for the V region of the human antibody which binds to the antigen can be determined by analyzing the genes of the selected phages. The DNA sequence of the scFv that binds to the antigen is determined and fused in-frame with the sequence for the C region of the desired human antibody. The fusion protein may be expressed in a suitable cell to obtain the human antibody. Such methods are already known in the art, and may be practiced by referring to International Publications WO 1992/001047, WO 1992/020791, WO 1993/006213, WO 1993/011236, WO 1993/019172, WO 1995/001438 and WO 1995/015388.

The antibody gene constructed as described above may be expressed and isolated by known methods. When a mammalian cell is used, the antibody gene can be expressed by operably combining a commonly used promoter, the antibody gene to be expressed and a poly A signal on the 3' downstream. A preferred example of a promoter/enhancer is the human cytomegalovirus immediate early promoter/enhancer. Other useful promoter/enhancers include virus promoter/enhancers of retroviruses, polyomaviruses, adenoviruses and simian virus 40 (SV40); and promoter/enhancers derived from mammalian cells such as human elongation factor 1α (HEF1α).

When the SV40 promoter/enhancer is used, gene expression may be easily carried out by the method of Mulligan et al. (Nature 277, 108 (1979)). When the HEF1α promoter/enhancer is used, gene expression may be easily carried out by the method of Mizushima et al. (Nucleic Acids Res. 18, 5322 (1990)).

When E. coli is used, the antibody gene may be expressed by operably combining a commonly used promoter, the signal sequence for antibody secretion and the antibody gene to be expressed. Preferred examples of the promoter include the lacZ promoter and the araB promoter. When the lacZ promoter is used, the gene is expressed by the method of Ward et al. (Nature 341, 544-546 (1989); FASEBJ. 6, 2422-2427 (1992)). When the araB promoter is used, the gene is expressed by the method of Better et al. (Science 240, 1041-1043 (1988)).

When the antibody is produced in the periplasm of E. coli, the pe1B signal sequence (Lei, S.P. et al.: J. Bacteriol. 169, 4379 (1987)) may be used as the signal sequence for antibody secretion. After the antibody produced in the periplasm has been isolated, the structure of the antibody may be refolded using a protein denaturant such as urea or guanidine hydrochloride so that the antibody has the desired binding activity.

The origin of replication to be inserted into the expression vector is preferably selected from, for example, SV40, polyomaviruses, adenoviruses and bovine papillomaviruses (BPV). Also, to amplify the number of gene copies in the host cell system, an aminoglycoside transferase (APH) gene, a thymidine kinase (TK) gene, an Escherichia coli xanthine guanine phosphoribosyl transferase (Ecogpt) gene or a dihydrofolate reductase (dhfr) gene may be preferably inserted as a selection marker into the expression vector.

To produce the antibody of the present invention, any expression system, such as a eukaryotic cell or a prokaryotic cell may be used. Preferred examples of eukaryotic cells include animal cells such as established mammalian cell lines and insect cell lines, as well as filamentous fungal cells and yeast cells. Preferred examples of prokaryotic cells include bacterial cells such as *E. coli* cells. The antibody used in the present invention is preferably expressed using mammalian cells, such as CHO, COS, myeloma, BHK, Vero or Hela cells.

Next, the transformed host cell is cultured in vitro or in vivo to produce the target antibody. Culture of the host cell is carried out in accordance with a known method, using as the culture medium, for example, DMEM, MEM, RPMI1640 or IMDM. A serum complement such as fetal calf serum (FCS) may be used together with the culture medium.

The antibody expressed and produced as described above may be purified by using one or a combination of known methods conventionally employed in the purification of proteins. For example, the antibody may be isolated and purified by suitably selecting and combining an affinity column such as a Protein A column, a chromatography column, filtration, ultrafiltration, salting out, dialysis and the like (Antibodies: A Laboratory Manual; Ed Harlow, David Lane (Cold Spring Harbor Laboratory, 1988)).

Antibodies having modified sugar chains may also be preferably used in the invention. It is known that the ADCC activity of an antibody can be enhanced by modifying the sugar chain of the antibody, which will be described below in detail. In cases where the expression in liver cancer cells of the antigen to which the antibody binds is not high enough to enable the ADCC activity to be strongly exhibited, an antibody having modified sugar chain is advantageous used. Known antibodies in which the sugar chain has been modified include, for example, antibodies with modified glycosylation (e.g., WO 1999/54342), antibodies deficient in the fucose added to the sugar chain (e.g., WO 2000/061739, WO 2002/031140), and antibodies having a sugar chain with a bisecting GlcNAc (e.g., WO 2002/079255).

The binding of an antibody to its target (i.e., antigen) can be suitably evaluated by using a known method. Specifically, the binding activity of an antibody to cells expressing the antigen may be measured by the techniques described on pages 359 to 420 of Antibodies: A Laboratory Manual, Ed Harlow and David Lane (Cold Spring Harbor Laboratory, 1988) based on ELISA and FACS (fluorescence activated cell sorting) using the cells as the antigen. In the ELISA format, the antibody binding activity to the cell is quantitatively determined by comparing the signal levels generated by an enzyme reaction. Specifically, the antibody being tested is added to an ELISA plate on which antigen-expressing cells have been immobilized, and antibody bound to the cells is detected by utilizing an enzyme-labeled secondary antibody capable of recognizing the test antibody. Alternatively, the binding activity of an antibody to the cells can be compared in FACS where a dilution series of the test antibody is prepared and the titer of the antibody binding to the antigen-expressing cells is determined.

In a FACS format, binding between an antibody and an antigen expressed on the surface of cells is measured in a suspension, instead of using cells bound to a carrier such as an ELISA plate. Flow cytometers employed in the FACS format include FACSCantO™ II, FACSAria™, FACSArray™, FACSVantage™ SE and FACSCalibur™ (all available from BD Biosciences); as well as EPICS ALTRA HyPerSort, Cytomics FC 500, EPICS XL-MCL ADC, EPICS XL ADC and Cell Lab Quanta / Cell Lab Quanta SC (all available from Beckman Coulter).

In one preferred method for measuring the binding activity of a test antibody to an antigen, a test antibody is reacted with antigen-expressing cells, stained with an FITC-labeled secondary antibody capable of recognizing the test antibody, and then the binding activity is measured with FACSCalibur (BD) and the fluorescent intensity is analyzed using CELL QUEST Software (BD). This method enables the binding activity of the test antibody to be assessed by comparing the Geometric Mean value obtained using the test antibody (test Geo-Mean value) with a control Geo-Mean value obtained using a control antibody. The formula for calculating the Geo-Mean value (geometric mean) is described in the CELL QUEST Software User's Guide (BD Biosciences).

An antibody capable of binding to the epitope to which the GC33 antibody is capable of binding may be advantageously used as the antibody in the present invention. The binding ability of the antibody of the invention to the epitope can be tested by the above-mentioned FACS or ELISA technique. To test if the test antibody binds to the same epitope as the epitope to which the GC33 antibody binds, i.e., if it shares an epitope with the GC33 antibody, competition between the two antibodies for the same epitope may be assayed. In the present invention, competition between antibodies can be determined by, for example, FACS or a cross-blocking assay. In FACS, first the GC33 antibody is bound to GPC3 expressing cells and the fluorescence signal is measured. Next, the candidate competing antibody is reacted, then the GC33 antibody is reacted with the same cells, and the signal is similarly analyzed by FACS. Alternatively, the GC33 antibody and the competing antibody being tested may be concurrently reacted with the same cells. If the FACS analysis pattern for the GC33 antibody changes in the presence of the competing antibody, it is said that the competing antibody recognizes the same epitope as the GC33 antibody. A cross-blocking assay may be carried out according to the method specifically described herein or the method known in the art as described in, for example, "Antibodies, A Laboratory Manual, Cold Spring Harbor Laboratory, Ed. Harlow and David Lane (1988).

For example, the competing ELISA assay is a preferable cross-blocking assay. In a cross-blocking assay, cells expressing the GPC3 protein are immobilized on microplate wells, pre-incubated in the presence or absence of the candidate competing antibody, then GC33 antibody is added to the wells. The amount of GC33 antibody which binds to the GPC3 protein-expressing cells in the wells is inversely correlated with the binding ability of the candidate competing antibody (test antibody) which competes to bind with the same epitope. That is, the greater the affinity of the test antibody to the same epitope, the lower the amount of GC33 antibody which binds to the wells where GPC3 protein-expressing cells are immobilized. Or, conversely, the greater the affinity of the test antibody to the same epitope, the higher the amount of the test antibody which binds to the wells where GPC3 protein-expressing cells are immobilized.

The amount of antibody that binds to the wells can be easily measured by previously labeling the antibody. For example, biotin-labeled antibody can be measured by using an avidin peroxidase conjugate and a suitable substrate. In particular, cross-blocking assays which use enzyme labeling with peroxidase or the like are referred to as "competitive ELISA assays." The antibody may be suitably labeled with another labeling substance which can be detected or measured, for example, radiolabeling and fluorescent labeling.

In addition, in cases where the test antibody has a constant region that originates from a different species than the GC33 antibody, an antibody bound to the wells can be measured using a labeled antibody which specifically recognizes the constant region originating from that species. When the antibody is one which originates from the same species but of a different class, the antibody bound to the wells can be advantageously measured by means of antibodies which specifically distinguish the respective classes.

As compared with the binding activity obtained in a control test carried out in the absence of the candidate competing antibody, when the candidate antibody is able to block at least 20%, preferably at least 20 to 50%, and more preferably at least 50%, of binding by the GC33 antibody, the candidate competing antibody is an antibody which binds to substantially the same epitope as the GC33 antibody or competes to bind to the same epitope.

An antibody which binds to substantially the same epitope or competes with the binding on the same epitope as the GC33 antibody may be conventionally selected by the cross-blocking assay as described above or by any other methods. Preferred examples may include, but not limited to:

an antibody comprising the H chain variable region comprising CDR1, 2 and 3 of: CDR1 comprising the amino acid sequence shown in SEQ ID NO:5, CDR2 comprising the amino acid sequence shown in SEQ ID NO:6, and CDR3 comprising the amino acid sequence shown in SEQ ID NO:7; and the L chain variable region comprising CDR1, 2 and 3 of: CDR1 comprising the amino acid sequence shown in any one of SEQ ID NOs:9-23, CDR2 comprising the amino acid sequence shown in SEQ ID NO:24, and CDR3 comprising the amino acid sequence shown in SEQ ID NO:25;

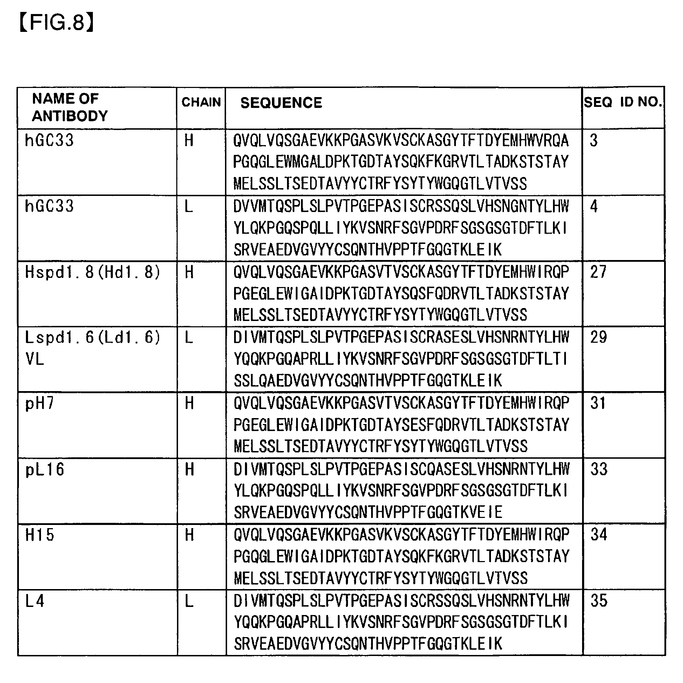

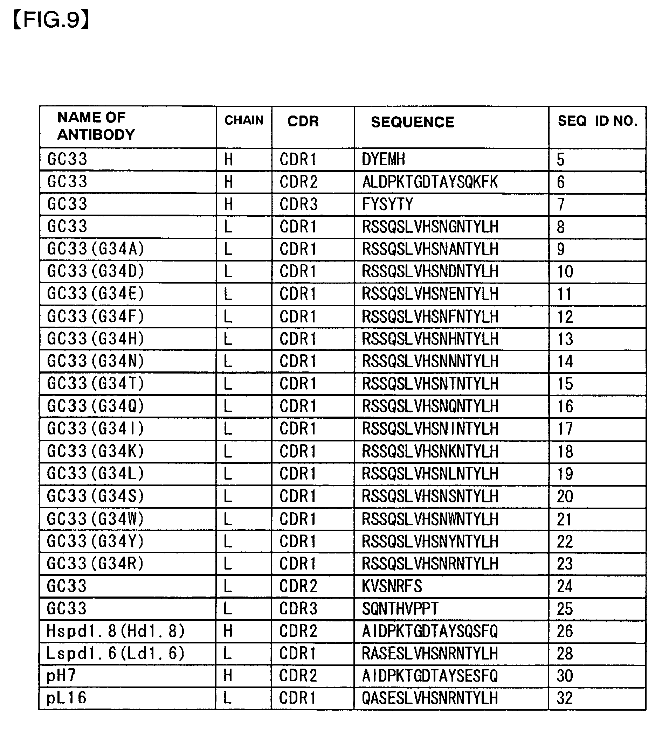

an antibody comprising the H chain variable region comprising CDR1, 2 and 3 of: CDR1 comprising the amino acid sequence shown in SEQ ID NO:5, CDR2 comprising the amino acid sequence shown in SEQ ID NO:26, and CDR3 comprising the amino acid sequence shown in SEQ ID NO:7; and the L chain variable region comprising CDR1, 2 and 3 of: CDR1 comprising the amino acid sequence shown in SEQ ID NO:28, CDR2 comprising the amino acid sequence shown in SEQ ID NO:24, and CDR3 comprising the amino acid sequence shown in SEQ ID NO:25; and

an antibody comprising the H chain variable region comprising CDR1, 2 and 3 of: CDR1 comprising the amino acid sequence shown in SEQ ID NO:5, CDR2 comprising the amino acid sequence shown in SEQ ID NO:30, and CDR3 comprising the amino acid sequence shown in SEQ ID NO:7; and the L chain variable region comprising CDR1, 2 and 3 of: CDR1 comprising the amino acid sequence shown in SEQ ID NO:32, CDR2 comprising the amino acid sequence shown in SEQ ID NO:24, and CDR3 comprising the amino acid sequence shown in SEQ ID NO:25. Figs. 8 and 9 show the amino acid sequence of the H chain- and the L chainvariable regions and CDRs of exemplary humanized antibodies which may be preferably used in the present invention.

The antibody used in the present invention has cytotoxicity. As used herein, "cytotoxicity" means that the antibody has an activity which causes damage to target cells that express the corresponding antigen. When the antigen-antibody complex is not internalized within the cell due to the nature of the antigen, preferred examples of the cytotoxicity exhibited by the antibody include antibody-dependent cellular cytotoxicity ("ADCC activity") and complement-dependent cellular cytotoxicity ("CDC activity"). On the other hand, when the antigen-antibody complex is internalized within the cell due to the nature of the antigen, a conjugated antibody composed of an antibody and a chemotherapeutic agent, a radioisotope or a toxic substance attached to the antibody may preferably used. In such a case, the cytotoxicity of the antibody is derived from the cytotoxicity exhibited by the chemotherapeutic agent, the radioisotope or the toxic substance attached to the conjugated antibody.

Known methods may be suitably used to measure whether the antibody used in the invention exhibits an ADCC activity and whether it exhibits a CDC activity (e.g., Current Protocols in Immunology, Chapter 7: Immunologic studies in humans; ed., John E. Coligan et al. (John Wiley & Sons, Inc.; 1993)).

First, preparation of the effector cells, complement solution and target cells is carried out according to the following procedures.

(1) Preparation of Effector Cells

Spleen is removed from CBA/N mice, and spleen cells are isolated in a RPMI1640 medium (Invitrogen). Effector cells can be prepared by rinsing the isolated spleen cells in the same medium containing 10% fetal bovine serum (FBS;

HyClone), and adjusting the cell concentration to $5 \times 10^6$/mL.

(2) Preparation of Complement Solution

A complement solution can be prepared by the 10-fold dilution of Baby Rabbit Complement (CEDARLANE) with a 10% FBS-containing medium (Invitrogen).

(3) Preparation of Target Cells

Antigen-expressing cells are incubated for 1 hour at 37°C in 0.2 mCi 51Cr sodium chromate (GE Healthcare Bio-Science) and 10% FBS-containing DMEM medium to radiolabel the target cells. Preferred examples of antigen-expressing cells that may be used in the invention include cells transformed by a gene coding for the antigen, primary hepatocellular carcinoma cells and metastatic hepatocellular carcinoma cells. After being radiolabeled, the cells are rinsed three times with a 10% FBS-containing RPMI1640 medium and the cell concentration is adjusted to $2 \times 10^5$ cells/mL to prepare the target cells.

The ADCC activity and the CDC activity can be measured by the methods described below. When measuring the ADCC activity, the target cells and the antibody according to the invention are added to a 96-well U-bottomed plate (Becton Dickinson) in an amount of 50 $\mu$L each per well, then reacted for 15 minutes on ice. Next, 100 $\mu$L of effector cells are added to the well. The plate is incubated for 4 hours in a carbon dioxide incubator. The final concentration of the antibody is set to 0 or 10 $\mu$g/mL, although the concentration is suitably adjusted based on the activity of the antibody. Following incubation, 100 $\mu$L of supernatant per well is removed, and the radioactivity is measured with a gamma counter (COBRAII AUTO-GAMMA, Model D5005; Packard Instrument Company). The radioactivity values obtained can be used to calculate the cytotoxicity (%) according to the formula (A-C)/(B-C) $\times$ 100

wherein A represents the radioactivity (cpm) of the sample, B represents the radioactivity (cpm) of a sample to which 1% NP-40 (Nakalai Tesque) has been added, and C represents the radioactivity (cpm) of a sample containing only the target cells.

When measuring the CDC activity, the target cells and the antibody according to the invention are added to a 96-well flat-bottomed plate (Becton Dickinson) in amounts of 50 $\mu$L each per well, then reacted for 15 minutes on ice. Next, 100 $\mu$L of the complement solution is added to the well. The plate is incubated for 4 hours in a carbon dioxide incubator. The final concentration of the antibody is set to 0 or 3 $\mu$g/mL, although the concentration is suitably adjusted based on the activity of the antibody. Following incubation, 100 $\mu$L of supernatant per well is removed and the radioactivity is measured with a gamma counter. The cytotoxicity may be calculated in the same way as the method used to measure the ADCC activity.

The cytotoxicity exhibited by the conjugated antibody can be preferably assessed by measuring the cytotoxicity exhibited by the chemotherapeutic agent, the radioisotope or the toxic substance attached to the antibody conjugate. When measuring the cytotoxicity exhibited by the chemotherapeutic agent, radioisotope or toxic substance attached to the conjugated antibody, the target cell and the conjugated antibody according to the invention are added to a 96-well flat-bottomed plate (Becton Dickinson) in an amount of 50 $\mu$L each per well, and reacted for 15 minutes on ice. Next, the plate is incubated for a period of from 1 to 4 hours in a carbon dioxide incubator. The final concentration of the antibody is set to 0 or 3 $\mu$g/mL, although the concentration is suitably adjusted based on the activity of the conjugated antibody. Following incubation, 100 $\mu$L of supernatant per well is removed, and the radioactivity is measured with a gamma counter. The cytotoxicity may be calculated in the same way as the method used to measure the ADCC activity.

Illustrative examples of chemotherapeutic agents which may be conjugated with the antibody of the invention and have a cytotoxic effect include:

azaribine, anastrozole, azacytidine, bleomycin, bortezomib, bryostatin-1, busulfan, camptothecin, 10-hydroxycamptothecin, carmustine, celebrex, chlorambucil, cisplatin, irinotecan, carboplatin, cladribine, cyclophosphamide, cytarabine, dacarbazine, docetaxel, dactinomycin, daunomycin glucuronide, daunorubicin, dexamethasone, diethylstilbestrol, doxorubicin, doxorubicin glucuronide, epirubicin, ethinyl estradiol, estramustine, etoposide, etoposide glucuronide, floxuridine, fludarabine, flutamide, fluorouracil, fluoxymesterone, gemcitabine, hydroxyprogesterone caproate, hydroxyurea, idarubicine, ifosfamide, leucovorin, lomustine, mechlorethamine, medroxyprogesterone acetate, megestrol acetate, melphalan, mercaptopurine, methotrexate, mitoxantrone, mithramycin, mitomycin, mitotane, phenylbutyrate, prednisone, procarbazine, paclitaxel, pentostatin, semustine, streptozocin, tamoxifen, taxanes, taxol, testosterone propionate, thalidomide, thioguanine, thiotepa, teniposide, topotecan, uracil mustard, vinblastine, vinorelbine and vincristine.

In the present invention, preferred chemotherapeutic agents are low-molecular-weight chemotherapeutic agents. Low-molecular-weight chemotherapeutic agents still have a low probability of interfering with antibody function after conjugation with the antibody. In the present invention, the low-molecular-weight chemotherapeutic agent typically has a molecular weight of from 100 to 2,000, and preferably from 200 to 1,000. The chemotherapeutic agents mentioned above are all low-molecular-weight chemotherapeutic agents. The chemotherapeutic agents used in the present invention include prodrugs which are converted in the body into active chemotherapeutic agents. Prodrugs can be preferably activated by enzymatic conversion or by non-enzymatic conversion.

Also, the antibody can be preferably modified using toxic peptides such as ricin, abrin, ribonuclease, onconase, DNase I, Staphylococcal enterotoxin-A, pokeweed antiviral protein, gelonin, diphtheria toxin, Pseudomonas exotoxin, Pseudomonas endotoxin, L-asparaginase and PEG L-asparaginase. In another aspect, one or two or more low-molecular-weight chemotherapeutic agent and a toxic peptide may be combined and used to modify the antibody. The antibody of the invention may be conjugated with the above-mentioned low-molecular-weight chemotherapeutic agent through a covalent bond or non-covalent bond. Methods for producing antibodies conjugated with a chemotherapeutic agent are known in the art.

In addition, a proteinaceous drug or toxin may be preferably conjugated to the antibody by a genetic engineering technique. Specifically, by fusing DNA coding for the above toxic peptide with DNA coding for the antibody of the invention and expressing the fused DNA in a suitable host cell. The antibody conjugated with a toxic peptide can be preferably obtained as a fused protein. A fused protein with the antibody is generally designed such that the proteinaceous drug or toxin is arranged at the C-terminus side of the antibody. A peptide linker may be preferably inserted between the antibody and the proteinaceous drug or toxin.

Pharmaceutical composition

The present invention provides a pharmaceutical composition for effectively treating or preventing liver cancer comprising a combination of a chemotherapy agent and an anti-glypican 3 antibody, as well as an effective method of treating liver cancer using the pharmaceutical composition. In another aspect, the invention provides a method for using an anti-glypican 3 antibody to enhance the therapeutic effects of a chemotherapeutic agent in the treatment of a liver cancer patient with the chemotherapeutic agent, and to reduce side effects caused by the chemotherapeutic agent. As used herein, the phrase "enhance the therapeutic effects" means that the response rate is improved, the amount of chemotherapeutic agent administered for treatment is reduced, and/or the period of treatment with the chemotherapeutic agent is shortened. In yet another aspect, the invention provides a method of using an anti-glypican 3 antibody to prepare a pharmaceutical composition for treating or preventing liver cancer, which composition comprises a chemotherapeutic agent and an anti-glypican 3 antibody as active ingredients. In addition, the invention provides a method of treatment or prevention for liver cancer patients using a chemotherapeutic agent and an anti-glypican 3 antibody.

In the present invention, the phrase "comprise a chemotherapeutic agent and/or an anti-glypican 3 antibody as an active ingredient" means to comprise a chemotherapeutic agent and/or an anti-glypican 3 antibody as the main active ingredient, although the content of the chemotherapeutic agent and/or the anti-glypican 3 antibody is not particularly limited. The term "treatment" means that, by administering the pharmaceutical composition of the invention to a subject, liver cancer cells are destroyed or the number of such cells is reduced, the growth of liver cancer cells is suppressed, or the various symptoms caused by liver cancer are ameliorated. The term "prevention" means to prevent an increase in the number of liver cancer cells due to regrowth or to prevent the regrowth of liver cancer cells of which growth had been suppressed.

The therapeutic antibody of the invention may be administered orally or parenterally. A parenteral administration is especially preferred. Illustrative examples of such methods of administration include administration by injection, nasal administration, pulmonary administration and percutaneous administration. Examples of administration by injection include intravenous injection, intramuscular injection, intraperitoneal administration and hypodermic injection, where the therapeutic antibody of the invention may be administered systemically or locally. The method of administration may be suitably selected according to the age and particular symptoms of the patient. The dose may be selected from a range of 0.0001 mg to 1,000 mg per kilogram of body weight per unit dose. Alternatively, the dose may be selected from a range of from 0.001 to 100,000 mg/body per patient. However, the dose of the therapeutic antibody of the invention is not limited to the doses mentioned above.

Combined use of the chemotherapeutic agent and the anti-glypican 3 antibody in the invention means that the chemotherapeutic agent and the anti-glypican 3 antibody are administered or used (indicated collectively below as simply "administered") together; and is not to be interpreted as limiting the order of administration, the dosing interval and the like. Also, the chemotherapeutic agent and anti-glypican 3 antibody of the invention may be used as a kit containing both ingredients. In accordance with the invention, the chemotherapeutic agent and the anti-glypican 3 antibody may be used in combination, if desired, at lower doses than each doses used alone.

The order of administration of the chemotherapeutic agent and the anti-glypican 3 antibody of the invention may include: first administering the anti-glypican 3 antibody then administering the chemotherapeutic agent; administering the chemotherapeutic agent and the anti-glypican 3 antibody simultaneously; or first administering the chemotherapeutic agent then administering the anti-glypican 3 antibody.

In cases where the chemotherapeutic agent and the anti-glypican 3 antibody of the invention are separately administered, the dosing interval of the chemotherapeutic agent and the anti-glypican 3 antibody may be selected with taking into consideration those factors including the route of administration and the dosage form. For example, the dosing interval is typically from 0 to 168 hours, preferably from 0 to 72 hours, more preferably from 0 to 24 hours, and even more preferably from 0 to 12 hours. Aside from factors such as the route of administration and the dosage form, the residual concentrations in the subject of the chemotherapeutic agent and the anti-glypican 3 antibody of the invention may also be taken into account. In cases where the chemotherapeutic agent is administered prior to administration of the anti-glypican 3 antibody, the anti-glypican 3 antibody may be administered while the residual concentration of the chemotherapeutic agent in the subject is sufficient to obtain the desired effect of the anti-glypican 3 antibody. The concentration can be determined by collecting a sample from the subject and analyzing the sample by any methods familiar to persons of ordinary skill in the art using a separatory apparatus such as any types of chromatographs.

Conversely, in cases where the anti-glypican 3 antibody is administered prior to administration of the chemotherapeutic agent, the chemotherapeutic agent may be administered while the residual concentration of the anti-glypican 3 antibody in the subject is sufficient to obtain the desired effect of the chemotherapeutic agent. The concentration can be determined by collecting a sample from the subject and analyzing the sample by an immunological measurement familiar to persons of ordinary skill in the art, such as the ELISA technique described below.

The therapeutic antibody of the invention may be formulated in accordance with a conventional method (see, for example, the latest edition of Remington's Pharmaceutical Science (Mack Publishing Company, Easton, USA), which may also comprise a pharmaceutically acceptable carrier and additive. Examples of additives that may be used include, but not limited to, surfactants, excipients, colorants, fragrances, preservatives, stabilizers, buffers, suspensions, tonicity agents, binders, disintegrants, lubricants, flow enhancers and flavorings. In addition, conventional carriers may be suitably used. Illustrative examples of such carriers include precipitated silica, lactose, crystalline cellulose, mannitol, starch, carmellose calcium, carmellose sodium, hydroxypropyl cellulose, hydroxypropylmethyl cellulose, polyvinyl acetal diethylaminoacetate, polyvinyl pyrrolidone, gelatin, medium chain fatty acid triglycerides, polyoxyethylene-hardened castor oil 60, white sugar, carboxymethylcellulose, corn starch and inorganic salts.

The entire contents of all patents and references cited in the specification are incorporated herein by reference in its entirety.

The present invention is described in detail in the following examples, which are illustrative only and are not to be construed as limiting the invention.

EXAMPLES

Example 1

Effects of combined use of anti-glypican 3 antibody and chemotherapeutic agent (mitoxantrone or doxorubicin hydrochloride) in mouse models implanted with glypican 3-expressing human liver cancer cell line

(1) Cell line

HuH-7 cells (Human Science Research Resource Bank) and HepG2 cells (ATCC) were used as the glypican 3-expressing human liver cancer cell lines. The HuH-7 cells were maintained and subcultured in Dulbecco's Modified Eagle's Medium (SIGMA) containing 10% FBS (BIONET), and the HepG2 cells were maintained and subcultured in Minimum Essential Medium Eagle medium (SIGMA) containing 10% FBS, 1 mmol/L MEM Sodium Pyruvate (Invitrogen) and 1 mmol/L MEM Non-Essential Amino Acid (Invitrogen).

(2) Preparation of Mouse Models Implanted with Human Liver Cancer Cells

Each type of cell was prepared at a cell count of $5 \times 10^7$ cells per milliliter of a solution containing equal amounts of the subculturing medium and MATRIGEL Matrix (BD Science). Next, 100 μL (i.e., $5 \times 10^6$ cells per mouse) of the cell suspension was implanted subcutaneously in the abdominal region of SCID mice (5-week-old male; CLEA Japan, Inc.) which, on the day prior to cell implantation, had been intraperitoneally administered with 100 μL of anti-asialo GM1 antibody (Wako Pure Chemical Industries, Ltd.; dissolved in 5 mL of fluid within one vial). The models were considered to have been established when, on calculating the tumor volume with the following formula, the average tumor volume became between 237 and 298 mm$^3$:

$$\text{tumor volume} = \text{major axis} \times \text{minor axis} \times \text{minor axis}/2$$

(3) Preparation of Antibody and Chemotherapeutic Agent

Mouse anti-human glypican 3 monoclonal antibody (clone name: GC33; described in WO 2006/006693) was prepared at a concentration of 0.5 mg/mL (5 mg/kg group) and 0.1 mg/mL (1 mg/kg group) using PBS(-). The doxorubicin hydrochloride (Adriacin Injection, available from Kyowa Hakko Kogyo Co., Ltd.) was dissolved at a concentration of 10 mg/mL in distilled water for injection (Otsuka Pharmaceutical Co., Ltd.), then diluted to a concentration of 0.5 mg/mL using PBS(-). The mitoxantrone hydrochloride (Novantrone Injection; available from Wyeth) was dissolved at 10 mg/mL in physiological saline (Otsuka Pharmaceutical Co., Ltd.), then diluted to a concentration of 0.1 mg/mL using PBS(-).

(4) Administration of Chemotherapeutic Agent

In the human liver cancer-implanted mouse model created in (2) above, 10 mL/kg doses of the antibody sample prepared in (3) above were administered through the caudal vein once per week over a period of three weeks starting on day 11 following implantation for the HuH-7 cell xenograft model, and starting on day 20 following implantation for the HepG2 cell xenograft model. As a negative control, 10 mL/kg doses of filtration sterilized PBS(-) (vehicle) were similarly administered once per week over a period of three weeks through the caudal vein. The HuH-7 cell xenograft model received a single 10 mL/kg dose, on day 10 following implantation, of the doxorubicin hydrochloride (DOX) prepared in (3) above or PBS(-) as a negative control, with each dose being administered through the caudal vein. The HepG2 cell xenograft model received a single 10 mL/kg dose per week for a period of three weeks, starting on day 20 following implantation, of the mitoxantrone hydrochloride (MX) prepared in (3) above or PBS(-) as a negative control, with each dose being administered through the caudal vein. Each group was composed of 6 animals. Details concerning administration of the chemotherapeutic agents are shown in Tables 1 and 2.

Table 1: HuH-7 Xenograft Model

| Group | Number of animals | Drug | Dose (mg/kg) | Method of administration | Days of administration |
|---|---|---|---|---|---|
| 1 | 6 | PBS(-) | -- | caudal vein | days 10, 17, 24 after implantation |
| | | PBS(-) | -- | caudal vein | day 10 after implantation |
| 2 | 6 | GC33 | 5 | caudal vein | days 10, 17, 24 after implantation |
| | | PBS(-) | -- | caudal vein | day 10 after implantation |
| 3 | 6 | PBS(-) | -- | caudal vein | days 10, 17, 24 after implantation |
| | | DOX | 5 | caudal vein | day 10 after implantation |
| 4 | 6 | GC33 | 5 | caudal vein | days 10, 17, 24 after implantation |
| | | DOX | 5 | caudal vein | day 10 after implantation |

Table 2: HepG2 Xenograft Model

| Group | Number of animals | Drug | Dose (mg/kg) | Method of administration | Days of administration |
|---|---|---|---|---|---|
| 1 | 6 | PBS(-) | -- | caudal vein | days 20, 27, 34 after implantation |
| | | PBS(-) | -- | caudal vein | days 20, 27, 34 after implantation |

(continued)

| Group | Number of animals | Drug | Dose (mg/kg) | Method of administration | Days of administration |
|---|---|---|---|---|---|
| 2 | 6 | GC33 | 1 | caudal vein | days 20, 27, 34 after implantation |
| | | PBS(-) | -- | caudal vein | days 20, 27, 34 after implantation |
| 3 | 6 | PBS(-) | -- | caudal vein | days 20, 27, 34 after implantation |
| | | MX | 1 | caudal vein | days 20, 27, 34 after implantation |
| 4 | 6 | GC33 | 1 | caudal vein | days 20, 27, 34 after implantation |
| | | MX | 1 | caudal vein | days 20, 27, 34 after implantation |

(5) Evaluation of Antitumor Effect

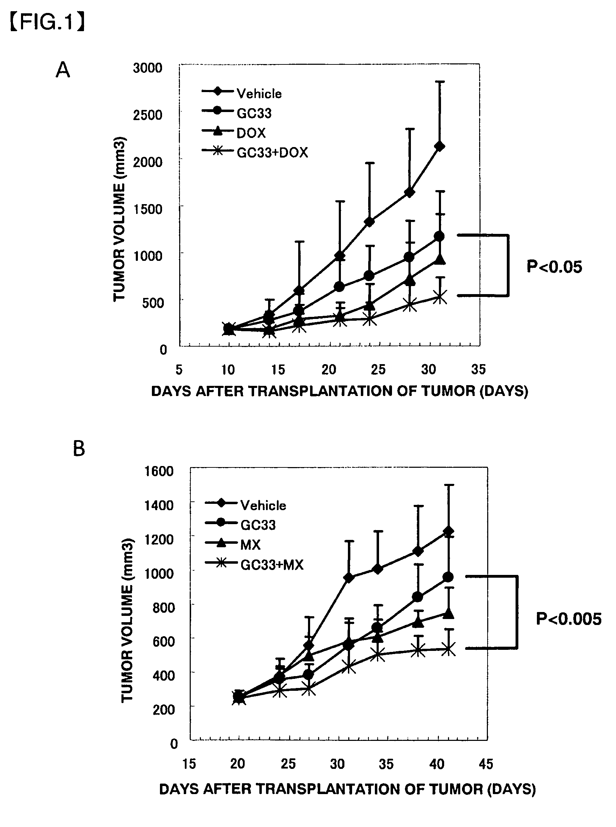

The antitumor effects of the combination of the GC33 antibody and a chemotherapeutic agent in human liver cancer implantation mouse models were evaluated based on the tumor volume one week following the final administration. Statistical analysis was carried out by the t-test using the tumor volumes on the final day of measurement. The SAS Preclinical Package (SAS Institute, Inc.) was used for statistical analysis. The results are shown in Fig. 1.

Fig. 1A is a graph showing the change in tumor volume when GC33 antibody and doxorubicin (DOX) were administered together in a mouse model implanted with the cells of the human liver cancer cell line HuH-7. The diamonds indicate the change in tumor volume in the group given the vehicle. The circles indicate the change in tumor volume in the group given only the GC33 antibody. The triangles indicate the change in tumor volume in the group given only doxorubicin (DOX). The asterisks indicate the change in tumor volume in the group in which the GC33 antibody and doxorubicin (DOX) were administered together. Fig. 1B is a graph showing the change in tumor volume when GC33 antibody and mitoxantrone (MX) were administered together in a mouse model implanted with the HepG2 cell line. The diamonds indicate the change in tumor volume in the group given the vehicle. The circles indicate the change in tumor volume in the group given only the GC33 antibody. The triangles indicate the change in tumor volume in the group given only mitoxantrone (MX). The asterisks indicate the change in tumor volume in the group in which the GC33 antibody and mitoxantrone (MX) were administered together.

As is apparent from Fig. 1, compared with tumor growth in the group given GC33 only, tumor growth in the groups given a combination of GC33 and doxorubicin (DOX, Fig. 1A) or mitoxantrone (MX, Fig. 1B) was significantly suppressed.

Example 2

Effects of concomitant use of anti-glypican 3 antibody and chemotherapeutic agent (Sorafenib) in mouse models implanted with glypican 3-expressing human liver cancer cell line

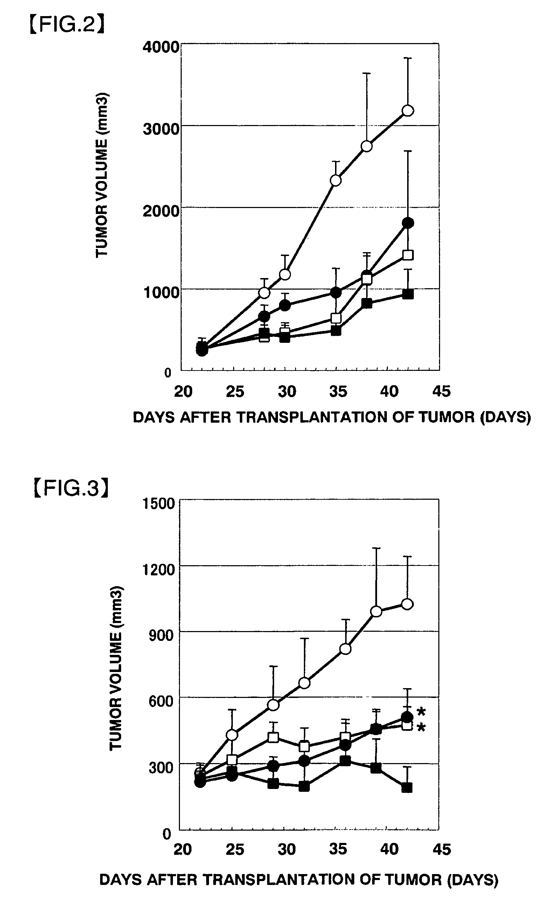

Six-week-old male CB-17 SCID mice were purchased from CLEA Japan, Inc. Prior to tumor implantation, the mice were intraperitoneally administered with 200 $\mu$g of anti-asialo GM antibody (WAKO). HepG2 cells or HuH-7 cells ($5 \times 10^5$ cells) dispersed in 50% Matrigel (Becton Dickinson) were subcutaneously implanted. When the tumor volume reached 250 mm$^3$, the mice were divided into groups and administration commenced. The humanized anti-glypican 3 antibody (hGC33, WO 2006/006693) was prepared at a suitable concentration in PBS(-), and administered intravenously once a week for 3 weeks. Sorafenib was synthesized according to the method described in Organic Process Research & Development 6, 777-781 (2002), and suspended in pure water containing 10% ethanol and 10% Cremophor EL, and was orally administered 5 times per week for 3 weeks. Pure water containing PBS(-), 10% ethanol and 10% Cremophor EL served for a vehicle control. The tumor volume V (mm$^3$) was calculated by the formula described in Example 1. The results are shown in Figs. 2 to 4.

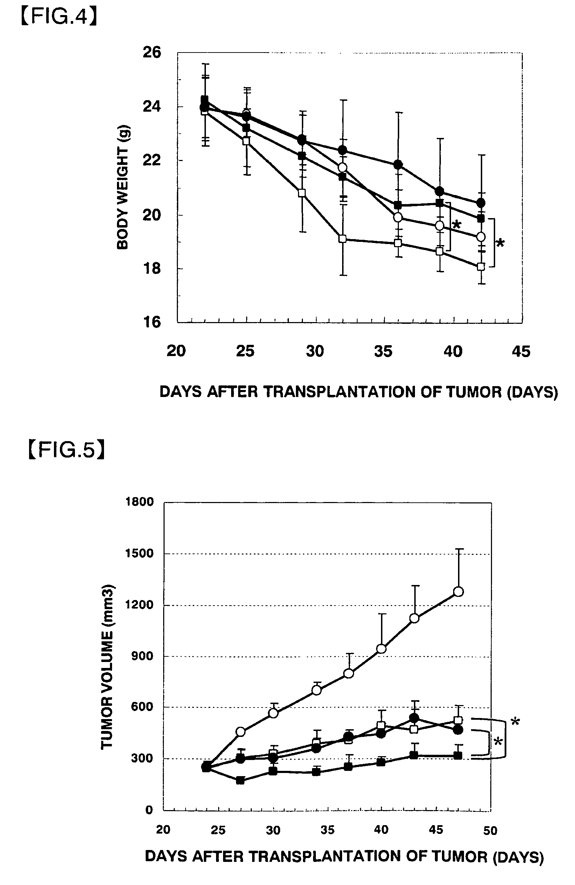

Fig. 2 is a graph showing the antitumor effects of the hGC33 antibody and Sorafenib on a mouse model implanted with cells of the human liver cancer cell line HuH-7 as indicated by the change in the tumor volume (average + standard deviation). The open circles indicate the change in tumor volume in the group given the vehicle. The closed circles indicate the change in tumor volume in the group given only hGC33 antibody in a dose of 5 mg/kg. The open squares indicate the change in tumor volume in the group given only Sorafenib in a dose of 80 mg/kg. The closed squares indicate the change in tumor volume in the group given a combination of hGC33 antibody in a dose of 5 mg/kg and Sorafenib in a dose of 80 mg/kg. Fig. 3 is a graph showing the antitumor effects of hGC33 antibody and Sorafenib on a mouse model implanted with cells of the human liver cancer cell line HepG2, as indicated by the change in the tumor volume (average + standard deviation). The open circles indicate the change in tumor volume in the group given the vehicle. The closed circles indicate the change in tumor volume in the group given only hGC33 antibody in a dose of 5 mg/kg. The open squares indicate the change in tumor volume in the group given only Sorafenib in a dose of 80 mg/kg. The closed squares indicate the change in tumor volume in the group given a combination of hGC33 antibody in a dose of 5 mg/kg and Sorafenib in a dose of 80 mg/kg. The asterisks in the figures indicate that $P < 0.05$, based on the Dunnett's test. Fig. 4 is a graph showing the effect of hGC33 antibody and Sorafenib on body weight loss in a mouse model implanted with cells of the human liver cancer cell line HepG2, as indicated by the change in weight of the model (average $\pm$ standard deviation). The open circles indicate the change in body weight in the group given the vehicle. The closed circles indicate the change in body weight in the group given only hGC33 antibody in a dose of 5 mg/kg. The open squares indicate the change in body weight in the group given only Sorafenib in a dose of 80 mg/kg. The closed squares indicate the change in body weight in the group given a combination of hGC33 antibody in a dose of 5 mg/kg and Sorafenib in a dose of 80 mg/kg. The asterisks in the figures indicate $P < 0.05$, based on the Dunnett's test.

As a result, tumor growth was suppressed in the HuH-7 xenograft model by administering 5 mg/kg of hGC33 alone or 80 mg/kg of Sorafenib alone. In addition, when both were administered together, the tumor growth was observed to be more markedly suppressed than the case where hGC33 or Sorafenib was administered alone (Fig. 2).

The tumor growth suppressing effect was indicated by the tumor volume on the final day of measurement (i.e., on day 42). In the HepG2 xenograft model, the tumor growth suppressing effect in the group given a combination of 1 mg/kg of humanized GC33 and 80 mg/kg (the maximum tolerated dose) of Sorafenib was significantly higher than that in the groups received either hGC33 or Sorafenib alone at the same doses. Weight loss is usually observed in this model associated with the tumor growth, and the weight loss was enhanced with administration of Sorafenib alone. When the combination of Sorafenib and humanized GC33 was administered, the weight loss was significantly attenuated (see, in particular, the results at days 39 and 42 (Fig. 3)) in addition to the enhancement of the efficacy compared to Sorafenib alone.

Reference Example 3

(1) Construction of point-mutation genes of the humanized H0L0 antibody

Various point-mutation genes were constructed starting from a gene encoding anti-glypican 3 antibody comprising the CDR of the humanized H0L0 antibody. Oligo DNAs designed based on the sequences of the sense and antisense chains containing the modification sites were synthesized. A plurality of point-mutation genes were constructed using the commercial QuikChange Site-Directed Mutagenesis Kit (Stratagene). Construction of the point-mutation genes was carried out by PCR under the following conditions. After heating for 30 seconds at 95°C, a reaction mixture of 10 ng template plasmid, 10 pmol forward chain and reverse chain synthetic oligo-DNAs and 10X buffer, dNTP mix, and Pfu Turbo DNA Polymerase provided with the kit was subjected to 18 cycles of 95°C 30 sec, 55°C 1 min and 68°C 4 min. The DpnI provided with the kit was added to the reaction mixture, and restriction digestion with the restriction enzyme was carried for 1 hour at 37°C. DH5$\alpha$ competent cells (Toyobo) were transformed with the resulting reaction solution to obtain transformants. The introduction of point mutation was confirmed by determining the nucleotide sequence of the plasmid DNA isolated from the transformants. Each point-mutation gene was cloned into expression vectors capable of expressing the insert gene in animal cells. Modified genes were prepared by modifications as described below.

Transient expression of the humanized H0L0 antibody and its point mutation-modified antibodies was carried out using polyethyleneimine (Polysciences Inc.). HEK293 cells were separated by trypsin EDTA (Invitrogen), and seeded to a 10 $cm^2$ culture dish at $6 \times 10^6$ cells/10 mL. The next day, SFMII culture medium and polyethyleneimine were mixed with a heavy chain expression plasmid DNA and a light chain expression plasmid DNA according to the manufacturer's instructions, and the resulting mixture was left stand for 10 minutes at room temperature. The entire mixture was added dropwise to the culture dish containing HEK293 cells seeded as described above. The culture supernatant was recovered after approximately 72 hours and the expressed humanized H0L0 antibody and its point mutation-modified antibodies were purified using rProteinA Sepharose™ Fast Flow (GE Healthcare) according to the manufacturer's instructions.

(1-1) Modification of the Tm value of the humanized H0L0 antibody

The thermal denaturation midpoint temperature (Tm) was determined by the top of the denaturation peak in the thermogram (Cp versus T) obtained after heating the test sample solution at a constant programmed heating rate. The Tm value of the humanized H0L0 antibody was measured using a sample solution for DSC measurement prepared as described in the following. The antibody solution (corresponding to 50 to 100 μg) filled in a dialysis membrane was first dialyzed for 24 hours against a dialysis external solution of 20 mol/L sodium acetate buffer solution (pH 6.0) containing 150 mmol/L sodium chloride. Subsequently, the sample solution was adjusted at its antibody concentration of 50 to 100 μg/mL with dialysis external solution and used as the sample solution for DSC measurement.

A suitable DSC instrument, for example, DSC-II (Calorimetry Sciences Corporation), is used for this experiment. The sample solution prepared as described above and the reference solution (dialysis external solution) were thoroughly degassed, and each of these test specimens was placed in a calorimeter cell and was thermally equilibrated at 40°C. A DSC scan was then run from 40°C to 100°C at a scan rate of approximately 1 K/minute. The results of this measurement are given as the top of the denaturation peak as a function of temperature. The thermal denaturation midpoint temperature of the humanized H0L0 antibody was calculated by peak assignment of the Fab domain according to Rodolfo et al., Immunology Letters (1999), 47-52.

The humanized H0L0 antibody, comprising the heavy chain shown in SEQ ID NO: 3 and the light chain shown in SEQ ID NO: 4, has a Tm value of 76.6°C as calculated by the method described above. The Tm values of Synagis and Herceptin, provided as examples of existing antibodies, are measured at 85.4°C and 81.8°C, respectively. It was thus shown that the Tm value of the humanized H0L0 antibody is lower than that of existing antibodies.

Modified antibodies were therefore prepared from humanized H0L0 antibody with the aim of raising the Tm value. Modifications of V37I, A40P, M48I, and L51I were introduced into FR2 of the humanized H0L0 antibody heavy chain shown in SEQ ID NO: 3 to prepare the antibody H15 (SEQ ID NO: 34), where its subclass was changed from VH1b to VH4. The Tm value was improved to 79.1°C. Also the humanized H0L0 antibody light chain shown in SEQ ID NO: 4 was modified by introducing L42Q, S48A, and Q50R modifications into the FR2 which changed the subclass from VK2 to VK3, and introducing V2I modification to replace the V2 of FR1 with I (germline sequence), thereby L4 (SEQ ID NO: 35) was prepared. The Tm value of each antibody was measured as described above. The Tm value of H15L0 and H0L4 was 79.2°C and 77.2°C, respectively, which shows improvement from the Tm value 76.6°C of H0L0. The Tm value of the H15L4 antibody comprising the combination of these two modifications was improved to 80.5°C.

(1-2) Modification of the pI value of the humanized H0L0 antibody

The plasma half-life of an antibody is extended by lowering the pI value exhibited by the antibody. Therefore, modified humanized H0L0 antibody with a lowered pI was prepared and evaluated whether the modification provides a higher tumor-suppressing activity.

The pI value of each antibody was calculated based on the analysis by isoelectric electrophoresis. Electrophoresis was carried out as described in the following. Using a PhastSystem Cassette (Amersham Bioscience), PhastGel Dry IEF (Amersham Bioscience) gel was swollen for about 60 minutes with a swelling solution with the composition given below.

(a) Composition of the swelling solution for high pI:

1.5 mL 10% glycerol
100 μL Pharmalyte 8-10.5 for IEF (Amersham Bioscience)

(b) Composition of the swelling solution for low pI: 1.5 mL purified water

20 μL Pharmalyte 8-10.5 for IEF (Amersham Bioscience) 80 μL Pharmalyte 5-8 for IEF (Amersham Bioscience)

Approximately 0.5 μg antibody was loaded on the swollen gel and isoelectric electrophoresis was run using the PhastSystem (Amersham Bioscience) controlled by the program described below. The sample was added to the gel at Step 2 of this program. A Calibration Kit for pI (Amersham Bioscience) was used for the pI markers.

Step 1: 2000 V, 2.5 mA, 3.5 W, 15°C, 75 Vh
Step 2: 200 V, 2.5 mA, 3.5 W, 15°C, 15 Vh
Step 3: 2000 V, 2.5 mA, 3.5 W, 15°C 410 Vh

After electrophoresis, the gel was fixed with 20% TCA and silver staining was then carried out using a Silver Staining Kit, Protein (Amersham Bioscience) according to the instructions provided with the kit. After staining, the isoelectric point of each antibody (test sample) was calculated based on the already known isoelectric points of the pI markers.

Hspd1.8 (Hd1.8) (SEQ ID NO: 27) was prepared, in which the K19T, Q43E, K63S, K65Q, and G66D modifications were additionally implemented in H15.Lspd1.6 (Ld1.6) (SEQ ID NO: 29) was prepared by making the following modifications: the Q27E modification in L4; modification of KISRVE at 79-84 of the FR3 in L4 to TISSLQ; and the S25A modification. The pI value of the Hspd1.8Lspd1.6 (Hd1.8Ld1.6) antibody composed of Hspd1.8 (Hd1.8) and Lspd1.6 (Ld1.6), was measured at 7.4. Since the pI of the humanized H0L0 antibody is 8.9, the pI of the Hspd1.8Lspd1.6 (Hd1.8Ld1.6) antibody was reduced by 1.5.

(2) Evaluation by competitive ELISA of the binding activity of the point-mutation modified antibodies from the humanized H0L0 antibody

The humanized H0L0 antibody and its point mutation-modified antibodies purified in (1) was evaluated by competitive ELISA. 100 $\mu$L of the soluble GPC3 core polypeptide (SEQ ID NO: 36) at 1 $\mu$g/mL was added to each well of a 96-well plate. The soluble GPC3 core polypeptide was immobilized on the plate by allowing the plate to stand overnight at 4°C. The soluble GPC3 core polypeptide immobilized on the plate was washed 3 times with a washing buffer using a Skan WASHER400 (Molecular Devices); and blocked with 200 $\mu$L blocking buffer at 4°C for at least 30 min. The blocked plate on which soluble GPC3 core polypeptide was immobilized was then washed 3 times with washing buffer using the Skan WASHER400. Subsequently, each well of the plate received 200 $\mu$L of a mixture containing 100 $\mu$L of biotinylated humanized H0L0 antibody (final concentration = 0.3 $\mu$g/mL) and 100 $\mu$L of the humanized H0L0 antibody or its point mutation-modified antibody (at various concentrations). The humanized H0L0 antibody was biotinylated using a Biotin Labeling Kit (Roche) according to the instructions provided with the kit. The plate was left stand for 1 hour at room temperature, then washed 5 times with washing buffer using the Skan WASHER400 (Molecular Devices). 100 $\mu$L goat anti-streptavidin alkaline phosphatase (ZYMED), diluted 20,000X with substrate buffer, was added to each well, and the resulting plate was left stand for 1 hour at room temperature, and then washed 5 times with washing buffer using the Skan WASHER400. Phosphatase Substrate (Sigma) was prepared at 1 mg/mL in the substrate buffer, added at 100 $\mu$L per well for 1 hour. The absorbance at 405 nm of the reaction solution in each well was measured using a Benchmark Plus (BIO-RAD), with the control absorbance at 655 nm.

The antigen binding activity of the H15L4 antibody and the Hspd1.8Lspd1.6 (Hd1.8Ld1.6) antibody was shown to be about the same as that of the humanized H0L0 antibody subjected to the modification.

Reference Example 4

(1) Selection of modification sites for decreasing pI for preparation of pI modified antibody by point mutation

To improve the tumor suppression activity of the Hd1.8Ld1.6 antibody, modification sites were selected for the ability of decreasing in the pI value of the variable region. Amino acid residues involving the decrease in the pI value of the variable region were found. A specific example of these modifications for decreasing the pI value is pH7pL16 antibody, which was prepared as follows.

The modification sites were created by Assemble PCR. Oligo DNAs designed based on the sense and antisense sequences containing the modification site were synthesized. A pair of an antisense oligo DNA containing the modification site and a sense oligo DNA corresponding to the vector bearing the gene to be modified, or a pair of a sense oligo DNA containing the modification site and an antisense oligo DNA corresponding to the vector bearing the gene to be modified was used in PCR with PrimeSTAR (TAKARA) to obtain 5'-side and 3'-side fragments containing the modification site. The two fragments were linked using Assemble PCR to prepare each mutant.

The mutant thus obtained was inserted into an expression vector which allows for expression of the inserted gene in animal cells. The nucleotide sequence of the expression vector was determined by a method known in the art. Introduction of the point mutation was confirmed by the nucleotide sequence of the plasmid DNA. The gene containing the point mutation was cloned into an expression vector which allows for expression of the inserted gene in animal cells. The expression and purification of the antibody was according to the method described in Example 1 or a similar method.

Starting from Hspd1.8 (Hd1.8), the 61st glutamine (Q) (according to the Kabat numbering) present in CDR2 of Hspd1.8 (Hd1.8) was substituted with glutamic acid (E) to prepare pH7 (SEQ ID NO:31). Starting from Ld1.6, the 24th arginine (R) (according to the Kabat numbering) present in CDR1 of Ld1.6 was substituted with glutamine (Q), the 37th glutamine (Q) was substituted with leucine (L), the 43rd alanine (A) was substituted with serine (S), the 45th arginine (R) was substituted with glutamine (Q), the 74th threonine (T) was substituted with lysine (K), the 77th serine (S) was substituted with arginine (R), the 78th leucine (L) was substituted with valine (V), and the 79th glutamine (Q) was substituted with glutamic acid (E), each present in FR2 and FR3, to prepare pL14. Then starting from PL14, the 104th leucine (L) (according to the Kabat numbering) was substituted with valine (V), the 107th lysine (K) was substituted with glutamic acid (E), each present in FR4 of pL14, to prepare pL16 (SEQ ID NO:33).

(2) Measurement of pI value of point mutation pI modified antibodies

The pI values of the Hd1.8Ld1.6 antibody and pH7pL16 antibody were measured by electrophoresis with PhastGel IEF 4-6.5 (GE Healthcase) using the method described in Reference Example 3 or similar method. The pI value of Hd1.8Ld1.6 antibody and pH7pL16 antibody was 7.47 and 6.52, respectively, indicating that the pI value of the pH7pL16 antibody was lower than that of the Hd1.8Ld1.6 antibody by 0.95.

(3) Measurement of Tm value of point mutation pI modified antibodies

The Tm values of Fabs obtained from Hd1.8Ld1.6 antibody and pH7pL16 antibody were measured with VP-DSC (Micro Cal) using the method similar to Reference Example 3. In this experiment, PBS was used as a dialysis solution, and the antibody concentration in the test solution for DSC measurement was adjusted to 25-100 $\mu$g/ml. DSC scanning was set from 20°C to 115 °C at the scanning rate of about 4K/min, with the reference solution (dialysis solution) and DSC measurement test solution. The thermal denaturation midpoint temperature of the Fabs of the Hd1.8Ld1.6 antibody and pH7pL16 antibody was 77.5 and 74.7 °C, respectively.

(4) Evaluation of binding activity to antigen of point mutation pI modified antibodies by competitive ELISA

The binding activity to the antigen glypican 3 of each point mutation pI modified antibody was measured using the method described in Reference Example 3. The binding activity to glypican 3 of the pH7pL16 antibody was shown to be comparative to that of the humanized H0L0 antibody.

Example 5

(1) Combination therapy test of a humanized anti-glypican 3 antibody and chemotherapeutic agent (Sorafenib) on a mouse model implanted with a human liver cancer cell line expressing glypican 3

Male CB-17 SCID mice of 6 weeks age were purchased from CLEA Japan Inc. Mice were intraperitoneally administered with 200$\mu$g of anti-asialo GM1 antibody (WAKO) just before tumor implant, then $5\times10^5$ HepG2 cells dispersed in 50% Matrigel (Becton Dickinson) were implanted subcutaneously. When the tumor volume reached to 250 mm$^3$, the mice was divided into groups and administration commenced. The antibody pH7pL16 prepared in PBS(-) at a suitable concentration was administered intravenously once a week for 3 weeks. Sorafenib was synthesized according to Organic Process Research & Development (2002) 6, 777-781. Sorafenib was suspended in pure water containing 10% ethanol and 10% Cremophor EL, and orally administered at 5 times per week for 3 weeks. Pure water containing PBS(-), 10% ethanol and 10% Cremophor EL served for a vehicle control. The tumor volume V (mm$^3$) was calculated by the formula described in Example 1.

(2) Combination therapy test results of a humanized anti-glypican 3 antibody and a chemotherapeutic agent (Sorafenib) on a mouse model implanted with a human liver cancer cell line expressing glypican 3

Fig. 5 is a graph showing the anti-tumor effect of a combination of the antibody pH7pL16 and Sorafenib on a mouse model implanted with a human liver cancer cell line HepG2. The data is expressed by the change in the tumor volume (mean $\pm$ SD). Open circle shows the vehicle group; closed circle shows the antibody pH7pL16 only at 1mg/kg; open square shows Sorafenib only at 80mg/kg; and closed square shows a combination of the antibody pH7pL16 at 1mg/kg and Sorafenib at 80mg/kg. Asterisk indicates P<0.05 based on the Dunnett's test.

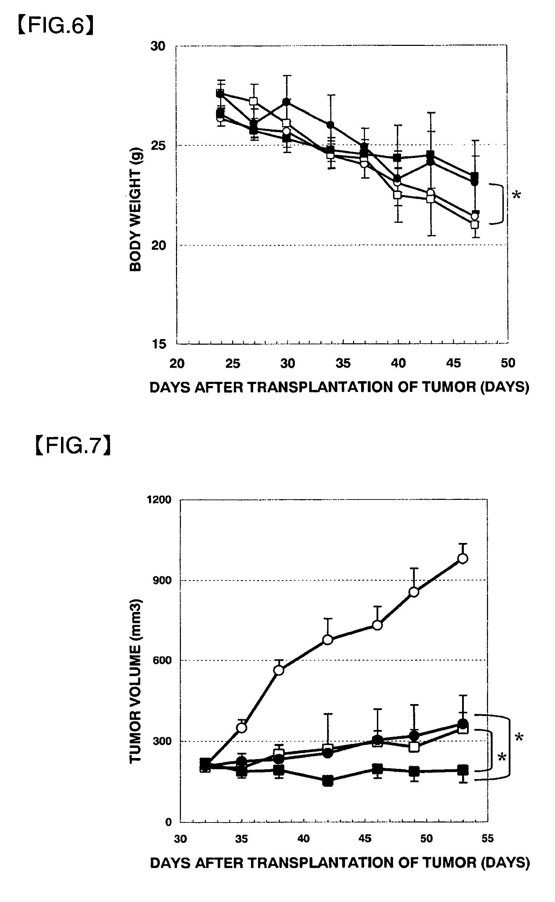

Fig. 6 is a graph showing the effect of a combination of the antibody pH7pL16 and Sorafenib on the weight loss in a mouse model implanted with a human liver cancer cell line HepG2. The data is expressed by the time course of the body weight of the mice (mean $\pm$SD). Open circle shows the vehicle group; closed circle shows the antibody pH7pL16 only at 1mg/kg; open square shows Sorafenib only at 80mg/kg; and closed square shows a combination of the antibody pH7pL16 at 1mg/kg and Sorafenib at 80mg/kg. Asterisk indicates P<0.05 based on the Dunnett's test.
The tumor growth suppressing effect was expressed as the tumor volume at the end of the test (day 47). In the HepG2 xenograft model, the tumor growth suppressing effect in the group received a combination of the antibody pH7pL16 (1mg/kg) and Sorafenib (maximum tolerance dose at 80mg/kg) was significantly higher than that in the group received the antibody alone or Sorafenib alone (Fig. 5). Weight loss is usually observed in this model associated with the tumor growth, and the weight loss was enhanced with administration of Sorafenib alone. When the combination of Sorafenib and the antibody pH7pL16 was administered, the weight loss was significantly attenuated (Fig. 6) in addition to the enhancement of the efficacy compared to Sorafenib alone.

Example 6

(1) Combination therapy test of a humanized anti-glypican 3 antibody and chemotherapeutic agent (Sunitinib) on a mouse model implanted with a human liver cancer cell line expressing glypican 3

Male CB-17 SCID mice of 6 weeks age were purchased from CLEA Japan Inc. Mice were intraperitoneally administered with 200µg of anti-asialo GM1 antibody (WAKO) just before tumor implant, then $5\times10^5$ HepG2 cells dispersed in 50% Matrigel (Becton Dickinson) were implanted subcutaneously. When the tumor volume reached to 250 mm$^3$, the mice was divided into groups and administration commenced.

A humanized anti-glypican 3 antibody (hGC33, W02006006693) prepared in PBS(-) at a suitable concentration was administered intravenously once a week for 3 weeks. Sunitinib (purchased from Sequoia Research Products; cat. # SRP01785S) was suspended in a pure water containing 10% ethanol and 10% Cremophor EL, and orally administered 5 times a week for 3 weeks. Pure water containing PBS(-), 10% ethanol and 10% Cremophor EL served for a vehicle control. The tumor volume V (mm$^3$) was calculated by the formula described in Example 1.

(2) Combination therapy test results of a humanized anti-glypican 3 antibody and a chemotherapeutic agent (Sunitinib) on a mouse model implanted with a human liver cancer cell line expressing glypican 3

Fig. 7 is a graph showing the anti-tumor effect of a combination of the antibody hCG33 and Sunitinib on a mouse model implanted with a human liver cancer cell line HepG2. The data is expressed by the change in the tumor volume (mean $\pm$ SD). Open circle shows the vehicle group; closed circle shows the antibody hCG33 only at 1mg/kg; open square shows Sunitinib only at 80mg/kg; and closed square shows a combination of the antibody hCG33 at 1mg/kg and Sunitinib at 80mg/kg. Asterisk indicates P<0.05 based on the Dunnett's test.

The tumor growth suppressing effect was expressed as the tumor volume at the end of the test (day 53). In the HepG2 xenograft model, the tumor growth suppressing effect in the group received a combination of the antibody hGC33 (1mg/kg) and Sunitinib (maximum tolerance dose at 80mg/kg) was significantly higher than that in the group received the antibody alone or Sorafenib alone (Fig. 7).

**Claims**

1. A pharmaceutical composition for treating or preventing a liver cancer comprising a combination of a chemotherapeutic agent and an anti-glypican 3 antibody.

2. The pharmaceutical composition according to Claim 1, wherein the pharmaceutical composition is a combination preparation.

3. The pharmaceutical composition according to Claim 1, wherein the chemotherapeutic agent and the anti-glypican 3 antibody are concomitantly administered.

4. The pharmaceutical composition according to Claim 3, wherein the chemotherapeutic agent and the anti-glypican 3 antibody are administered simultaneously or sequentially.

5. The pharmaceutical composition according to Claim 3, wherein the chemotherapeutic agent and the anti-glypican 3 antibody are administered separately.

6. A pharmaceutical composition for treating or preventing a liver cancer for use in combination with a chemotherapeutic agent, said composition comprising an anti-glypican 3 antibody as an active ingredient.

7. The pharmaceutical composition according to Claim 6, wherein the anti-glypican 3 antibody is administered simultaneously with the chemotherapeutic agent.

8. The pharmaceutical composition according to Claim 6, wherein the anti-glypican 3 antibody is administered before or after administration of the chemotherapeutic agent.

9. A pharmaceutical composition for treating or preventing a liver cancer for use in combination with an anti-glypican 3 antibody, said composition comprising a chemotherapeutic agent as an active ingredient.

10. The pharmaceutical composition according to Claim 9, wherein the chemotherapeutic agent is administered simultaneously with the anti-glypican 3 antibody.

11. The pharmaceutical composition according to Claim 9, wherein the chemotherapeutic agent is administered before or after administration of the anti-glypican 3 antibody.

12. The pharmaceutical composition according to any one of Claims 1-11, wherein the chemotherapeutic agent is a kinase inhibitor.

13. The pharmaceutical composition according to Claim 12, wherein the chemotherapeutic agent is a multi-kinase inhibitor.

14. The pharmaceutical composition according to Claim 12 or 13, wherein the chemotherapeutic agent is Sorafenib (BAY43-9006).

15. The pharmaceutical composition according to Claim 12 or 13, wherein the chemotherapeutic agent is Sunitinib.

16. The pharmaceutical composition according to any one of Claims 1-15, wherein the anti-glypican 3 antibody has cytotoxicity.

17. The pharmaceutical composition according to any one of Claims 1-16, wherein the anti-glypican 3 antibody comprises:

    the H chain variable region comprising the CDR1, 2 and 3 of:

        CDR1 comprising the amino acid sequence shown in SEQ ID NO:5,
        CDR2 comprising the amino acid sequence shown in SEQ ID NO:6, and
        CDR3 comprising the amino acid sequence shown in SEQ ID NO:7; and
        the L chain variable region comprising the CDR1, 2 and 3 of:

CDR1 comprising the amino acid sequence shown in SEQ ID NO:8,
CDR2 comprising the amino acid sequence shown in SEQ ID NO:24, and
CDR3 comprising the amino acid sequence shown in SEQ ID NO:25.

18. The pharmaceutical composition according to any one of Claims 1-17, wherein the anti-glypican 3 antibody is capable of binding to an epitope to which a second antibody can bind, wherein said second antibody comprises:

the H chain variable region comprising CDR1, 2, and 3 comprising the amino acid sequence shown in SEQ ID NOs:5, 6, and 7, respectively, and
the L chain variable region comprising CDR1, 2, and 3 comprising the amino acid sequence shown in SEQ ID NOs:8, 24, and 25, respectively.

19. The pharmaceutical composition according to any one of Claims 1-16 or 18, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of:

CDR1 comprising the amino acid sequence shown in any one of SEQ ID NOs:9-23,
CDR2 comprising the amino acid sequence shown in SEQ ID NO:24, and
CDR3 comprising the amino acid sequence shown in SEQ ID NO:25.

20. The pharmaceutical composition according to any one of Claims 1-16 or 18, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:26, and
CDR3 comprising the amino acid sequence shown in SEQ ID NO:7; and
the L chain variable region comprising CDR1, 2 and 3 of:

CDR1 comprising the amino acid sequence shown in SEQ ID NO:28,
CDR2 comprising the amino acid sequence shown in SEQ ID NO:24, and
CDR3 comprising the amino acid sequence shown in SEQ ID NO:25.

21. The pharmaceutical composition according to any one of Claims 1-16 or 18, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:30, and
CDR3 comprising the amino acid sequence shown in SEQ ID NO:7; and
the L chain variable region comprising CDR1, 2 and 3 of:

CDR1 comprising the amino acid sequence shown in SEQ ID NO:32,
CDR2 comprising the amino acid sequence shown in SEQ ID NO:24, and
CDR3 comprising the amino acid sequence shown in SEQ ID NO:25.

22. The pharmaceutical composition according to any one of Claims 1-21, wherein the anti-glypican 3 antibody is a humanized antibody.

23. The pharmaceutical composition according to Claim 22, wherein the anti-glypican 3 antibody comprises:

the H chain variable region comprising the amino acid sequence shown in SEQ ID NO:3; and
the L chain variable region comprising the amino acid sequence shown in SEQ ID NO:4.

24. The pharmaceutical composition according to Claim 22, wherein the anti-glypican 3 antibody comprises:

the H chain variable region comprising the amino acid sequence shown in SEQ ID NO:3; and
the L chain variable region comprising the amino acid sequence wherein the 34th Gly of SEQ ID NO.4 is substituted with another amino acid residue.

25. The pharmaceutical composition according to Claim 22, wherein the anti-glypican 3 antibody comprises:

the H chain variable region comprising the amino acid sequence shown in SEQ ID NO:27; and
the L chain variable region comprising the amino acid sequence shown in SEQ ID NO:29.

26. The pharmaceutical composition according to Claim 22, wherein the anti-glypican 3 antibody comprises:

the H chain variable region comprising the amino acid sequence shown in SEQ ID NO:31; and
the L chain variable region comprising the amino acid sequence shown in SEQ ID NO:33.

27. An agent for reducing a side-effect caused by a treatment of a liver cancer by chemotherapeutic agent, said agent comprising a therapeutic antibody as an active ingredient.

28. The side-effect reducing agent according to Claim 27, wherein the chemotherapeutic agent is a kinase inhibitor.

29. The side-effect reducing agent according to Claim 27 or 28, wherein the chemotherapeutic agent is a multi-kinase inhibitor.

30. The side-effect reducing agent according to Claim 28 or 29, wherein the chemotherapeutic agent is Sorafenib (BAY43-9006).

31. The side-effect reducing agent according to Claim 28 or 29, wherein the chemotherapeutic agent is Sunitinib.

32. The side-effect reducing agent according to any one of Claims 27-31, wherein the side effect is weight loss.

33. The side-effect reducing agent according to any one of Claims 27-32, wherein the therapeutic antibody is an anti-glypican 3 antibody.

34. The side-effect reducing agent according to Claim 33, wherein the anti-glypican 3 antibody has cytotoxicity.

35. The side-effect reducing agent according to Claim 33 or 34, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of:

CDR1 comprising the amino acid sequence shown in SEQ ID NO:8,
CDR2 comprising the amino acid sequence shown in SEQ ID NO:24, and
CDR3 comprising the amino acid sequence shown in SEQ ID NO:25.

36. The side-effect reducing agent according to any one of Claims 33-35, wherein the anti-glypican 3 antibody is capable of binding to an epitope to which a second antibody can bind, wherein said second antibody comprises:

the H chain variable region comprising CDR1, 2, and 3 comprising the amino acid sequence shown in SEQ ID NOs:5, 6, and 7, respectively, and
the L chain variable region comprising CDR1, 2, and 3 comprising the amino acid sequence shown in SEQ ID

# EP 2 275 135 A1

NOs:8, 24, and 25, respectively.

**37.** The side-effect reducing agent according to any one of Claims 33, 34 or 36, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of:

CDR1 comprising the amino acid sequence shown in any one of SEQ ID NOs:9-23,
CDR2 comprising the amino acid sequence shown in SEQ ID NO:24, and CDR3 comprising the amino acid sequence shown in SEQ ID NO:25.

**38.** The side-effect reducing agent according to any one of Claims 33, 34 or 36, wherein the anti-glypican 3 antibody comprises the H chain variable region comprising CDR1, 2 and 3 of:

CDR1 comprising the amino acid sequence shown in SEQ ID NO:5,
CDR2 comprising the amino acid sequence shown in SEQ ID NO:26, and
CDR3 comprising the amino acid sequence shown in SEQ ID NO:7; and
the L chain variable region comprising CDR1, 2 and 3 of:

CDR1 comprising the amino acid sequence shown in SEQ ID NO:28,
CDR2 comprising the amino acid sequence shown in SEQ ID NO:24, and
CDR3 comprising the amino acid sequence shown in SEQ ID NO:25.

**39.** The side-effect reducing agent according to any one of Claims 33, 34 or 36, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:30, and
CDR3 comprising the amino acid sequence shown in SEQ ID NO:7; and
the L chain variable region comprising CDR1, 2 and 3 of:

CDR1 comprising the amino acid sequence shown in SEQ ID NO:32,
CDR2 comprising the amino acid sequence shown in SEQ ID NO:24, and
CDR3 comprising the amino acid sequence shown in SEQ ID NO:25.

**40.** The side-effect reducing agent according to any one of Claims 33-39, wherein the anti-glypican 3 antibody is a humanized antibody.

**41.** The side-effect reducing agent according to Claim 40, wherein the anti-glypican 3 antibody comprises:

the H chain variable region comprising the amino acid sequence shown in SEQ ID NO:3; and
the L chain variable region comprising the amino acid sequence shown in SEQ ID NO:4.

**42.** The side-effect reducing agent according to Claim 40, wherein the anti-glypican 3 antibody comprises:

the H chain variable region comprising the amino acid sequence shown in SEQ ID NO:3; and
the L chain variable region comprising the amino acid sequence wherein the 34th Gly of SEQ ID NO:4 is substituted with another amino acid residue.

**43.** The side-effect reducing agent according to Claim 40, wherein the anti-glypican 3 antibody comprises:

54

the H chain variable region comprising the amino acid sequence shown in SEQ ID NO:27; and
the L chain variable region comprising the amino acid sequence shown in SEQ ID NO:29.

44. The side-effect reducing agent according to Claim 40, wherein the anti-glypican 3 antibody comprises:

the H chain variable region comprising the amino acid sequence shown in SEQ ID NO:31; and
the L chain variable region comprising the amino acid sequence shown in SEQ ID NO:33.

45. A pharmaceutical composition for enhancing the efficacy of treatment of a liver cancer by a chemotherapeutic agent, said composition comprising an anti-glypican 3 antibody as an active ingredient.

46. The pharmaceutical composition according to Claim 45, wherein the anti-glypican 3 antibody is administered simultaneously with the chemotherapeutic agent.

47. The pharmaceutical composition according to Claim 45, wherein the anti-glypican 3 antibody is administered before or after administration of the chemotherapeutic agent.

48. The pharmaceutical composition according to any one of Claims 45-47, wherein the chemotherapeutic agent is a kinase inhibitor.

49. The pharmaceutical composition according to Claim 48, wherein the chemotherapeutic agent is a multi-kinase inhibitor.

50. The pharmaceutical composition according to any one of Claims 45-49, wherein the chemotherapeutic agent is Sorafenib (BAY43-9006).

51. The pharmaceutical composition according to any one of Claims 45-49, wherein the chemotherapeutic agent is Sunitinib.

52. The pharmaceutical composition according to any one of Claims 45-51, wherein the anti-glypican 3 antibody has cytotoxicity.

53. The pharmaceutical composition according to any one of Claims 45-52, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of:

CDR1 comprising the amino acid sequence shown in SEQ ID NO:8,
CDR2 comprising the amino acid sequence shown in SEQ ID NO:24, and
CDR3 comprising the amino acid sequence shown in SEQ ID NO:25.

54. The pharmaceutical composition according to any one of Claims 45-53, wherein the anti-glypican 3 antibody is capable of binding to an epitope to which a second antibody can bind, wherein said second antibody comprises:

the H chain variable region comprising CDR1, 2, and 3 comprising the amino acid sequence shown in SEQ ID NOs:5, 6, and 7, respectively, and
the L chain variable region comprising CDR1, 2, and 3 comprising the amino acid sequence shown in SEQ ID NOs:8, 24, and 25, respectively.

55. The pharmaceutical composition according to any one of Claims 45-52 or 54, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of:

CDR1 comprising the amino acid sequence shown in any one of SEQ ID NOs:9-23,
CDR2 comprising the amino acid sequence shown in SEQ ID NO:24, and
CDR3 comprising the amino acid sequence shown in SEQ ID NO:25.

56. The pharmaceutical composition according to any one of Claims 45-52 or 54, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:26, and
CDR3 comprising the amino acid sequence shown in SEQ ID NO:7; and
the L chain variable region comprising CDR1, 2 and 3 of:

CDR1 comprising the amino acid sequence shown in SEQ ID NO:28,
CDR2 comprising the amino acid sequence shown in SEQ ID NO:24, and
CDR3 comprising the amino acid sequence shown in SEQ ID NO:25.

57. The pharmaceutical composition according to any one of Claims 45-52 or 54, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:30, and
CDR3 comprising the amino acid sequence shown in SEQ ID NO:7; and
the L chain variable region comprising CDR1, 2 and 3 of:

CDR1 comprising the amino acid sequence shown in SEQ ID NO:32,
CDR2 comprising the amino acid sequence shown in SEQ ID NO:24, and
CDR3 comprising the amino acid sequence shown in SEQ ID NO:25.

58. The pharmaceutical composition according to any one of Claims 45-57, wherein the anti-glypican 3 antibody is a humanized antibody.

59. The pharmaceutical composition according to Claim 58, wherein the anti-glypican 3 antibody comprises:

the H chain variable region comprising the amino acid sequence shown in SEQ ID NO:3; and
the L chain variable region comprising the amino acid sequence shown in SEQ ID NO:4.

60. The pharmaceutical composition according to Claim 58, wherein the anti-glypican 3 antibody comprises:

the H chain variable region comprising the amino acid sequence shown in SEQ ID NO:3; and
the L chain variable region comprising the amino acid sequence wherein the 34th Gly of SEQ ID NO:4 is substituted with another amino acid residue.

61. The pharmaceutical composition according to Claim 58, wherein the anti-glypican 3 antibody comprises:

the H chain variable region comprising the amino acid sequence shown in SEQ ID NO:27; and
the L chain variable region comprising the amino acid sequence shown in SEQ ID NO:29.

62. The pharmaceutical composition according to Claim 58, wherein the anti-glypican 3 antibody comprises:

the H chain variable region comprising the amino acid sequence shown in SEQ ID NO:31; and
the L chain variable region comprising the amino acid sequence shown in SEQ ID NO:33.

63. A method for treating or preventing a liver cancer in a subject comprising administering to the subject a combination of an effective amount of a chemotherapeutic agent and an anti-glypican 3 antibody.

64. The method according to Claim 63, wherein the chemotherapeutic agent and the anti-glypican 3 antibody are administered simultaneously or sequentially.

65. The method according to Claim 63, wherein the chemotherapeutic agent and the anti-glypican 3 antibody are administered separately.

66. The method according to any one of Claims 63-65, wherein the chemotherapeutic agent is a kinase inhibitor.

67. The method according to Claim 66, wherein the chemotherapeutic agent is a multi-kinase inhibitor.

68. The method according to Claim 66 or 67, wherein the chemotherapeutic agent is Sorafenib (BAY43-9006).

69. The method according to Claim 66 or 67, wherein the chemotherapeutic agent is Sunitinib.

70. The method according to any one of Claims 63-69, wherein the anti-glypican 3 antibody has cytotoxicity.

71. The method according to any one of Claims 63-70, wherein the anti-glypican 3 antibody comprises:

the H chain variable region comprising the CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising the CDR1, 2 and 3 of:

CDR1 comprising the amino acid sequence shown in SEQ ID NO:8,
CDR2 comprising the amino acid sequence shown in SEQ ID NO:24, and
CDR3 comprising the amino acid sequence shown in SEQ ID NO:25.

72. The method according to any one of Claims 63-71, wherein the anti-glypican 3 antibody is capable of binding to an epitope to which a second antibody can bind, wherein said second antibody comprises:

the H chain variable region comprising CDR1, 2, and 3 comprising the amino acid sequence shown in SEQ ID NOs:5, 6, and 7, respectively, and
the L chain variable region comprising CDR1, 2, and 3 comprising the amino acid sequence shown in SEQ ID NOs:8, 24, and 25, respectively.

73. The method according to any one of Claims 63-70 or 72, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of:

CDR1 comprising the amino acid sequence shown in any one of SEQ ID NOs:9-23,
CDR2 comprising the amino acid sequence shown in SEQ ID NO:24, and
CDR3 comprising the amino acid sequence shown in SEQ ID NO:25.

74. The method according to any one of Claims 63-70 or 72, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:26, and
CDR3 comprising the amino acid sequence shown in SEQ ID NO:7; and
the L chain variable region comprising CDR1, 2 and 3 of:

CDR1 comprising the amino acid sequence shown in SEQ ID NO:28,
CDR2 comprising the amino acid sequence shown in SEQ ID NO:24, and
CDR3 comprising the amino acid sequence shown in SEQ ID NO:25.

75. The method according to any one of Claims 63-70 or 72, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:30, and
CDR3 comprising the amino acid sequence shown in SEQ ID NO:7; and
the L chain variable region comprising CDR1, 2 and 3 of:

CDR1 comprising the amino acid sequence shown in SEQ ID NO:32,
CDR2 comprising the amino acid sequence shown in SEQ ID NO:24, and
CDR3 comprising the amino acid sequence shown in SEQ ID NO:25.

76. The method according to any one of Claims 63-75, wherein the anti-glypican 3 antibody is a humanized antibody.

77. The method according to Claim 76, wherein the anti-glypican 3 antibody comprises:

the H chain variable region comprising the amino acid sequence shown in SEQ ID NO:3; and
the L chain variable region comprising the amino acid sequence shown in SEQ ID NO:4.

78. The method according to Claim 76, wherein the anti-glypican 3 antibody comprises:

the H chain variable region comprising the amino acid sequence shown in SEQ ID NO:3; and
the L chain variable region comprising the amino acid sequence wherein the 34th Gly of SEQ ID NO:4 is substituted with another amino acid residue.

79. The method according to Claim 76, wherein the anti-glypican 3 antibody comprises:

the H chain variable region comprising the amino acid sequence shown in SEQ ID NO:27; and
the L chain variable region comprising the amino acid sequence shown in SEQ ID NO:29.

80. The method according to Claim 76, wherein the anti-glypican 3 antibody comprises:

the H chain variable region comprising the amino acid sequence shown in SEQ ID NO:31; and
the L chain variable region comprising the amino acid sequence shown in SEQ ID NO:33.

81. A method for reducing a side-effect caused by a treatment of a liver cancer by chemotherapeutic agent in a subject comprising administering to the subject an effective amount of a therapeutic antibody.

82. The method according to Claim 81, wherein the chemotherapeutic agent is a kinase inhibitor.

83. The method according to Claim 81 or 82, wherein the chemotherapeutic agent is a multi-kinase inhibitor.

84. The method according to Claim 82 or 83, wherein the chemotherapeutic agent is Sorafenib (BAY43-9006).

85. The method according to Claim 82 or 83, wherein the chemotherapeutic agent is Sunitinib.

**86.** The method according to any one of Claims 81-85, wherein the side effect is weight loss.

**87.** The method according to any one of Claims 81-86, wherein the therapeutic antibody is an anti-glypican 3 antibody.

**88.** The method according to Claim 87, wherein the anti-glypican 3 antibody has cytotoxicity.

**89.** The method according to Claim 87 or 88, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of:

CDR1 comprising the amino acid sequence shown in SEQ ID NO:8,
CDR2 comprising the amino acid sequence shown in SEQ ID NO:24, and
CDR3 comprising the amino acid sequence shown in SEQ ID NO:25.

**90.** The method according to any one of Claims 87-89, wherein the anti-glypican 3 antibody is capable of binding to an epitope to which a second antibody can bind, wherein said second antibody comprises:

the H chain variable region comprising CDR1, 2, and 3 comprising the amino acid sequence shown in SEQ ID NOs:5, 6, and 7, respectively, and
the L chain variable region comprising CDR1, 2, and 3 comprising the amino acid sequence shown in SEQ ID NOs:8, 24, and 25, respectively.

**91.** The method according to any one of Claims 87, 88 or 90, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of:

CDR1 comprising the amino acid sequence shown in any one of SEQ ID NOs:9-23,
CDR2 comprising the amino acid sequence shown in SEQ ID NO:24, and CDR3 comprising the amino acid sequence shown in SEQ ID NO:25.

**92.** The method according to any one of Claims 87, 88 or 89, wherein the anti-glypican 3 antibody comprises the H chain variable region comprising CDR1, 2 and 3 of:

CDR1 comprising the amino acid sequence shown in SEQ ID NO:5,
CDR2 comprising the amino acid sequence shown in SEQ ID NO:26, and
CDR3 comprising the amino acid sequence shown in SEQ ID NO:7; and
the L chain variable region comprising CDR1, 2 and 3 of:

CDR1 comprising the amino acid sequence shown in SEQ ID NO:28,
CDR2 comprising the amino acid sequence shown in SEQ ID NO:24, and
CDR3 comprising the amino acid sequence shown in SEQ ID NO:25.

**93.** The method according to any one of Claims 87, 88 or 90, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:30, and

CDR3 comprising the amino acid sequence shown in SEQ ID NO:7; and
the L chain variable region comprising CDR1, 2 and 3 of:

CDR1 comprising the amino acid sequence shown in SEQ ID NO:32,
CDR2 comprising the amino acid sequence shown in SEQ ID NO:24, and
CDR3 comprising the amino acid sequence shown in SEQ ID NO:25.

**94.** The method according to any one of Claims 87-93, wherein the anti-glypican 3 antibody is a humanized antibody.

**95.** The method according to Claim 94, wherein the anti-glypican 3 antibody comprises:

the H chain variable region comprising the amino acid sequence shown in SEQ ID NO:3; and
the L chain variable region comprising the amino acid sequence shown in SEQ ID NO:4.

**96.** The method according to Claim 94, wherein the anti-glypican 3 antibody comprises:

the H chain variable region comprising the amino acid sequence shown in SEQ ID NO:3; and
the L chain variable region comprising the amino acid sequence wherein the 34th Gly of SEQ ID NO:4 is substituted with another amino acid residue.

**97.** The method according to Claim 94, wherein the anti-glypican 3 antibody comprises:

the H chain variable region comprising the amino acid sequence shown in SEQ ID NO:27; and
the L chain variable region comprising the amino acid sequence shown in SEQ ID NO:29.

**98.** The method according to Claim 94, wherein the anti-glypican 3 antibody comprises:

the H chain variable region comprising the amino acid sequence shown in SEQ ID NO:31; and
the L chain variable region comprising the amino acid sequence shown in SEQ ID NO:33.

**99.** A method for enhancing the efficacy of treatment of a liver cancer by a chemotherapeutic agent in a subject comprising administering to the subject an effective amount of an anti-glypican 3 antibody.

**100.** The method according to Claim 99, wherein the anti-glypican 3 antibody is administered simultaneously with the chemotherapeutic agent.

**101.** The method according to Claim 99, wherein the anti-glypican 3 antibody is administered before or after administration of the chemotherapeutic agent.

**102.** The method according to any one of Claims 99-101 wherein the chemotherapeutic agent is a kinase inhibitor.

**103.** The method according to Claim 102, wherein the chemotherapeutic agent is a multi-kinase inhibitor.

**104.** The method according to any one of Claims 99-103, wherein the chemotherapeutic agent is Sorafenib (BAY43-9006).

**105.** The method according to any one of Claims 99-103, wherein the chemotherapeutic agent is Sunitinib.

**106.** The method according to any one of Claims 99-105, wherein the anti-glypican 3 antibody has cytotoxicity.

**107.** The method according to any one of Claims 99-106, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of:

CDR1 comprising the amino acid sequence shown in SEQ ID NO:8,
CDR2 comprising the amino acid sequence shown in SEQ ID NO:24, and
CDR3 comprising the amino acid sequence shown in SEQ ID NO:25.

108. The method according to any one of Claims 99-107, wherein the anti-glypican 3 antibody is capable of binding to an epitope to which a second antibody can bind, wherein said second antibody comprises:

the H chain variable region comprising CDR1, 2, and 3 comprising the amino acid sequence shown in SEQ ID NOs:5, 6, and 7, respectively, and
the L chain variable region comprising CDR1, 2, and 3 comprising the amino acid sequence shown in SEQ ID NOs:8, 24, and 25, respectively.

109. The method according to any one of Claims 99-106 or 108, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:6, and
CDR3 comprising the amino acid sequence shown in SEQ ID NO:7; and
the L chain variable region comprising CDR1, 2 and 3 of:

CDR1 comprising the amino acid sequence shown in any one of SEQ ID NOs:9-23,
CDR2 comprising the amino acid sequence shown in SEQ ID NO:24, and
CDR3 comprising the amino acid sequence shown in SEQ ID NO:25.

110. The method according to any one of Claims 99-106 or 108, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:26, and
CDR3 comprising the amino acid sequence shown in SEQ ID NO:7; and
the L chain variable region comprising CDR1, 2 and 3 of:

CDR1 comprising the amino acid sequence shown in SEQ ID NO:28,
CDR2 comprising the amino acid sequence shown in SEQ ID NO:24, and
CDR3 comprising the amino acid sequence shown in SEQ ID NO:25.

111. The method according to any one of Claims 99-106 or 108, wherein the anti-glypican 3 antibody comprises:

the H chain variable region comprising CDR1, 2 and 3 of:

CDR1 comprising the amino acid sequence shown in SEQ ID NO:5,
CDR2 comprising the amino acid sequence shown in SEQ ID NO:30, and
CDR3 comprising the amino acid sequence shown in SEQ ID NO:7; and
the L chain variable region comprising CDR1, 2 and 3 of:

CDR1 comprising the amino acid sequence shown in SEQ ID NO:32,
CDR2 comprising the amino acid sequence shown in SEQ ID NO:24, and
CDR3 comprising the amino acid sequence shown in SEQ ID NO:25.

112. The method according to any one of Claims 99-111, wherein the anti-glypican 3 antibody is a humanized antibody.

113. The method according to Claim 112, wherein the anti-glypican 3 antibody comprises:

the H chain variable region comprising the amino acid sequence shown in SEQ ID NO:3; and
the L chain variable region comprising the amino acid sequence shown in SEQ ID NO:4.

**114.** The method according to Claim 112, wherein the anti-glypican 3 antibody comprises:

the H chain variable region comprising the amino acid sequence shown in SEQ ID NO:3; and
the L chain variable region comprising the amino acid sequence wherein the 34th Gly of SEQ ID NO:4 is substituted with another amino acid residue.

**115.** The method according to Claim 112, wherein the anti-glypican 3 antibody comprises:

the H chain variable region comprising the amino acid sequence shown in SEQ ID NO:27; and
the L chain variable region comprising the amino acid sequence shown in SEQ ID NO:29.

**116.** The method according to Claim 112, wherein the anti-glypican 3 antibody comprises:

the H chain variable region comprising the amino acid sequence shown in SEQ ID NO:31; and
the L chain variable region comprising the amino acid sequence shown in SEQ ID NO:33.

【FIG.1】

【FIG.2】

DAYS AFTER TRANSPLANTATION OF TUMOR (DAYS)

【FIG.3】

DAYS AFTER TRANSPLANTATION OF TUMOR (DAYS)

【FIG.4】

DAYS AFTER TRANSPLANTATION OF TUMOR (DAYS)

【FIG.5】

DAYS AFTER TRANSPLANTATION OF TUMOR (DAYS)

【FIG.6】

Y-axis: BODY WEIGHT (g)
X-axis: DAYS AFTER TRANSPLANTATION OF TUMOR (DAYS)

【FIG.7】

Y-axis: TUMOR VOLUME (mm3)
X-axis: DAYS AFTER TRANSPLANTATION OF TUMOR (DAYS)

【FIG.8】

| NAME OF ANTIBODY | CHAIN | SEQUENCE | SEQ ID NO. |
|---|---|---|---|
| hGC33 | H | QVQLVQSGAEVKKPGASVKVSCKASGYTFTDYEMHWVRQA PGQGLEWMGALDPKTGDTAYSQKFKGRVTLTADKSTSTAY MELSSLTSEDTAVYYCTRFYSYTYWGQGTLVTVSS | 3 |
| hGC33 | L | DVVMTQSPLSLPVTPGEPASISCRSSQSLVHSNGNTYLHW YLQKPGQSPQLLIYKVSNRFSGVPDRFSGSGSGTDFTLKI SRVEAEDVGVYYCSQNTHVPPTFGQGTKLEIK | 4 |
| Hspd1.8(Hd1.8) | H | QVQLVQSGAEVKKPGASVTVSCKASGYTFTDYEMHWIRQP PGEGLEWIGAIDPKTGDTAYSQSFQDRVTLTADKSTSTAY MELSSLTSEDTAVYYCTRFYSYTYWGQGTLVTVSS | 27 |
| Lspd1.6(Ld1.6) VL | L | DIVMTQSPLSLPVTPGEPASISCRASESLVHSNRNTYLHW YQQKPGQAPRLLIYKVSNRFSGVPDRFSGSGSGTDFTLTI SSLQAEDVGVYYCSQNTHVPPTFGQGTKLEIK | 29 |
| pH7 | H | QVQLVQSGAEVKKPGASVTVSCKASGYTFTDYEMHWIRQP PGEGLEWIGAIDPKTGDTAYSESFQDRVTLTADKSTSTAY MELSSLTSEDTAVYYCTRFYSYTYWGQGTLVTVSS | 31 |
| pL16 | H | DIVMTQSPLSLPVTPGEPASISCQASESLVHSNRNTYLHW YLQKPGQSPQLLIYKVSNRFSGVPDRFSGSGSGTDFTLKI SRVEAEDVGVYYCSQNTHVPPTFGQGTKVEIE | 33 |
| H15 | H | QVQLVQSGAEVKKPGASVKVSCKASGYTFTDYEMHWIRQP PGQGLEWIGAIDPKTGDTAYSQKFKGRVTLTADKSTSTAY MELSSLTSEDTAVYYCTRFYSYTYWGQGTLVTVSS | 34 |
| L4 | L | DIVMTQSPLSLPVTPGEPASISCRSSQSLVHSNRNTYLHW YQQKPGQAPRLLIYKVSNRFSGVPDRFSGSGSGTDFTLKI SRVEAEDVGVYYCSQNTHVPPTFGQGTKLEIK | 35 |

【FIG.9】

| NAME OF ANTIBODY | CHAIN | CDR | SEQUENCE | SEQ ID NO. |
|---|---|---|---|---|
| GC33 | H | CDR1 | DYEMH | 5 |
| GC33 | H | CDR2 | ALDPKTGDTAYSQKFK | 6 |
| GC33 | H | CDR3 | FYSYTY | 7 |
| GC33 | L | CDR1 | RSSQSLVHSNGNTYLH | 8 |
| GC33 (G34A) | L | CDR1 | RSSQSLVHSNANTYLH | 9 |
| GC33 (G34D) | L | CDR1 | RSSQSLVHSNDNTYLH | 10 |
| GC33 (G34E) | L | CDR1 | RSSQSLVHSNENTYLH | 11 |
| GC33 (G34F) | L | CDR1 | RSSQSLVHSNFNTYLH | 12 |
| GC33 (G34H) | L | CDR1 | RSSQSLVHSNHNTYLH | 13 |
| GC33 (G34N) | L | CDR1 | RSSQSLVHSNNNTYLH | 14 |
| GC33 (G34T) | L | CDR1 | RSSQSLVHSNTNTYLH | 15 |
| GC33 (G34Q) | L | CDR1 | RSSQSLVHSNQNTYLH | 16 |
| GC33 (G34I) | L | CDR1 | RSSQSLVHSNINTYLH | 17 |
| GC33 (G34K) | L | CDR1 | RSSQSLVHSNKNTYLH | 18 |
| GC33 (G34L) | L | CDR1 | RSSQSLVHSNLNTYLH | 19 |
| GC33 (G34S) | L | CDR1 | RSSQSLVHSNSNTYLH | 20 |
| GC33 (G34W) | L | CDR1 | RSSQSLVHSNWNTYLH | 21 |
| GC33 (G34Y) | L | CDR1 | RSSQSLVHSNYNTYLH | 22 |
| GC33 (G34R) | L | CDR1 | RSSQSLVHSNRNTYLH | 23 |
| GC33 | L | CDR2 | KVSNRFS | 24 |
| GC33 | L | CDR3 | SQNTHVPPT | 25 |
| Hspd1. 8 (Hd1. 8) | H | CDR2 | AIDPKTGDTAYSQSFQ | 26 |
| Lspd1. 6 (Ld1. 6) | L | CDR1 | RASESLVHSNRNTYLH | 28 |
| pH7 | H | CDR2 | AIDPKTGDTAYSESFQ | 30 |
| pL16 | L | CDR1 | QASESLVHSNRNTYLH | 32 |

## INTERNATIONAL SEARCH REPORT

| | International application No. |
|---|---|
| | PCT/JP2009/001249 |

### A. CLASSIFICATION OF SUBJECT MATTER
*A61K39/395*(2006.01)i, *A61K31/404*(2006.01)i, *A61K31/44*(2006.01)i,
*A61K45/00*(2006.01)i, *A61P35/00*(2006.01)i, *A61P43/00*(2006.01)i

According to International Patent Classification (IPC) or to both national classification and IPC

### B. FIELDS SEARCHED

Minimum documentation searched (classification system followed by classification symbols)
A61K39/395, A61K31/404, A61K31/44, A61K45/00, A61P35/00, A61P43/00

Documentation searched other than minimum documentation to the extent that such documents are included in the fields searched
Jitsuyo Shinan Koho          1922-1996     Jitsuyo Shinan Toroku Koho     1996-2009
Kokai Jitsuyo Shinan Koho    1971-2009     Toroku Jitsuyo Shinan Koho     1994-2009

Electronic data base consulted during the international search (name of data base and, where practicable, search terms used)
CA/MEDLINE/EMBASE/BIOSIS(STN), JSTPlus/JMEDPlus/JST7580(JDreamII),
SwissProt/PIR/GeneSeq

### C. DOCUMENTS CONSIDERED TO BE RELEVANT

| Category* | Citation of document, with indication, where appropriate, of the relevant passages | Relevant to claim No. |
|---|---|---|
| X<br>Y | WO 2006/006693 A1  (Chugai Pharmaceutical Co.,<br>Ltd.),<br>19 January, 2006 (19.01.06),<br>Claims; examples 18 to 27; sequence listing 90,<br>92<br>& JP 4011100 B          & JP 2007-300927 A<br>& US 2007/0190599 A1     & EP 1674111 A1<br>& CA 2544692 A           & NO 20063539 A<br>& CN 1842540 A | 6-26,45-62<br>1-5,27-44 |
| X<br>Y | WO 2006/046751 A1  (Chugai Pharmaceutical Co.,<br>Ltd.),<br>04 May, 2006 (04.05.06),<br>Claims; each examples; referential examples<br>& US 2008/0124330 A1     & EP 1816140 A1<br>& CA 2585196 A           & KR 10-2007-0070222 A<br>& CN 101068836 A | 6-26,45-62<br>1-5,27-44 |

☒ Further documents are listed in the continuation of Box C.          ☐ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered  to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 02 April, 2009 (02.04.09) | 12 May, 2009 (12.05.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.

PCT/JP2009/001249

C (Continuation).  DOCUMENTS CONSIDERED TO BE RELEVANT

| Category* | Citation of document, with indication, where appropriate, of the relevant passages | Relevant to claim No. |
|---|---|---|
| X<br>Y | WO 2006/067913 A1  (Chugai Pharmaceutical Co., Ltd.),<br>29 June, 2006 (29.06.06),<br>Each examples; referential examples<br>& US 2008/0166756 A1    & EP 1829962 A1<br>& EP 1829961 A1 | 6-26,45-62<br>1-5,27-44 |
| X<br>Y | WO 03/000883 A1  (Chugai Pharmaceutical Co., Ltd.),<br>03 January, 2003 (03.01.03),<br>Each Claims; each examples<br>& JP 2005-225892 A      & JP 2007-51159 A<br>& JP 2007-238632 A      & JP 2008-120828 A<br>& US 2004/0236080 A1    & US 2007/0172488 A1<br>& EP 1411118 A1         & EP 1780273 A1<br>& DE 60228721 D         & CA 2451493 A<br>& HK 1079547 A          & CN 1688692 A<br>& KR 10-2008-0077295 A  & AT 407204 T | 6-26,45-62<br>1-5,27-44 |
| Y<br>A | WO 2007/053573 A2  (BAYER PHARMACEUTICALS CORP.),<br>10 May, 2007 (10.05.07),<br>Claims<br>& EP 1954272 A2         & CA 2627873 A | 1-5,27-44<br>6-26,45-62 |
| Y<br>A | WO 2007/091622 A1  (Sankyo Co., Ltd.),<br>16 August, 2007 (16.08.07),<br>Par. No. [0003]; each examples<br>& EP 1982718 A1         & CA 2642665 A | 1-5,27-44<br>6-26,45-62 |
| Y<br>A | Masafumi IKEDA et al., "Genpatsusei Kangan no Yakubutsu Ryoho to Kinase Sogaizai Sorafenib", Hematology & Oncology, 28 January, 2008 (28.01.08), Vol.56, No.1, pages 70 to 75, ISSN:0915-8529 | 1-5,27-44<br>6-26,45-62 |
| Y<br>A | JP 2008-501677 A  (Pfizer Products Inc.),<br>24 January, 2008 (24.01.08),<br>Each Claims<br>& US 2005/0272755 A1    & EP 1761281 A<br>& WO 2005/117980 A1     & CA 2569277 A | 1-5,27-44<br>6-26,45-62 |
| Y<br>A | WO 2007/099988 A1  (Kyowa Hakko Kogyo Co., Ltd.),<br>07 September, 2007 (07.09.07),<br>Par. No. [0199]<br>(Family: none) | 1-5,27-44<br>6-26,45-62 |
| Y<br>A | JP 2004-503582 A  (Pharmacia Italia S.p.A.),<br>05 February, 2004 (05.02.04),<br>Each Claims; Par. Nos. [0021] to [0022]<br>& US 2005/0032759 A1    & GB 17635 D<br>& EP 1315486 A2         & WO 2002/005791 A2<br>& AU 1041502 A | 1-5,27-44<br>6-26,45-62 |

Form PCT/ISA/210 (continuation of second sheet) (April 2007)

EP 2 275 135 A1

**INTERNATIONAL SEARCH REPORT**

International application No.

PCT/JP2009/001249

**Box No. II**      **Observations where certain claims were found unsearchable (Continuation of item 2 of first sheet)**

This international search report has not been established in respect of certain claims under Article 17(2)(a) for the following reasons:

1. ☒ Claims Nos.: 63-116
because they relate to subject matter not required to be searched by this Authority, namely:
It pertain to methods for treatment of the human body or animal body by surgery or therapy and thus relate to a subject matter which this International Searching Authority is not required, under the provisions of Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III**      **Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**     ☐ The additional search fees were accompanied by the applicant's protest and, where applicable,
the                      payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008098309 A
- JP 2008002690 W
- WO 2003000883 A
- WO 2006006693 A
- WO 1994011523 A
- EP 239400 A
- WO 96002576 A
- JP H159878 B
- WO 1993012227 A
- WO 199203918 A
- WO 1994002602 A
- WO 1994025585 A

- WO 1996034096 A
- WO 1996033735 A
- WO 1992001047 A
- WO 1992020791 A
- WO 1993006213 A
- WO 1993011236 A
- WO 1993019172 A
- WO 1995001438 A
- WO 1995015388 A
- WO 199954342 A
- WO 2000061739 A
- WO 2002031140 A
- WO 2002079255 A

**Non-patent literature cited in the description**

- **Llovet, J.M. ; Burroughs, A. ; Bruix, J.** *Lancet,* 2003, vol. 362, 1907-17
- **Bosch, F.X. ; Ribes, J. ; Cleries, R.** *Gastroenterology,* 2004, vol. 127, 5-16
- **Takenaka, K. ; Kawahara, N. ; Yamamoto, K. ; Kajiyama, K. ; Maeda, T. ; Itasaka, H. ; Shirabe, K. ; Nishizaki, T. ; Yanaga, K. ; Sugimachi, K.** *Arch Surg.,* 1996, vol. 131, 71-6
- **Yeo, W. ; Mok, T.S. ; Zee, B. ; Leung, T.W. ; Lai, P.B. ; Lau, W.Y. ; Koh, J. ; Mo, F.K. ; Yu, S.C. ; Chan, A.T.** *J. Natl. Cancer Inst.,* 2005, vol. 97, 1532-8
- **Furuse, J. ; Ishii, H. ; Nakachi, K. ; Suzuki, E. ; Shimizu, S. ; Nakajima, K.** *Cancer Sci.,* 22 October 2007
- **Philip, P.A. ; Mahoney, M.R. ; Allmer, C. ; Thomas, J. ; Pitot, H.C. ; Kim, G. ; Donehower, R.C. ; Fitch, T. ; Picus, J. ; Erlichman, C.** *J. Clin. Oncol.,* 2005, vol. 23, 6657-63
- **Thomas, M.B. ; Dutta, A. ; Brown, T. ; Charnsangavej, C. ; Rashid, A. ; Hoff, P.M. ; Dancey, J. ; Abbruzzese, J.L.** *J. Clin. Oncol., 2005 ASCO Annual Meeting Proceedings,* 2005, vol. 23, 16
- **Mendel DB ; Laird AD ; Xin X ; Louie SG ; Christensen JG ; Li G ; Schreck RE ; Abrams TJ ; Ngai TJ ; Lee LB.** *Clin Cancer Res,* 2003, vol. 9, 327-37
- **Zhu A ; Sahani D ; di Tomaso E et al.** *99th AACR annual meeting.,* 12 April 2008
- **Vandamme, A.M. et al.** *Eur. J. Biochem.,* 1990, vol. 192, 767-75

- **Chirgwin, J.M. et al.** *Biochemistry,* 1979, vol. 18, 5294-5299
- **Chomczynski, P. et al.** *Anal. Biochem.,* 1987, vol. 162, 156-159
- **Frohman, M.A. et al.** *Proc. Natl. Acad. Sci. USA,* 1988, vol. 85, 8998-9002
- **Belyavsky, A. et al.** *Nucleic Acids Res.,* 1989, vol. 17, 2919-2932
- **Ebert, K.M. et al.** *Bio/Technology,* 1994, vol. 12, 699-702
- **Sato, K. et al.** *Cancer Res,* 1993, vol. 53, 851-856
- **Mulligan et al.** *Nature,* 1979, vol. 277, 108
- **Mizushima et al.** *Nucleic Acids Res.,* 1990, vol. 18, 5322
- **Ward et al.** *Nature,* 1989, vol. 341, 544-546
- *FASEBJ.,* 1992, vol. 6, 2422-2427
- **Better et al.** *Science,* 1988, vol. 240, 1041-1043
- **Lei, S.P. et al.** *J. Bacteriol.,* 1987, vol. 169, 4379
- Antibodies: A Laboratory Manual. Cold Spring Harbor Laboratory, 1988
- Antibodies: A Laboratory Manual. Cold Spring Harbor Laboratory, 1988, 359-420
- Antibodies, A Laboratory Manual. Cold Spring Harbor Laboratory, 1988
- Current Protocols in Immunology. Immunologic studies in humans. John Wiley & Sons, Inc, 1993
- Remington's Pharmaceutical Science. Mack Publishing Company

- *Organic Process Research & Development,* 2002, vol. 6, 777-781

- **Rodolfo et al.** *Immunology Letters,* 1999, 47-52

```
                                SEQUENCE LISTING
<110>  Chugai Seiyaku Kabushiki Kaisha
<120>  combination therapy
<130>  PCG-9026WO
<150>  JP 2008-098309
<151>  2008-04-04
<150>  PCT/JP2008/002690
<151>  2008-09-26
<160>  36
<170>  PatentIn version 3.1
<210>  1
<211>  115
<212>  PRT
<213>  Artificial Sequence
<220>
<223>  Modified antibody
<400>  1
Gln Val Gln Leu Gln Gln Ser Gly Ala Glu Leu Val Arg Pro Gly Ala
1                   5                   10                  15


Ser Val Lys Leu Ser Cys Lys Ala Ser Gly Tyr Thr Phe Thr Asp Tyr
            20                  25                  30


Glu Met His Trp Val Lys Gln Thr Pro Val His Gly Leu Lys Trp Ile
        35                  40                  45


Gly Ala Leu Asp Pro Lys Thr Gly Asp Thr Ala Tyr Ser Gln Lys Phe
    50                  55                  60


Lys Gly Lys Ala Thr Leu Thr Ala Asp Lys Ser Ser Ser Thr Ala Tyr
65                  70                  75                  80


Met Glu Leu Arg Ser Leu Thr Ser Glu Asp Ser Ala Val Tyr Tyr Cys
                85                  90                  95


Thr Arg Phe Tyr Ser Tyr Thr Tyr Trp Gly Gln Gly Thr Leu Val Thr
            100                 105                 110


Val Ser Ala
        115


<210>  2
<211>  112
<212>  PRT
<213>  Artificial Sequence
<220>
<223>  Modified antibody
<400>  2
Asp Val Val Met Thr Gln Thr Pro Leu Ser Leu Pro Val Ser Leu Gly
1                   5                   10                  15


Asp Gln Ala Ser Ile Ser Cys Arg Ser Ser Gln Ser Leu Val His Ser
            20                  25                  30


Asn Gly Asn Thr Tyr Leu His Trp Tyr Leu Gln Lys Pro Gly Gln Ser
        35                  40                  45


Pro Lys Leu Leu Ile Tyr Lys Val Ser Asn Arg Phe Ser Gly Val Pro
    50                  55                  60


Asp Arg Phe Ser Gly Ser Gly Ser Gly Thr Asp Phe Thr Leu Lys Ile
65                  70                  75                  80


Ser Arg Val Glu Ala Glu Asp Leu Gly Val Tyr Phe Cys Ser Gln Asn
                85                  90                  95


Thr His Val Pro Pro Thr Phe Gly Ser Gly Thr Lys Leu Glu Ile Lys
            100                 105                 110
```

```
<210>   3
<211>   115
<212>   PRT
<213>   Artificial Sequence
<220>
<223>   Modified antibody
<400>   3
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Glu Met His Trp Val Arg Gln Ala Pro Gly Gln Gly Leu Glu Trp Met
        35                  40                  45

Gly Ala Leu Asp Pro Lys Thr Gly Asp Thr Ala Tyr Ser Gln Lys Phe
    50                  55                  60

Lys Gly Arg Val Thr Leu Thr Ala Asp Lys Ser Thr Ser Thr Ala Tyr
65                  70                  75                  80

Met Glu Leu Ser Ser Leu Thr Ser Glu Asp Thr Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ser
        115


<210>   4
<211>   112
<212>   PRT
<213>   Artificial Sequence
<220>
<223>   Modified antibody
<400>   4
Asp Val Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Pro Thr Phe Gly Gln Gly Thr Lys Leu Glu Ile Lys
            100                 105                 110

<210>   5
<211>   5
<212>   PRT
<213>   Artificial Sequence
<220>
<223>   Modified antibody
<400>   5
Asp Tyr Glu Met His
```

1                    5

<210>  6
<211>  16
<212>  PRT
<213>  Artificial Sequence
<220>
<223>  Modified antibody
<400>  6
Ala Leu Asp Pro Lys Thr Gly Asp Thr Ala Tyr Ser Gln Lys Phe Lys
1                    5                    10                   15


<210>  7
<211>  6
<212>  PRT
<213>  Artificial Sequence
<220>
<223>  Modified antibody
<400>  7
Phe Tyr Ser Tyr Thr Tyr
1                    5


<210>  8
<211>  16
<212>  PRT
<213>  Artificial Sequence
<220>
<223>  Modified antibody
<400>  8
Arg Ser Ser Gln Ser Leu Val His Ser Asn Gly Asn Thr Tyr Leu His
1                    5                    10                   15


<210>  9
<211>  16
<212>  PRT
<213>  Artificial Sequence
<220>
<223>  Modified antibody
<400>  9
Arg Ser Ser Gln Ser Leu Val His Ser Asn Ala Asn Thr Tyr Leu His
1                    5                    10                   15


<210>  10
<211>  16
<212>  PRT
<213>  Artificial Sequence
<220>
<223>  Modified antibody
<400>  10
Arg Ser Ser Gln Ser Leu Val His Ser Asn Asp Asn Thr Tyr Leu His
1                    5                    10                   15


<210>  11
<211>  16
<212>  PRT
<213>  Artificial Sequence
<220>
<223>  Modified antibody
<400>  11
Arg Ser Ser Gln Ser Leu Val His Ser Asn Glu Asn Thr Tyr Leu His
1                    5                    10                   15


<210>  12
<211>  16
<212>  PRT
<213>  Artificial Sequence
<220>
<223>  Modified antibody

```
<400>  12
Arg Ser Ser Gln Ser Leu Val His Ser Asn Phe Asn Thr Tyr Leu His
1               5                   10                  15

<210>  13
<211>  16
<212>  PRT
<213>  Artificial Sequence
<220>
<223>  Modified antibody
<400>  13
Arg Ser Ser Gln Ser Leu Val His Ser Asn His Asn Thr Tyr Leu His
1               5                   10                  15

<210>  14
<211>  16
<212>  PRT
<213>  Artificial Sequence
<220>
<223>  Modified antibody
<400>  14
Arg Ser Ser Gln Ser Leu Val His Ser Asn Asn Asn Thr Tyr Leu His
1               5                   10                  15

<210>  15
<211>  16
<212>  PRT
<213>  Artificial Sequence
<220>
<223>  Modified antibody
<400>  15
Arg Ser Ser Gln Ser Leu Val His Ser Asn Thr Asn Thr Tyr Leu His
1               5                   10                  15

<210>  16
<211>  16
<212>  PRT
<213>  Artificial Sequence
<220>
<223>  Modified antibody
<400>  16
Arg Ser Ser Gln Ser Leu Val His Ser Asn Gln Asn Thr Tyr Leu His
1               5                   10                  15

<210>  17
<211>  16
<212>  PRT
<213>  Artificial Sequence
<220>
<223>  Modified antibody
<400>  17
Arg Ser Ser Gln Ser Leu Val His Ser Asn Ile Asn Thr Tyr Leu His
1               5                   10                  15

<210>  18
<211>  16
<212>  PRT
<213>  Artificial Sequence
<220>
<223>  Modified antibody
<400>  18
Arg Ser Ser Gln Ser Leu Val His Ser Asn Lys Asn Thr Tyr Leu His
1               5                   10                  15

<210>  19
<211>  16
<212>  PRT
<213>  Artificial Sequence
```

```
<220>
<223>  Modified antibody
<400>  19
Arg Ser Ser Gln Ser Leu Val His Ser Asn Leu Asn Thr Tyr Leu His
1               5                   10                  15


<210>  20
<211>  16
<212>  PRT
<213>  Artificial Sequence
<220>
<223>  Modified antibody
<400>  20
Arg Ser Ser Gln Ser Leu Val His Ser Asn Ser Asn Thr Tyr Leu His
1               5                   10                  15


<210>  21
<211>  16
<212>  PRT
<213>  Artificial Sequence
<220>
<223>  Modified antibody
<400>  21
Arg Ser Ser Gln Ser Leu Val His Ser Asn Trp Asn Thr Tyr Leu His
1               5                   10                  15


<210>  22
<211>  16
<212>  PRT
<213>  Artificial Sequence
<220>
<223>  Modified antibody
<400>  22
Arg Ser Ser Gln Ser Leu Val His Ser Asn Tyr Asn Thr Tyr Leu His
1               5                   10                  15


<210>  23
<211>  16
<212>  PRT
<213>  Artificial Sequence
<220>
<223>  Modified antibody
<400>  23
Arg Ser Ser Gln Ser Leu Val His Ser Asn Arg Asn Thr Tyr Leu His
1               5                   10                  15


<210>  24
<211>  7
<212>  PRT
<213>  Artificial Sequence
<220>
<223>  Modified antibody
<400>  24
Lys Val Ser Asn Arg Phe Ser
1               5


<210>  25
<211>  9
<212>  PRT
<213>  Artificial Sequence
<220>
<223>  Modified antibody
<400>  25
Ser Gln Asn Thr His Val Pro Pro Thr
1               5


<210>  26
<211>  16
```

```
<212>  PRT
<213>  Artificial Sequence
<220>
<223>  Modified antibody
<400>  26
Ala Ile Asp Pro Lys Thr Gly Asp Thr Ala Tyr Ser Gln Ser Phe Gln
1               5                   10                  15


<210>  27
<211>  115
<212>  PRT
<213>  Artificial Sequence
<220>
<223>  Modified antibody
<400>  27
Gln Val Gln Leu Val Gln Ser Gly Ala Glu Val Lys Lys Pro Gly Ala
1               5                   10                  15

Ser Val Thr Val Ser Cys Lys Ala Ser Gly Tyr Thr Phe Thr Asp Tyr
            20                  25                  30

Glu Met His Trp Ile Arg Gln Pro Pro Gly Glu Gly Leu Glu Trp Ile
            35                  40                  45

Gly Ala Ile Asp Pro Lys Thr Gly Asp Thr Ala Tyr Ser Gln Ser Phe
        50                  55                  60

Gln Asp Arg Val Thr Leu Thr Ala Asp Lys Ser Thr Ser Thr Ala Tyr
65                  70                  75                  80

Met Glu Leu Ser Ser Leu Thr Ser Glu Asp Thr Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ser
        115


<210>  28
<211>  16
<212>  PRT
<213>  Artificial Sequence
<220>
<223>  Modified antibody
<400>  28
Arg Ala Ser Glu Ser Leu Val His Ser Asn Arg Asn Thr Tyr Leu His
1               5                   10                  15


<210>  29
<211>  112
<212>  PRT
<213>  Artificial Sequence
<220>
<223>  Modified antibody
<400>  29
Asp Ile Val Met Thr Gln Ser Pro Leu Ser Leu Pro Val Thr Pro Gly
1               5                   10                  15

Glu Pro Ala Ser Ile Ser Cys Arg Ala Ser Glu Ser Leu Val His Ser
            20                  25                  30

Asn Arg Asn Thr Tyr Leu His Trp Tyr Gln Gln Lys Pro Gly Gln Ala
            35                  40                  45

Pro Arg Leu Leu Ile Tyr Lys Val Ser Asn Arg Phe Ser Gly Val Pro
        50                  55                  60
```

Asp Arg Phe Ser Gly Ser Gly Ser Gly Thr Asp Phe Thr Leu Thr Ile
65                  70                  75                  80

Ser Ser Leu Gln Ala Glu Asp Val Gly Val Tyr Tyr Cys Ser Gln Asn
                85                  90                  95

Thr His Val Pro Pro Thr Phe Gly Gln Gly Thr Lys Leu Glu Ile Lys
            100                 105                 110

<210>  30
<211>  16
<212>  PRT
<213>  Artificial Sequence
<220>
<223>  Modified antibody
<400>  30
Ala Ile Asp Pro Lys Thr Gly Asp Thr Ala Tyr Ser Glu Ser Phe Gln
1               5                   10                  15

<210>  31
<211>  115
<212>  PRT
<213>  Artificial Sequence
<220>
<223>  Modified antibody
<400>  31
Gln Val Gln Leu Val Gln Ser Gly Ala Glu Val Lys Lys Pro Gly Ala
1               5                   10                  15

Ser Val Thr Val Ser Cys Lys Ala Ser Gly Tyr Thr Phe Thr Asp Tyr
                20                  25                  30

Glu Met His Trp Ile Arg Gln Pro Pro Gly Glu Gly Leu Glu Trp Ile
            35                  40                  45

Gly Ala Ile Asp Pro Lys Thr Gly Asp Thr Ala Tyr Ser Glu Ser Phe
        50                  55                  60

Gln Asp Arg Val Thr Leu Thr Ala Asp Lys Ser Thr Ser Thr Ala Tyr
65                  70                  75                  80

Met Glu Leu Ser Ser Leu Thr Ser Glu Asp Thr Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ser
            115

<210>  32
<211>  16
<212>  PRT
<213>  Artificial Sequence
<220>
<223>  Modified antibody
<400>  32
Gln Ala Ser Glu Ser Leu Val His Ser Asn Arg Asn Thr Tyr Leu His
1               5                   10                  15

<210>  33
<211>  112
<212>  PRT
<213>  Artificial Sequence
<220>
<223>  Modified antibody
<400>  33
Asp Ile Val Met Thr Gln Ser Pro Leu Ser Leu Pro Val Thr Pro Gly

```
        1                   5                   10                  15

        Glu Pro Ala Ser Ile Ser Cys Gln Ala Ser Glu Ser Leu Val His Ser
                        20                  25                  30

        Asn Arg Asn Thr Tyr Leu His Trp Tyr Leu Gln Lys Pro Gly Gln Ser
                    35                  40                  45

        Pro Gln Leu Leu Ile Tyr Lys Val Ser Asn Arg Phe Ser Gly Val Pro
                50                  55                  60

        Asp Arg Phe Ser Gly Ser Gly Ser Gly Thr Asp Phe Thr Leu Lys Ile
        65                  70                  75                  80

        Ser Arg Val Glu Ala Glu Asp Val Gly Val Tyr Tyr Cys Ser Gln Asn
                        85                  90                  95

        Thr His Val Pro Pro Thr Phe Gly Gln Gly Thr Lys Val Glu Ile Glu
                    100                 105                 110
```

```
<210>   34
<211>   115
<212>   PRT
<213>   Artificial Sequence
<220>
<223>   Modified antibody
<400>   34
```

```
        Gln Val Gln Leu Val Gln Ser Gly Ala Glu Val Lys Lys Pro Gly Ala
        1                   5                   10                  15

        Ser Val Lys Val Ser Cys Lys Ala Ser Gly Tyr Thr Phe Thr Asp Tyr
                        20                  25                  30

        Glu Met His Trp Ile Arg Gln Pro Pro Gly Gln Gly Leu Glu Trp Ile
                    35                  40                  45

        Gly Ala Ile Asp Pro Lys Thr Gly Asp Thr Ala Tyr Ser Gln Lys Phe
                50                  55                  60

        Lys Gly Arg Val Thr Leu Thr Ala Asp Lys Ser Thr Ser Thr Ala Tyr
        65                  70                  75                  80

        Met Glu Leu Ser Ser Leu Thr Ser Glu Asp Thr Ala Val Tyr Tyr Cys
                        85                  90                  95

        Thr Arg Phe Tyr Ser Tyr Thr Tyr Trp Gly Gln Gly Thr Leu Val Thr
                    100                 105                 110

        Val Ser Ser
                115
```

```
<210>   35
<211>   112
<212>   PRT
<213>   Artificial Sequence
<220>
<223>   Modified antibody
<400>   35
```

```
        Asp Ile Val Met Thr Gln Ser Pro Leu Ser Leu Pro Val Thr Pro Gly
        1                   5                   10                  15

        Glu Pro Ala Ser Ile Ser Cys Arg Ser Ser Gln Ser Leu Val His Ser
                        20                  25                  30

        Asn Arg Asn Thr Tyr Leu His Trp Tyr Gln Gln Lys Pro Gly Gln Ala
                    35                  40                  45

        Pro Arg Leu Leu Ile Tyr Lys Val Ser Asn Arg Phe Ser Gly Val Pro
```

```
          50                    55                    60

    Asp Arg Phe Ser Gly Ser Gly Ser Gly Thr Asp Phe Thr Leu Lys Ile
    65              70                  75                  80

    Ser Arg Val Glu Ala Glu Asp Val Gly Val Tyr Tyr Cys Ser Gln Asn
                85                  90                  95

    Thr His Val Pro Pro Thr Phe Gly Gln Gly Thr Lys Leu Glu Ile Lys
                100                 105                 110

    <210>  36
    <211>  545
    <212>  PRT
    <213>  homo sapiens
    <400>  36
    Gln Pro Pro Pro Pro Pro Asp Ala Thr Cys His Gln Val Arg Ser
    1               5               10                  15

    Phe Phe Gln Arg Leu Gln Pro Gly Leu Lys Trp Val Pro Glu Thr Pro
                20                  25                  30

    Val Pro Gly Ser Asp Leu Gln Val Cys Leu Pro Lys Gly Pro Thr Cys
                35                  40                  45

    Cys Ser Arg Lys Met Glu Glu Lys Tyr Gln Leu Thr Ala Arg Leu Asn
                50                  55                  60

    Met Glu Gln Leu Leu Gln Ser Ala Ser Met Glu Leu Lys Phe Leu Ile
    65              70                  75                  80

    Ile Gln Asn Ala Ala Val Phe Gln Glu Ala Phe Glu Ile Val Val Arg
                85                  90                  95

    His Ala Lys Asn Tyr Thr Asn Ala Met Phe Lys Asn Asn Tyr Pro Ser
                100                 105                 110

    Leu Thr Pro Gln Ala Phe Glu Phe Val Gly Glu Phe Phe Thr Asp Val
                115                 120                 125

    Ser Leu Tyr Ile Leu Gly Ser Asp Ile Asn Val Asp Asp Met Val Asn
                130                 135                 140

    Glu Leu Phe Asp Ser Leu Phe Pro Val Ile Tyr Thr Gln Leu Met Asn
    145                 150                 155                 160

    Pro Gly Leu Pro Asp Ser Ala Leu Asp Ile Asn Glu Cys Leu Arg Gly
                165                 170                 175

    Ala Arg Arg Asp Leu Lys Val Phe Gly Asn Phe Pro Lys Leu Ile Met
                180                 185                 190

    Thr Gln Val Ser Lys Ser Leu Gln Val Thr Arg Ile Phe Leu Gln Ala
                195                 200                 205

    Leu Asn Leu Gly Ile Glu Val Ile Asn Thr Thr Asp His Leu Lys Phe
                210                 215                 220

    Ser Lys Asp Cys Gly Arg Met Leu Thr Arg Met Trp Tyr Cys Ser Tyr
    225                 230                 235                 240

    Cys Gln Gly Leu Met Met Val Lys Pro Cys Gly Gly Tyr Cys Asn Val
                245                 250                 255

    Val Met Gln Gly Cys Met Ala Gly Val Val Glu Ile Asp Lys Tyr Trp
                260                 265                 270

    Arg Glu Tyr Ile Leu Ser Leu Glu Glu Leu Val Asn Gly Met Tyr Arg
```

```
                275                        280                        285

        Ile Tyr Asp Met Glu Asn Val Leu Leu Gly Leu Phe Ser Thr Ile His
            290                     295                 300

        Asp Ser Ile Gln Tyr Val Gln Lys Asn Ala Gly Lys Leu Thr Thr Thr
        305                     310                 315                 320

        Ile Gly Lys Leu Cys Ala His Ser Gln Gln Arg Gln Tyr Arg Ser Ala
                        325                 330                 335

        Tyr Tyr Pro Glu Asp Leu Phe Ile Asp Lys Lys Val Leu Lys Val Ala
                    340                 345                 350

        His Val Glu His Glu Glu Thr Leu Ser Ser Arg Arg Arg Glu Leu Ile
                355                 360                 365

        Gln Lys Leu Lys Ser Phe Ile Ser Phe Tyr Ser Ala Leu Pro Gly Tyr
            370                 375                 380

        Ile Cys Ser His Ser Pro Val Ala Glu Asn Asp Thr Leu Cys Trp Asn
        385                 390                 395                 400

        Gly Gln Glu Leu Val Glu Arg Tyr Ser Gln Lys Ala Ala Arg Asn Gly
                    405                 410                 415

        Met Lys Asn Gln Phe Asn Leu His Glu Leu Lys Met Lys Gly Pro Glu
                    420                 425                 430

        Pro Val Val Ser Gln Ile Ile Asp Lys Leu Lys His Ile Asn Gln Leu
                435                 440                 445

        Leu Arg Thr Met Ser Met Pro Lys Gly Arg Val Leu Asp Lys Asn Leu
            450                 455                 460

        Asp Glu Glu Gly Phe Glu Ala Gly Asp Cys Gly Asp Asp Glu Asp Glu
        465                 470                 475                 480

        Cys Ile Gly Gly Ala Gly Asp Gly Met Ile Lys Val Lys Asn Gln Leu
                        485                 490                 495

        Arg Phe Leu Ala Glu Leu Ala Tyr Asp Leu Asp Val Asp Asp Ala Pro
                    500                 505                 510

        Gly Asn Ser Gln Gln Ala Thr Pro Lys Asp Asn Glu Ile Ser Thr Phe
                515                 520                 525

        His Asn Leu Gly Asn Val His Ser Pro Leu Lys His His His His His
            530                 535                 540

        His
        545
```

```
                              SEQUENCE LISTING
            <110>  Chugai Seiyaku Kabushiki Kaisha
            <120>  combination therapy
            <130>  PCG-9026WO
            <150>  JP 2008-098309
            <151>  2008-04-04
            <150>  PCT/JP2008/002690
            <151>  2008-09-26
            <160>  36
            <170>  PatentIn version 3.1
            <210>  1
            <211>  115
            <212>  PRT
            <213>  Artificial Sequence
            <220>
            <223>  Modified antibody
            <400>  1
            Gln Val Gln Leu Gln Gln Ser Gly Ala Glu Leu Val Arg Pro Gly Ala
            1               5                   10                  15


            Ser Val Lys Leu Ser Cys Lys Ala Ser Gly Tyr Thr Phe Thr Asp Tyr
                        20                  25                  30


            Glu Met His Trp Val Lys Gln Thr Pro Val His Gly Leu Lys Trp Ile
                    35                  40                  45


            Gly Ala Leu Asp Pro Lys Thr Gly Asp Thr Ala Tyr Ser Gln Lys Phe
                50                  55                  60


            Lys Gly Lys Ala Thr Leu Thr Ala Asp Lys Ser Ser Ser Thr Ala Tyr
            65                  70                  75                  80


            Met Glu Leu Arg Ser Leu Thr Ser Glu Asp Ser Ala Val Tyr Tyr Cys
                            85                  90                  95


            Thr Arg Phe Tyr Ser Tyr Thr Tyr Trp Gly Gln Gly Thr Leu Val Thr
                        100                 105                 110


            Val Ser Ala
                    115


            <210>  2
            <211>  112
            <212>  PRT
            <213>  Artificial Sequence
            <220>
            <223>  Modified antibody
            <400>  2
            Asp Val Val Met Thr Gln Thr Pro Leu Ser Leu Pro Val Ser Leu Gly
            1               5                   10                  15


            Asp Gln Ala Ser Ile Ser Cys Arg Ser Ser Gln Ser Leu Val His Ser
                        20                  25                  30


            Asn Gly Asn Thr Tyr Leu His Trp Tyr Leu Gln Lys Pro Gly Gln Ser
                        35                  40                  45


            Pro Lys Leu Leu Ile Tyr Lys Val Ser Asn Arg Phe Ser Gly Val Pro
                50                  55                  60


            Asp Arg Phe Ser Gly Ser Gly Ser Gly Thr Asp Phe Thr Leu Lys Ile
```

                    65                      70                      75                      80

        Ser Arg Val Glu Ala Glu Asp Leu Gly Val Tyr Phe Cys Ser Gln Asn
                            85                      90                      95

        Thr His Val Pro Pro Thr Phe Gly Ser Gly Thr Lys Leu Glu Ile Lys
                    100                     105                     110

        <210> 3
        <211> 115
        <212> PRT
        <213> Artificial Sequence
        <220>
        <223> Modified antibody
        <400> 3
        Gln Val Gln Leu Val Gln Ser Gly Ala Glu Val Lys Lys Pro Gly Ala
        1               5                       10                      15

        Ser Val Lys Val Ser Cys Lys Ala Ser Gly Tyr Thr Phe Thr Asp Tyr
                    20                      25                      30

        Glu Met His Trp Val Arg Gln Ala Pro Gly Gln Gly Leu Glu Trp Met
                    35                      40                      45

        Gly Ala Leu Asp Pro Lys Thr Gly Asp Thr Ala Tyr Ser Gln Lys Phe
                50                      55                      60

        Lys Gly Arg Val Thr Leu Thr Ala Asp Lys Ser Thr Ser Thr Ala Tyr
        65                      70                      75                      80

        Met Glu Leu Ser Ser Leu Thr Ser Glu Asp Thr Ala Val Tyr Tyr Cys
                            85                      90                      95

        Thr Arg Phe Tyr Ser Tyr Thr Tyr Trp Gly Gln Gly Thr Leu Val Thr
                    100                     105                     110

        Val Ser Ser
                115

        <210> 4
        <211> 112
        <212> PRT
        <213> Artificial Sequence
        <220>
        <223> Modified antibody
        <400> 4
        Asp Val Val Met Thr Gln Ser Pro Leu Ser Leu Pro Val Thr Pro Gly
        1               5                       10                      15

        Glu Pro Ala Ser Ile Ser Cys Arg Ser Ser Gln Ser Leu Val His Ser
                    20                      25                      30

        Asn Gly Asn Thr Tyr Leu His Trp Tyr Leu Gln Lys Pro Gly Gln Ser
                    35                      40                      45

        Pro Gln Leu Leu Ile Tyr Lys Val Ser Asn Arg Phe Ser Gly Val Pro
                50                      55                      60

        Asp Arg Phe Ser Gly Ser Gly Ser Gly Thr Asp Phe Thr Leu Lys Ile
        65                      70                      75                      80

```
Ser Arg Val Glu Ala Glu Asp Val Gly Val Tyr Tyr Cys Ser Gln Asn
                85              90                  95

Thr His Val Pro Pro Thr Phe Gly Gln Gly Thr Lys Leu Glu Ile Lys
                100             105             110


<210>  5
<211>  5
<212>  PRT
<213>  Artificial Sequence
<220>
<223>  Modified antibody
<400>  5
Asp Tyr Glu Met His
1               5


<210>  6
<211>  16
<212>  PRT
<213>  Artificial Sequence
<220>
<223>  Modified antibody
<400>  6
Ala Leu Asp Pro Lys Thr Gly Asp Thr Ala Tyr Ser Gln Lys Phe Lys
1               5                   10                  15


<210>  7
<211>  6
<212>  PRT
<213>  Artificial Sequence
<220>
<223>  Modified antibody
<400>  7
Phe Tyr Ser Tyr Thr Tyr
1               5


<210>  8
<211>  16
<212>  PRT
<213>  Artificial Sequence
<220>
<223>  Modified antibody
<400>  8
Arg Ser Ser Gln Ser Leu Val His Ser Asn Gly Asn Thr Tyr Leu His
1               5                   10                  15


<210>  9
<211>  16
<212>  PRT
<213>  Artificial Sequence
<220>
<223>  Modified antibody
<400>  9
Arg Ser Ser Gln Ser Leu Val His Ser Asn Ala Asn Thr Tyr Leu His
1               5                   10                  15


<210>  10
<211>  16
<212>  PRT
<213>  Artificial Sequence
<220>
```

```
<223>  Modified antibody
<400>  10
Arg Ser Ser Gln Ser Leu Val His Ser Asn Asp Asn Thr Tyr Leu His
1                   5                   10                  15


<210>  11
<211>  16
<212>  PRT
<213>  Artificial Sequence
<220>
<223>  Modified antibody
<400>  11
Arg Ser Ser Gln Ser Leu Val His Ser Asn Glu Asn Thr Tyr Leu His
1                   5                   10                  15


<210>  12
<211>  16
<212>  PRT
<213>  Artificial Sequence
<220>
<223>  Modified antibody
<400>  12
Arg Ser Ser Gln Ser Leu Val His Ser Asn Phe Asn Thr Tyr Leu His
1                   5                   10                  15


<210>  13
<211>  16
<212>  PRT
<213>  Artificial Sequence
<220>
<223>  Modified antibody
<400>  13
Arg Ser Ser Gln Ser Leu Val His Ser Asn His Asn Thr Tyr Leu His
1                   5                   10                  15


<210>  14
<211>  16
<212>  PRT
<213>  Artificial Sequence
<220>
<223>  Modified antibody
<400>  14
Arg Ser Ser Gln Ser Leu Val His Ser Asn Asn Asn Thr Tyr Leu His
1                   5                   10                  15


<210>  15
<211>  16
<212>  PRT
<213>  Artificial Sequence
<220>
<223>  Modified antibody
<400>  15
Arg Ser Ser Gln Ser Leu Val His Ser Asn Thr Asn Thr Tyr Leu His
1                   5                   10                  15


<210>  16
<211>  16
<212>  PRT
<213>  Artificial Sequence
<220>
<223>  Modified antibody
```

```
<400>  16
Arg Ser Ser Gln Ser Leu Val His Ser Asn Gln Asn Thr Tyr Leu His
1               5                   10                  15

<210>  17
<211>  16
<212>  PRT
<213>  Artificial Sequence
<220>
<223>  Modified antibody
<400>  17
Arg Ser Ser Gln Ser Leu Val His Ser Asn Ile Asn Thr Tyr Leu His
1               5                   10                  15

<210>  18
<211>  16
<212>  PRT
<213>  Artificial Sequence
<220>
<223>  Modified antibody
<400>  18
Arg Ser Ser Gln Ser Leu Val His Ser Asn Lys Asn Thr Tyr Leu His
1               5                   10                  15

<210>  19
<211>  16
<212>  PRT
<213>  Artificial Sequence
<220>
<223>  Modified antibody
<400>  19
Arg Ser Ser Gln Ser Leu Val His Ser Asn Leu Asn Thr Tyr Leu His
1               5                   10                  15

<210>  20
<211>  16
<212>  PRT
<213>  Artificial Sequence
<220>
<223>  Modified antibody
<400>  20
Arg Ser Ser Gln Ser Leu Val His Ser Asn Ser Asn Thr Tyr Leu His
1               5                   10                  15

<210>  21
<211>  16
<212>  PRT
<213>  Artificial Sequence
<220>
<223>  Modified antibody
<400>  21
Arg Ser Ser Gln Ser Leu Val His Ser Asn Trp Asn Thr Tyr Leu His
1               5                   10                  15

<210>  22
<211>  16
<212>  PRT
<213>  Artificial Sequence
<220>
<223>  Modified antibody
<400>  22
```

Arg Ser Ser Gln Ser Leu Val His Ser Asn Tyr Asn Thr Tyr Leu His
1               5                   10                  15

<210>  23
<211>  16
<212>  PRT
<213>  Artificial Sequence
<220>
<223>  Modified antibody
<400>  23
Arg Ser Ser Gln Ser Leu Val His Ser Asn Arg Asn Thr Tyr Leu His
1               5                   10                  15

<210>  24
<211>  7
<212>  PRT
<213>  Artificial Sequence
<220>
<223>  Modified antibody
<400>  24
Lys Val Ser Asn Arg Phe Ser
1               5

<210>  25
<211>  9
<212>  PRT
<213>  Artificial Sequence
<220>
<223>  Modified antibody
<400>  25
Ser Gln Asn Thr His Val Pro Pro Thr
1               5

<210>  26
<211>  16
<212>  PRT
<213>  Artificial Sequence
<220>
<223>  Modified antibody
<400>  26
Ala Ile Asp Pro Lys Thr Gly Asp Thr Ala Tyr Ser Gln Ser Phe Gln
1               5                   10                  15

<210>  27
<211>  115
<212>  PRT
<213>  Artificial Sequence
<220>
<223>  Modified antibody
<400>  27
Gln Val Gln Leu Val Gln Ser Gly Ala Glu Val Lys Lys Pro Gly Ala
1               5                   10                  15

Ser Val Thr Val Ser Cys Lys Ala Ser Gly Tyr Thr Phe Thr Asp Tyr
            20                  25                  30

Glu Met His Trp Ile Arg Gln Pro Pro Gly Glu Gly Leu Glu Trp Ile
        35                  40                  45

Gly Ala Ile Asp Pro Lys Thr Gly Asp Thr Ala Tyr Ser Gln Ser Phe
        50                  55                  60

45

```
Gln Asp Arg Val Thr Leu Thr Ala Asp Lys Ser Thr Ser Thr Ala Tyr
65                  70                  75                  80

Met Glu Leu Ser Ser Leu Thr Ser Glu Asp Thr Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ser
        115

<210>  28
<211>  16
<212>  PRT
<213>  Artificial Sequence
<220>
<223>  Modified antibody
<400>  28
Arg Ala Ser Glu Ser Leu Val His Ser Asn Arg Asn Thr Tyr Leu His
1               5                   10                  15

<210>  29
<211>  112
<212>  PRT
<213>  Artificial Sequence
<220>
<223>  Modified antibody
<400>  29
Asp Ile Val Met Thr Gln Ser Pro Leu Ser Leu Pro Val Thr Pro Gly
1               5                   10                  15

Glu Pro Ala Ser Ile Ser Cys Arg Ala Ser Glu Ser Leu Val His Ser
            20                  25                  30

Asn Arg Asn Thr Tyr Leu His Trp Tyr Gln Gln Lys Pro Gly Gln Ala
            35                  40                  45

Pro Arg Leu Leu Ile Tyr Lys Val Ser Asn Arg Phe Ser Gly Val Pro
        50                  55                  60

Asp Arg Phe Ser Gly Ser Gly Ser Gly Thr Asp Phe Thr Leu Thr Ile
65                  70                  75                  80

Ser Ser Leu Gln Ala Glu Asp Val Gly Val Tyr Tyr Cys Ser Gln Asn
                85                  90                  95

Thr His Val Pro Pro Thr Phe Gly Gln Gly Thr Lys Leu Glu Ile Lys
            100                 105                 110

<210>  30
<211>  16
<212>  PRT
<213>  Artificial Sequence
<220>
<223>  Modified antibody
<400>  30
Ala Ile Asp Pro Lys Thr Gly Asp Thr Ala Tyr Ser Glu Ser Phe Gln
1               5                   10                  15
```

```
<210>  31
<211>  115
<212>  PRT
<213>  Artificial Sequence
<220>
<223>  Modified antibody
<400>  31
Gln Val Gln Leu Val Gln Ser Gly Ala Glu Val Lys Lys Pro Gly Ala
1               5                   10                  15

Ser Val Thr Val Ser Cys Lys Ala Ser Gly Tyr Thr Phe Thr Asp Tyr
            20                  25                  30

Glu Met His Trp Ile Arg Gln Pro Pro Gly Glu Gly Leu Glu Trp Ile
        35                  40                  45

Gly Ala Ile Asp Pro Lys Thr Gly Asp Thr Ala Tyr Ser Glu Ser Phe
    50                  55                  60

Gln Asp Arg Val Thr Leu Thr Ala Asp Lys Ser Thr Ser Thr Ala Tyr
65                  70                  75                  80

Met Glu Leu Ser Ser Leu Thr Ser Glu Asp Thr Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ser
        115

<210>  32
<211>  16
<212>  PRT
<213>  Artificial Sequence
<220>
<223>  Modified antibody
<400>  32
Gln Ala Ser Glu Ser Leu Val His Ser Asn Arg Asn Thr Tyr Leu His
1               5                   10                  15

<210>  33
<211>  112
<212>  PRT
<213>  Artificial Sequence
<220>
<223>  Modified antibody
<400>  33
Asp Ile Val Met Thr Gln Ser Pro Leu Ser Leu Pro Val Thr Pro Gly
1               5                   10                  15

Glu Pro Ala Ser Ile Ser Cys Gln Ala Ser Glu Ser Leu Val His Ser
            20                  25                  30

Asn Arg Asn Thr Tyr Leu His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
```

```
                65                      70                      75                      80

        Ser Arg Val Glu Ala Glu Asp Val Gly Val Tyr Tyr Cys Ser Gln Asn
                            85                      90                      95

        Thr His Val Pro Pro Thr Phe Gly Gln Gly Thr Lys Val Glu Ile Glu
                    100                     105                     110


        <210>   34
        <211>   115
        <212>   PRT
        <213>   Artificial Sequence
        <220>
        <223>   Modified antibody
        <400>   34
        Gln Val Gln Leu Val Gln Ser Gly Ala Glu Val Lys Lys Pro Gly Ala
        1                   5                       10                      15

        Ser Val Lys Val Ser Cys Lys Ala Ser Gly Tyr Thr Phe Thr Asp Tyr
                            20                      25                      30

        Glu Met His Trp Ile Arg Gln Pro Pro Gly Gln Gly Leu Glu Trp Ile
                        35                      40                      45

        Gly Ala Ile Asp Pro Lys Thr Gly Asp Thr Ala Tyr Ser Gln Lys Phe
                    50                      55                      60

        Lys Gly Arg Val Thr Leu Thr Ala Asp Lys Ser Thr Ser Thr Ala Tyr
        65                      70                      75                      80

        Met Glu Leu Ser Ser Leu Thr Ser Glu Asp Thr Ala Val Tyr Tyr Cys
                            85                      90                      95

        Thr Arg Phe Tyr Ser Tyr Thr Tyr Trp Gly Gln Gly Thr Leu Val Thr
                    100                     105                     110

        Val Ser Ser
                115


        <210>   35
        <211>   112
        <212>   PRT
        <213>   Artificial Sequence
        <220>
        <223>   Modified antibody
        <400>   35
        Asp Ile Val Met Thr Gln Ser Pro Leu Ser Leu Pro Val Thr Pro Gly
        1                   5                       10                      15

        Glu Pro Ala Ser Ile Ser Cys Arg Ser Ser Gln Ser Leu Val His Ser
                            20                      25                      30

        Asn Arg Asn Thr Tyr Leu His Trp Tyr Gln Gln Lys Pro Gly Gln Ala
                        35                      40                      45

        Pro Arg Leu Leu Ile Tyr Lys Val Ser Asn Arg Phe Ser Gly Val Pro
                    50                      55                      60

        Asp Arg Phe Ser Gly Ser Gly Ser Gly Thr Asp Phe Thr Leu Lys Ile
        65                      70                      75                      80
```

```
Ser Arg Val Glu Ala Glu Asp Val Gly Val Tyr Tyr Cys Ser Gln Asn
              85              90                  95

Thr His Val Pro Pro Thr Phe Gly Gln Gly Thr Lys Leu Glu Ile Lys
            100             105             110

<210>  36
<211>  545
<212>  PRT
<213>  homo sapiens
<400>  36
Gln Pro Pro Pro Pro Pro Asp Ala Thr Cys His Gln Val Arg Ser
1               5               10              15

Phe Phe Gln Arg Leu Gln Pro Gly Leu Lys Trp Val Pro Glu Thr Pro
            20              25              30

Val Pro Gly Ser Asp Leu Gln Val Cys Leu Pro Lys Gly Pro Thr Cys
            35              40              45

Cys Ser Arg Lys Met Glu Glu Lys Tyr Gln Leu Thr Ala Arg Leu Asn
        50              55              60

Met Glu Gln Leu Leu Gln Ser Ala Ser Met Glu Leu Lys Phe Leu Ile
65              70              75              80

Ile Gln Asn Ala Ala Val Phe Gln Glu Ala Phe Glu Ile Val Val Arg
            85              90              95

His Ala Lys Asn Tyr Thr Asn Ala Met Phe Lys Asn Asn Tyr Pro Ser
        100             105             110

Leu Thr Pro Gln Ala Phe Glu Phe Val Gly Glu Phe Phe Thr Asp Val
            115             120             125

Ser Leu Tyr Ile Leu Gly Ser Asp Ile Asn Val Asp Asp Met Val Asn
        130             135             140

Glu Leu Phe Asp Ser Leu Phe Pro Val Ile Tyr Thr Gln Leu Met Asn
145             150             155             160

Pro Gly Leu Pro Asp Ser Ala Leu Asp Ile Asn Glu Cys Leu Arg Gly
            165             170             175

Ala Arg Arg Asp Leu Lys Val Phe Gly Asn Phe Pro Lys Leu Ile Met
            180             185             190

Thr Gln Val Ser Lys Ser Leu Gln Val Thr Arg Ile Phe Leu Gln Ala
            195             200             205

Leu Asn Leu Gly Ile Glu Val Ile Asn Thr Thr Asp His Leu Lys Phe
        210             215             220

Ser Lys Asp Cys Gly Arg Met Leu Thr Arg Met Trp Tyr Cys Ser Tyr
225             230             235             240

Cys Gln Gly Leu Met Met Val Lys Pro Cys Gly Gly Tyr Cys Asn Val
            245             250             255

Val Met Gln Gly Cys Met Ala Gly Val Val Glu Ile Asp Lys Tyr Trp
            260             265             270
```

Arg Glu Tyr Ile Leu Ser Leu Glu Glu Leu Val Asn Gly Met Tyr Arg
        275             280             285

Ile Tyr Asp Met Glu Asn Val Leu Leu Gly Leu Phe Ser Thr Ile His
        290             295             300

Asp Ser Ile Gln Tyr Val Gln Lys Asn Ala Gly Lys Leu Thr Thr Thr
305             310             315             320

Ile Gly Lys Leu Cys Ala His Ser Gln Gln Arg Gln Tyr Arg Ser Ala
                325             330             335

Tyr Tyr Pro Glu Asp Leu Phe Ile Asp Lys Lys Val Leu Lys Val Ala
            340             345             350

His Val Glu His Glu Glu Thr Leu Ser Ser Arg Arg Arg Glu Leu Ile
        355             360             365

Gln Lys Leu Lys Ser Phe Ile Ser Phe Tyr Ser Ala Leu Pro Gly Tyr
        370             375             380

Ile Cys Ser His Ser Pro Val Ala Glu Asn Asp Thr Leu Cys Trp Asn
385             390             395             400

Gly Gln Glu Leu Val Glu Arg Tyr Ser Gln Lys Ala Ala Arg Asn Gly
                405             410             415

Met Lys Asn Gln Phe Asn Leu His Glu Leu Lys Met Lys Gly Pro Glu
                420             425             430

Pro Val Val Ser Gln Ile Ile Asp Lys Leu Lys His Ile Asn Gln Leu
            435             440             445

Leu Arg Thr Met Ser Met Pro Lys Gly Arg Val Leu Asp Lys Asn Leu
        450             455             460

Asp Glu Glu Gly Phe Glu Ala Gly Asp Cys Gly Asp Asp Glu Asp Glu
465             470             475             480

Cys Ile Gly Gly Ala Gly Asp Gly Met Ile Lys Val Lys Asn Gln Leu
            485             490             495

Arg Phe Leu Ala Glu Leu Ala Tyr Asp Leu Asp Val Asp Asp Ala Pro
        500             505             510

Gly Asn Ser Gln Gln Ala Thr Pro Lys Asp Asn Glu Ile Ser Thr Phe
        515             520             525

His Asn Leu Gly Asn Val His Ser Pro Leu Lys His His His His His
        530             535             540

His
545